(19) Europäisches Patentamt
European Patent Office
Office européen des brevets

(11) **EP 4 364 155 B1**

(12) **EUROPEAN PATENT SPECIFICATION**

(45) Date of publication and mention
of the grant of the patent:
**30.07.2025 Bulletin 2025/31**

(21) Application number: **22751495.7**

(22) Date of filing: **29.06.2022**

(51) International Patent Classification (IPC):
**G16B 50/30** (2019.01)          **G16B 40/20** (2019.01)
**G16B 30/00** (2019.01)

(52) Cooperative Patent Classification (CPC):
**G16B 30/00; G16B 40/20; G16B 50/30**

(86) International application number:
**PCT/US2022/035564**

(87) International publication number:
**WO 2023/278608 (05.01.2023 Gazette 2023/01)**

(54) **SELF-LEARNED BASE CALLER, TRAINED USING OLIGO SEQUENCES**

SELBSTLERNENDER BASENRUFER, TRAINIERT ANHAND VON OLIGOSEQUENZEN

APPEL DE BASE AUTO-APPRIS, FORMÉ À L'AIDE DE SÉQUENCES D'OLIGOS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.06.2021   US 202163216404 P
29.06.2021   US 202163216419 P
01.06.2022   US 202217830287
01.06.2022   US 202217830316**

(43) Date of publication of application:
**08.05.2024   Bulletin 2024/19**

(73) Proprietor: **Illumina, Inc.**
**San Diego, CA 92122 (US)**

(72) Inventors:
• **KIA, Amirali**
**San Mateo, Alaska 94401 (US)**
• **DUTTA, Anindita**
**San Diego, California 92122 (US)**

(74) Representative: **Lowden, Samuel Robert James**
**Marks & Clerk LLP**
**2nd Floor**
**Wytham Court**
**11 West Way**
**Oxford OX2 0JB (GB)**

(56) References cited:
**WO-A1-2015/095066     WO-A1-2019/147904**

**Description**

## FIELD OF THE TECHNOLOGY DISCLOSED

The technology disclosed relates to artificial intelligence type computers and digital data processing systems and corresponding data processing methods and products for emulation of intelligence (*i.e.,* knowledge based systems, reasoning systems, and knowledge acquisition systems); and including systems for reasoning with uncertainty (*e.g.*, fuzzy logic systems), adaptive systems, machine learning systems, and artificial neural networks. In particular, the technology disclosed relates to using deep neural networks such as deep convolutional neural networks for analyzing data.

## REFERENCES

Reference is made to the following:

PCT Patent Application titled "SELF-LEARNED BASE CALLER, TRAINED USING ORGANISM SEQUENCES," (Attorney Docket No. ILLM 1038-6/IP-2094-PCT) filed contemporaneously;
U.S. Provisional Patent Application No. 62/979,384, titled "ARTIFICIAL INTELLIGENCE-BASED BASE CALLING OF INDEX SEQUENCES," filed 20 February 2020 (Attorney Docket No. ILLM 1015-1/IP-1857-PRV);
U.S. Provisional Patent Application No. 62/979,414, titled "ARTIFICIAL INTELLIGENCE-BASED MANY-TO-MANY BASE CALLING," filed 20 February 2020 (Attorney Docket No. ILLM 1016-1/IP-1858-PRV);
U.S. Nonprovisional Patent Application No. 16/825,987, titled "TRAINING DATA GENERATION FOR ARTIFICIAL INTELLIGENCE-BASED SEQUENCING," filed 20 March 2020 (Attorney Docket No. ILLM 1008-16/IP-1693-US);
U.S. Nonprovisional Patent Application No. 16/825,991, titled "ARTIFICIAL INTELLIGENCE-BASED GENERATION OF SEQUENCING METADATA," filed 20 March 2020 (Attorney Docket No. ILLM 1008-17/IP-1741-US);
U.S. Nonprovisional Patent Application No. 16/826,126, titled "ARTIFICIAL INTELLIGENCE-BASED BASE CALLING," filed 20 March 2020 (Attorney Docket No. ILLM 1008-18/IP-1744-US);
U.S. Nonprovisional Patent Application No. 16/826,134, titled "ARTIFICIAL INTELLIGENCE-BASED QUALITY SCORING," filed 20 March 2020 (Attorney Docket No. ILLM 1008-19/IP-1747-US); and
U.S. Nonprovisional Patent Application No. 16/826,168, titled "ARTIFICIAL INTELLIGENCE-BASED SEQUENCING," filed 21 March 2020 (Attorney Docket No. ILLM 1008-20/IP-1752-PRV-US).

## BACKGROUND

The subject matter discussed in this section should not be assumed to be prior art merely as a result of its mention in this section. Similarly, a problem mentioned in this section or associated with the subject matter provided as background should not be assumed to have been previously recognized in the prior art. The subject matter in this section merely represents different approaches, which in and of themselves can also correspond to implementations of the claimed technology.

The rapid improvement in computation capability has made deep Convolution Neural Networks (CNNs) a great success in recent years on many computer vision tasks with significantly improved accuracy. During the inference phase, many applications demand low latency processing of one image with strict power consumption requirement, which reduces the efficiency of Graphics Processing Unit (GPU) and other general-purpose platform, bringing opportunities for specific acceleration hardware, **e.g.**, Field Programmable Gate Array (FPGA), by customizing the digital circuit specific for the deep learning algorithm inference. However, deploying CNNs on portable and embedded systems is still challenging due to large data volume, intensive computation, varying algorithm structures, and frequent memory accesses.

As convolution contributes most operations in CNNs, the convolution acceleration scheme significantly affects the efficiency and performance of a hardware CNN accelerator. Convolution involves multiply and accumulate (MAC) operations with four levels of loops that slide along kernel and feature maps. The first loop level computes the MAC of pixels within a kernel window. The second loop level accumulates the sum of products of the MAC across different input feature maps. After finishing the first and second loop levels, a final output element in the output feature map is obtained by adding the bias. The third loop level slides the kernel window within an input feature map. The fourth loop level generates different output feature maps.

FPGAs have gained increasing interest and popularity in particular to accelerate inference tasks, due to their (1) high degree of reconfigurability, (2) faster development time compared to Application Specific Integrated Circuits (ASICs) to catch up with the rapid evolving of CNNs, (3) good performance, and (4) superior energy efficiency compared to GPUs. The high performance and efficiency of an FPGA can be realized by synthesizing a circuit that is customized for a specific computation to directly process billions of operations with the customized memory systems. For instance, hundreds to thousands of digital signal processing (DSP) blocks on modern FPGAs support the core convolution operation, e.g.,

multiplication and addition, with high parallelism. Dedicated data buffers between external on-chip memory and on-chip processing engines (PEs) can be designed to realize the preferred dataflow by configuring tens of Mbyte on-chip block random access memories (BRAM) on the FPGA chip.

Efficient dataflow and hardware architecture of CNN acceleration are desired to minimize data communication while maximizing resource utilization to achieve high performance. An opportunity arises to design methodology and framework to accelerate the inference process of various CNN algorithms on acceleration hardware with high performance, efficiency, and flexibility.

WO 2015/095066 A1 describes a basecaller for DNA sequencing using machine learning.

## BRIEF DESCRIPTION OF THE DRAWINGS

In the drawings, like reference characters generally refer to like parts throughout the different views. Also, the drawings are not necessarily to scale, with an emphasis instead generally being placed upon illustrating the principles of the technology disclosed. In the following description, various implementations of the technology disclosed are described with reference to the following drawings, in which:

Fig. 1 illustrates a cross-section of a biosensor that can be used in various embodiments.

Fig. 2 depicts one implementation of a flow cell that contains clusters in its tiles.

Fig. 3 illustrates an example flow cell with eight lanes, and also illustrates a zoom-in on one tile and its clusters and their surrounding background.

Fig. 4 is a simplified block diagram of the system for analysis of sensor data from a sequencing system, such as base call sensor outputs.

Fig. 5 is a simplified diagram showing aspects of the base calling operation, including functions of a runtime program executed by a host processor.

Fig. 6 is a simplified diagram of a configuration of a configurable processor such as that of Fig. 4.

Fig. 7 is a diagram of a neural network architecture which can be executed using a configurable or a reconfigurable array configured as described herein.

Fig. 8A is a simplified illustration of an organization of tiles of sensor data used by a neural network architecture like that of Fig. 7.

Fig. 8B is a simplified illustration of patches of tiles of sensor data used by a neural network architecture like that of Fig. 7.

Fig. 9 illustrates part of a configuration for a neural network like that of Fig. 7 on a configurable or a reconfigurable array, such as a Field Programmable Gate Array (FPGA).

Fig. 10 is a diagram of another alternative neural network architecture which can be executed using a configurable or a reconfigurable array configured as described herein.

Fig. 11 illustrates one implementation of a specialized architecture of the neural network-based base caller that is used to segregate processing of data for different sequencing cycles.

Fig. 12 depicts one implementation of segregated layers, each of which can include convolutions.

Fig. 13A depicts one implementation of combinatory layers, each of which can include convolutions.

Fig. 13B depicts another implementation of the combinatory layers, each of which can include convolutions.

Fig. 14A illustrates a base calling system operating in a single-oligo training stage, to train a base caller comprising a neural network configuration, using a known synthetic oligo sequence, and Fig. 14A1 illustrates a comparison operation between a predicted base sequence and corresponding ground truth base sequence.

Fig. 14B illustrates further details of the base calling system of Fig. 14A operating in the single-oligo training stage, to train the base caller comprising the neural network configuration, using the known synthetic oligo sequence.

Fig. 15A illustrates the base calling system of Fig. 14A operating in a training data generation phase of a two-oligo training stage, to generate labelled training data using two known synthetic sequences.

Figs. 15B and 15C illustrate two corresponding example selections of two-oligo sequences discussed with respect to Fig. 15A.

Fig. 15D illustrates example mapping operations to either (i) map a predicted base call sequence to either of a first oligo or to a second oligo, or (ii) to declares inconclusiveness in mapping the predicted base call sequence to either of the two oligos.

Fig. 15E illustrates labelled training data generated from the mapping of Fig. 15D, where the training data is used by another neural network configuration illustrated in Fig. 16A.

Fig. 16A illustrates the base calling system of Fig. 14A operating in a training data consumption and training phase of a two-oligo training stage, to train the base caller comprising another neural network configuration (that is different from, and more complex, relative to the neural network configuration of Fig. 14A), using the two known synthetic oligo sequences.

Fig. 16B illustrates the base calling system of Fig. 14A operating in a second iteration of the training data generation phase of the two-oligo training stage.

Fig. 16C illustrates labelled training data generated from a mapping illustrated in Fig. 16B, where the training data is to be used for further training.

Fig. 16D illustrates the base calling system of Fig. 14A operating in a second iteration of the "training data consumption and training phase" of the "two-oligo training stage," to train the base caller comprising the neural network configuration of Fig. 16A, using the two known synthetic oligo sequences.

Fig. 17A illustrates a flowchart depicting an example method for iteratively training neural network configurations for base calling using single-oligo and two-oligo sequences.

Fig. 17B illustrates example labelled training data generated by the P$^{th}$ NN configuration at the end of method 1700 of Fig. 17A.

Fig. 18A illustrates the base calling system of Fig. 14A operating in a first iteration of a "training data consumption and training phase" of a "three-oligo training stage," to train the base caller comprising a 3-oligo neural network configuration.

Fig. 18B illustrates the base calling system of Fig. 14A operating in a "training data generation phase" of the "three-oligo training stage," to train the base caller comprising the 3-oligo neural network configuration of Fig. 18A.

Fig. 18C illustrates mapping operations to either (i) map a predicted base call sequence to any of the three oligos of Fig. 18B, or (ii) declare the mapping of the predicted base call sequence to be inconclusive.

Fig. 18D illustrates labelled training data generated from the mapping of Fig. 18C, where the training data is used to train another neural network configuration.

Fig. 18E illustrates a flowchart depicting an example method for iteratively training neural network configurations for base calling using 3-oligo ground truth sequences.

Fig. 19 illustrates a flowchart depicting an example method for iteratively training neural network configurations for base calling using multiple-oligo ground truth sequences.

Fig. 20A illustrates an organism sequence to be used to train the base caller of Fig. 14A.

Fig. 20B illustrates the base calling system of Fig. 14A operating in a training data generation phase of a first organism training stage, to train the base caller comprising a first organism level neural network configuration, using various subsequences of a first organism sequence of Fig. 20A.

Fig. 20C illustrates an example of fading, in which signal intensity is decreased as a function of cycle number is a sequencing run of a base calling operation.

Fig. 20D conceptually illustrates a decreasing signal-to-noise ratio as cycles of sequencing progress.

Fig. 20E illustrates base calling of a first L2 number of bases of L1 number of bases of a subsequence, where the first L2 number of bases of the subsequence is used to map the subsequence to the organism sequence of Fig. 20A.

Fig. 20F illustrates labelled training data generated from the mapping of Fig. 20E, wherein the labelled training data includes sections of the organism sequence of Fig. 20A as ground truth.

Fig. 20G illustrates the base calling system of Fig. 14A operating in a "training data consumption and training phase" of the "organism level training stage," to train the base caller comprising the first organism level neural network configuration.

Fig. 21 illustrates a flowchart depicting an example method for iteratively training neural network configurations for base calling using the simple organism sequence of Fig. 20A.

Fig. 22 illustrates usage of complex organism sequences for training of corresponding NN configurations for the base caller of Fig. 14A.

Fig. 23A illustrates a flowchart depicting an example method for iteratively training neural network configurations for base calling, and Figs. 23B-23E illustrate various charts illustrating effectiveness of the base caller training process discussed in this disclosure.

Fig. 24 is a block diagram of a base calling system in accordance with one implementation.

Fig. 25 is a block diagram of a system controller that can be used in the system of Fig. 24.

Fig. 26 is a simplified block diagram of a computer system that can be used to implement the technology disclosed.

## DETAILED DESCRIPTION

As used herein, the terms "polynucleotide" or "nucleic acids" refer to deoxyribonucleic acid (DNA), but where appropriate the skilled artisan will recognize that the systems and devices herein can also be utilized with ribonucleic acid (RNA). The terms should be understood to include, as equivalents, analogs of either DNA or RNA made from nucleotide analogs. The terms as used herein also encompasses cDNA, that is complementary, or copy, DNA produced from an RNA template, for example by the action of reverse transcriptase.

The single stranded polynucleotide molecules sequenced by the systems and devices herein can have originated in single-stranded form, as DNA or RNA or have originated in double-stranded DNA (dsDNA) form (e.g.,

genomic DNA fragments, PCR and amplification products and the like). Thus, a single stranded polynucleotide may be the sense or antisense strand of a polynucleotide duplex. Methods of preparation of single stranded polynucleotide molecules suitable for use in the method of the disclosure using standard techniques are well known in the art. The precise sequence of the primary polynucleotide molecules is generally not material to the disclosure, and may be known or unknown. The single stranded polynucleotide molecules can represent genomic DNA molecules (e.g., human genomic DNA) including both intron and exon sequences (coding sequence), as well as non-coding regulatory sequences such as promoter and enhancer sequences.

In certain embodiments, the nucleic acid to be sequenced through use of the current disclosure is immobilized upon a substrate (e.g., a substrate within a flowcell or one or more beads upon a substrate such as a flowcell, etc.). The term "immobilized" as used herein is intended to encompass direct or indirect, covalent or non-covalent attachment, unless indicated otherwise, either explicitly or by context. In certain embodiments covalent attachment may be preferred, but generally all that is required is that the molecules (e.g., nucleic acids) remain immobilized or attached to the support under conditions in which it is intended to use the support, for example in applications requiring nucleic acid sequencing.

The term "solid support" (or "substrate" in certain usages) as used herein refers to any inert substrate or matrix to which nucleic acids can be attached, such as for example glass surfaces, plastic surfaces, latex, dextran, polystyrene surfaces, polypropylene surfaces, polyacrylamide gels, gold surfaces, and silicon wafers. In many embodiments, the solid support is a glass surface (e.g., the planar surface of a flowcell channel). In certain embodiments the solid support may comprise an inert substrate or matrix which has been "functionalized," for example by the application of a layer or coating of an intermediate material comprising reactive groups which permit covalent attachment to molecules such as polynucleotides. By way of non-limiting example such supports can include polyacrylamide hydrogels supported on an inert substrate such as glass. In such embodiments the molecules (polynucleotides) can be directly covalently attached to the intermediate material (e.g., the hydrogel) but the intermediate material can itself be non-covalently attached to the substrate or matrix (e.g., the glass substrate). Covalent attachment to a solid support is to be interpreted accordingly as encompassing this type of arrangement.

As indicated above, the present disclosure comprises novel systems and devices for sequencing nucleic acids. As will be apparent to those of skill in the art, references herein to a particular nucleic acid sequence may, depending on the context, also refer to nucleic acid molecules which comprise such nucleic acid sequence. Sequencing of a target fragment means that a read of the chronological order of bases is established. The bases that are read do not need to be contiguous, although this is preferred, nor does every base on the entire fragment have to be sequenced during the sequencing. Sequencing can be carried out using any suitable sequencing technique, wherein nucleotides or oligonucleotides are added successively to a free 3' hydroxyl group, resulting in synthesis of a polynucleotide chain in the 5' to 3' direction. The nature of the nucleotide added is preferably determined after each nucleotide addition. Sequencing techniques using sequencing by ligation, wherein not every contiguous base is sequenced, and techniques such as massively parallel signature sequencing (MPSS) where bases are removed from, rather than added to, the strands on the surface are also amenable to use with the systems and devices of the disclosure.

In certain embodiments, the current disclosure discloses sequencing-by-synthesis (SBS). In SBS, four fluorescently labeled modified nucleotides are used to sequence dense clusters of amplified DNA (possibly millions of clusters) present on the surface of a substrate (e.g., a flowcell). Various additional aspects regarding SBS procedures and methods, which can be utilized with the systems and devices herein, are disclosed in, for example, WO04018497, WO04018493 and U.S. Pat. No. 7,057,026 (nucleotides), WO05024010 and WO06120433 (polymerases), WO05065814 (surface attachment techniques), and WO 9844151, WO06064199 and WO07010251.

In particular uses of the systems/devices herein the flowcells containing the nucleic acid samples for sequencing are placed within the appropriate flowcell holder. The samples for sequencing can take the form of single molecules, amplified single molecules in the form of clusters, or beads comprising molecules of nucleic acid. The nucleic acids are prepared such that they comprise an oligonucleotide primer adjacent to an unknown target sequence. To initiate the first SBS sequencing cycle, one or more differently labeled nucleotides, and DNA polymerase, etc., are flowed into/through the flowcell by the fluid flow subsystem (various embodiments of which are described herein). Either a single nucleotide can be added at a time, or the nucleotides used in the sequencing procedure can be specially designed to possess a reversible termination property, thus allowing each cycle of the sequencing reaction to occur simultaneously in the presence of all four labeled nucleotides (A, C, T, G). Where the four nucleotides are mixed together, the polymerase is able to select the correct base to incorporate and each sequence is extended by a single base. In such methods of using the systems, the natural competition between all four alternatives leads to higher accuracy than wherein only one nucleotide is present in the reaction mixture (where most of the sequences are therefore not exposed to the correct nucleotide). Sequences where a particular base is repeated one after another (e.g., homopolymers) are addressed like any other sequence and with high accuracy.

The fluid flow subsystem also flows the appropriate reagents to remove the blocked 3' terminus (if appropriate) and the fluorophore from each incorporated base. The substrate can be exposed either to a second round of the four blocked nucleotides, or optionally to a second round with a different individual nucleotide. Such cycles are then repeated, and the sequence of each cluster is read over the multiple chemistry cycles. The computer aspect of the current disclosure can optionally align the sequence data gathered from each single molecule, cluster or bead to determine the sequence of longer polymers, etc. Alternatively, the image processing and alignment can be performed on a separate computer.

The heating/cooling components of the system regulate the reaction conditions within the flowcell channels and reagent storage areas/containers (and optionally the camera, optics, and/or other components), while the fluid flow components allow the substrate surface to be exposed to suitable reagents for incorporation (*e.g.,* the appropriate fluorescently labeled nucleotides to be incorporated) while unincorporated reagents are rinsed away. An optional movable stage upon which the flowcell is placed allows the flowcell to be brought into proper orientation for laser (or other light) excitation of the substrate and optionally moved in relation to a lens objective to allow reading of different areas of the substrate. Additionally, other components of the system are also optionally movable/adjustable (*e.g.,* the camera, the lens objective, the heater/cooler, etc.). During laser excitation, the image/location of emitted fluorescence from the nucleic acids on the substrate is captured by the camera component, thereby, recording the identity, in the computer component, of the first base for each single molecule, cluster or bead.

Embodiments described herein may be used in various biological or chemical processes and systems for academic or commercial analysis. More specifically, embodiments described herein may be used in various processes and systems where it is desired to detect an event, property, quality, or characteristic that is indicative of a desired reaction. For example, embodiments described herein include cartridges, biosensors, and their components as well as bioassay systems that operate with cartridges and biosensors. In particular embodiments, the cartridges and biosensors include a flow cell and one or more sensors, pixels, light detectors, or photodiodes that are coupled together in a substantially unitary structure.

The following detailed description of certain embodiments will be better understood when read in conjunction with the appended drawings. To the extent that the figures illustrate diagrams of the functional blocks of various embodiments, the functional blocks are not necessarily indicative of the division between hardware circuitry. Thus, for example, one or more of the functional blocks (*e.g.,* processors or memories) may be implemented in a single piece of hardware (*e.g.,* a general purpose signal processor or random access memory, hard disk, or the like). Similarly, the programs may be standalone programs, may be incorporated as subroutines in an operating system, may be functions in an installed software package, and the like. It should be understood that the various embodiments are not limited to the arrangements and instrumentality shown in the drawings.

As used herein, an element or step recited in the singular and proceeded with the word "a" or "an" should be understood as not excluding plural of said elements or steps, unless such exclusion is explicitly stated. Furthermore, references to "one embodiment" are not intended to be interpreted as excluding the existence of additional embodiments that also incorporate the recited features. Moreover, unless explicitly stated to the contrary, embodiments "comprising" or "having" or "including" an element or a plurality of elements having a particular property may include additional elements whether or not they have that property.

As used herein, a "desired reaction" includes a change in at least one of a chemical, electrical, physical, or optical property (or quality) of an analyte-of-interest. In particular embodiments, the desired reaction is a positive binding event (*e.g.,* incorporation of a fluorescently labeled biomolecule with the analyte-of-interest). More generally, the desired reaction may be a chemical transformation, chemical change, or chemical interaction. The desired reaction may also be a change in electrical properties. For example, the desired reaction may be a change in ion concentration within a solution. Exemplary reactions include, but are not limited to, chemical reactions such as reduction, oxidation, addition, elimination, rearrangement, esterification, amidation, etherification, cyclization, or substitution; binding interactions in which a first chemical binds to a second chemical; dissociation reactions in which two or more chemicals detach from each other; fluorescence; luminescence; bioluminescence; chemiluminescence; and biological reactions, such as nucleic acid replication, nucleic acid amplification, nucleic acid hybridization, nucleic acid ligation, phosphorylation, enzymatic catalysis, receptor binding, or ligand binding. The desired reaction can also be an addition or elimination of a proton, for example, detectable as a change in pH of a surrounding solution or environment. An additional desired reaction can be detecting the flow of ions across a membrane (*e.g.,* natural or synthetic bilayer membrane), for example as ions flow through a membrane the current is disrupted and the disruption can be detected.

In particular embodiments, the desired reaction includes the incorporation of a fluorescently-labeled molecule to an analyte. The analyte may be an oligonucleotide and the fluorescently-labeled molecule may be a nucleotide. The desired reaction may be detected when an excitation light is directed toward the oligonucleotide having the labeled nucleotide, and the fluorophore emits a detectable fluorescent signal. In alternative embodiments, the detected fluorescence is a result of chemiluminescence or bioluminescence. A desired reaction may also increase fluorescence (or Förster) resonance energy transfer (FRET), for example, by bringing a donor fluorophore in proximity to an acceptor fluorophore, decrease FRET by separating donor and acceptor fluorophores, increase fluorescence by separating a quencher from a fluorophore or decrease fluorescence by co-locating a quencher and fluorophore.

As used herein, a "reaction component" or "reactant" includes any substance that may be used to obtain a desired reaction. For example, reaction components include reagents, enzymes, samples, other biomolecules, and buffer solutions. The reaction components are typically delivered to a reaction site in a solution and/or immobilized at a reaction site. The reaction components may interact directly or indirectly with another substance, such as the analyte-of-interest.

As used herein, the term "reaction site" is a localized region where a desired reaction may occur. A reaction site may include support surfaces of a substrate where a substance may be immobilized thereon. For example, a reaction site may include a substantially planar surface in a channel of a flow cell that has a colony of nucleic acids thereon. Typically, but not always, the nucleic acids in the colony have the same sequence, being for example, clonal copies of a single stranded or double stranded template. However, in some embodiments a reaction site may contain only a single nucleic acid molecule, for example, in a single stranded or double stranded form. Furthermore, a plurality of reaction sites may be unevenly distributed along the support surface or arranged in a predetermined manner (*e.g.,* side-by-side in a matrix, such as in microarrays). A reaction site can also include a reaction chamber (or well) that at least partially defines a spatial region or volume configured to compartmentalize the desired reaction.

This application uses the terms "reaction chamber" and "well" interchangeably. As used herein, the term "reaction chamber" or "well" includes a spatial region that is in fluid communication with a flow channel. The reaction chamber may be at least partially separated from the surrounding environment or other spatial regions. For example, a plurality of reaction chambers may be separated from each other by shared walls. As a more specific example, the reaction chamber may include a cavity defined by interior surfaces of a well and have an opening or aperture so that the cavity may be in fluid communication with a flow channel. Biosensors including such reaction chambers are described in greater detail in international application no. PCT/US2011/057111, filed on October 20, 2011

In some embodiments, the reaction chambers are sized and shaped relative to solids (including semi-solids) so that the solids may be inserted, fully or partially, therein. For example, the reaction chamber may be sized and shaped to accommodate only one capture bead. The capture bead may have clonally amplified DNA or other substances thereon. Alternatively, the reaction chamber may be sized and shaped to receive an approximate number of beads or solid substrates. As another example, the reaction chambers may also be filled with a porous gel or substance that is configured to control diffusion or filter fluids that may flow into the reaction chamber.

In some embodiments, sensors (*e.g.,* light detectors, photodiodes) are associated with corresponding pixel areas of a sample surface of a biosensor. As such, a pixel area is a geometrical construct that represents an area on the biosensor's sample surface for one sensor (or pixel). A sensor that is associated with a pixel area detects light emissions gathered from the associated pixel area when a desired reaction has occurred at a reaction site or a reaction chamber overlying the associated pixel area. In a flat surface embodiment, the pixel areas can overlap. In some cases, a plurality of sensors may be associated with a single reaction site or a single reaction chamber. In other cases, a single sensor may be associated with a group of reaction sites or a group of reaction chambers.

As used herein, a "biosensor" includes a structure having a plurality of reaction sites and/or reaction chambers (or wells). A biosensor may include a solid-state imaging device (*e.g.,* CCD or CMOS imager) and, optionally, a flow cell mounted thereto. The flow cell may include at least one flow channel that is in fluid communication with the reaction sites and/or the reaction chambers. As one specific example, the biosensor is configured to fluidically and electrically couple to a bioassay system. The bioassay system may deliver reactants to the reaction sites and/or the reaction chambers according to a predetermined protocol (*e.g.*, sequencing-by-synthesis) and perform a plurality of imaging events. For example, the bioassay system may direct solutions to flow along the reaction sites and/or the reaction chambers. At least one of the solutions may include four types of nucleotides having the same or different fluorescent labels. The nucleotides may bind to corresponding oligonucleotides located at the reaction sites and/or the reaction chambers. The bioassay system may then illuminate the reaction sites and/or the reaction chambers using an excitation light source (*e.g.,* solid-state light sources, such as light-emitting diodes or LEDs). The excitation light may have a predetermined wavelength or wavelengths, including a range of wavelengths. The excited fluorescent labels provide emission signals that may be captured by the sensors.

In alternative embodiments, the biosensor may include electrodes or other types of sensors configured to detect other identifiable properties. For example, the sensors may be configured to detect a change in ion concentration. In another example, the sensors may be configured to detect the ion current flow across a membrane.

As used herein, a "cluster" is a colony of similar or identical molecules or nucleotide sequences or DNA strands. For example, a cluster can be an amplified oligonucleotide or any other group of a polynucleotide or polypeptide with a same or similar sequence. In other embodiments, a cluster can be any element or group of elements that occupy a physical area on a sample surface. In embodiments, clusters are immobilized to a reaction site and/or a reaction chamber during a base calling cycle.

As used herein, the term "immobilized," when used with respect to a biomolecule or biological or chemical substance, includes substantially attaching the biomolecule or biological or chemical substance at a molecular level to a surface. For example, a biomolecule or biological or chemical substance may be immobilized to a surface of the substrate material using adsorption techniques including non-covalent interactions (*e.g.,* electrostatic forces, van der Waals, and dehydration of hydrophobic interfaces) and covalent binding techniques where functional groups or linkers facilitate attaching the biomolecules to the surface. Immobilizing biomolecules or biological or chemical substances to a surface of a substrate material may be based upon the properties of the substrate surface, the liquid medium carrying the biomolecule or biological or chemical substance, and the properties of the biomolecules or biological or chemical substances themselves. In some cases, a substrate surface may be functionalized (*e.g.*, chemically or physically modified) to facilitate immobilizing the biomolecules (or biological or chemical substances) to the substrate surface. The substrate surface may be first modified to have functional groups bound to the surface. The functional groups may then bind to biomolecules or biological or chemical substances to immobilize them thereon. A substance can be immobilized to a surface via a gel, for example, as described in US Patent Publ. No. US 2011/0059865 A1.

In some embodiments, nucleic acids can be attached to a surface and amplified using bridge amplification. Useful bridge amplification methods are described, for example, in U.S. Patent No. 5,641,658; WO 2007/010251; U.S. Pat. No. 6,090,592; U.S. Patent Publ. No. 2002/0055100 A1; U.S. Patent No. 7,115,400; U.S. Patent Publ. No. 2004/0096853 A1; U.S. Patent Publ. No. 2004/0002090 A1; U.S. Patent Publ. No. 2007/0128624 A1; and U.S. Patent Publ. No. 2008/0009420 A1. Another useful method for amplifying nucleic acids on a surface is Rolling Circle Amplification (RCA), for example, using methods set forth in further detail below. In some embodiments, the nucleic acids can be attached to a surface and amplified using one or more primer pairs. For example, one of the primers can be in solution and the other primer can be immobilized on the surface (*e.g.,* 5'-attached). By way of example, a nucleic acid molecule can hybridize to one of the primers on the surface followed by extension of the immobilized primer to produce a first copy of the nucleic acid. The primer in solution then hybridizes to the first copy of the nucleic acid which can be extended using the first copy of the nucleic acid as a template. Optionally, after the first copy of the nucleic acid is produced, the original nucleic acid molecule can hybridize to a second immobilized primer on the surface and can be extended at the same time or after the primer in solution is extended. In any embodiment, repeated rounds of extension (*e.g.,* amplification) using the immobilized primer and primer in solution provide multiple copies of the nucleic acid.

In particular embodiments, the assay protocols executed by the systems and methods described herein include the use of natural nucleotides and also enzymes that are configured to interact with the natural nucleotides. Natural nucleotides include, for example, ribonucleotides (RNA) or deoxyribonucleotides (DNA). Natural nucleotides can be in the mono-, di-, or tri-phosphate form and can have a base selected from adenine (A), thymine (T), uracil (U), guanine (G) or cytosine (C). It will be understood however that non-natural nucleotides, modified nucleotides or analogs of the aforementioned nucleotides can be used. Some examples of useful non-natural nucleotides are set forth below in regard to reversible terminator-based sequencing by synthesis methods.

In embodiments that include reaction chambers, items or solid substances (including semisolid substances) may be disposed within the reaction chambers. When disposed, the item or solid may be physically held or immobilized within the reaction chamber through an interference fit, adhesion, or entrapment. Exemplary items or solids that may be disposed within the reaction chambers include polymer beads, pellets, agarose gel, powders, quantum dots, or other solids that may be compressed and/or held within the reaction chamber. In particular embodiments, a nucleic acid superstructure, such as a DNA ball, can be disposed in or at a reaction chamber, for example, by attachment to an interior surface of the reaction chamber or by residence in a liquid within the reaction chamber. A DNA ball or other nucleic acid superstructure can be preformed and then disposed in or at the reaction chamber. Alternatively, a DNA ball can be synthesized in situ at the reaction chamber. A DNA ball can be synthesized by rolling circle amplification to produce a concatemer of a particular nucleic acid sequence and the concatemer can be treated with conditions that form a relatively compact ball. DNA balls and methods for their synthesis are described, for example in, U.S. Patent Publication Nos. 2008/0242560 A1 or 2008/0234136 A1. A substance that is held or disposed in a reaction chamber can be in a solid, liquid, or gaseous state.

As used herein, "base calling" identifies a nucleotide base in a nucleic acid sequence. Base calling refers to the process of determining a base call (A, C, G, T) for every cluster at a specific cycle. As an example, base calling can be performed utilizing four-channel, two-channel or one-channel methods and systems described in U.S. Patent Application Publication No 2013/0079232. In particular embodiments, a base calling cycle is referred to as a "sampling event." In one dye and two-channel sequencing protocol, a sampling event comprises two illumination stages in time sequence, such that a pixel signal is generated at each stage. The first illumination stage induces illumination from a given cluster indicating nucleotide bases A and T in a AT pixel signal, and the second illumination stage induces illumination from a given cluster indicating nucleotide bases C and T in a CT pixel signal.

The technology disclosed, *e.g.,* the disclosed base callers can be implemented on processors like Central Processing Units (CPUs), Graphics Processing Units (GPUs), Field Programmable Gate Arrays (FPGAs), Coarse-Grained Reconfigurable Architectures (CGRAs), Application-Specific Integrated Circuits (ASICs), Application Specific Instruction-set Processor (ASIP), and Digital Signal Processors (DSPs).

## Biosensor

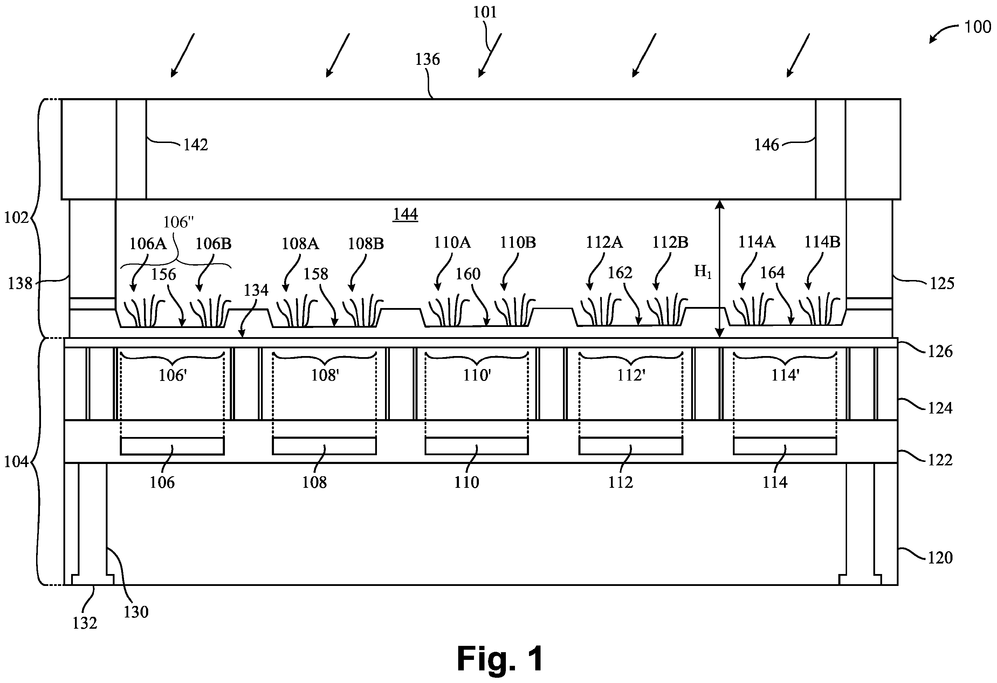

**Fig. 1** illustrates a cross-section of a biosensor 100 that can be used in various embodiments. Biosensor 100 has pixel areas 106', 108', 110', 112', and 114' that can each hold more than one cluster during a base calling cycle (*e.g.,* 2 clusters per pixel area). As shown, the biosensor 100 may include a flow cell 102 that is mounted onto a sampling device 104. In the illustrated embodiment, the flow cell 102 is affixed directly to the sampling device 104. However, in alternative embodiments, the flow cell 102 may be removably coupled to the sampling device 104. The sampling device 104 has a sample surface 134 that may be functionalized (*e.g.,* chemically or physically modified in a suitable manner for conducting the desired reactions). For example, the sample surface 134 may be functionalized and may include a plurality of pixel areas 106', 108', 110', 112', and 114' that can each hold more than one cluster during a base calling cycle (*e.g.*, each having a corresponding cluster pair 106A, 106B; 108A, 108B; 110A, 110B; 112A, 112B; and 114A, 114B immobilized thereto). Each pixel area is associated with a corresponding sensor (or pixel or photodiode) 106, 108, 110, 112, and 114, such that light received by the pixel area is captured by the corresponding sensor. A pixel area 106' can be also associated with a corresponding reaction site 106" on the sample surface 134 that holds a cluster pair, such that light emitted from the reaction site 106" is received by the pixel area 106' and captured by the corresponding sensor 106. As a result of this sensing structure, in the case in which two or more clusters are present in a pixel area of a particular sensor during a base calling cycle (*e.g.*, each having a corresponding cluster pair), the pixel signal in that base calling cycle carries information based on all of the two or more clusters. As a result, signal processing as described herein is used to distinguish each cluster, where there are more clusters than pixel signals in a given sampling event of a particular base calling cycle.

In the illustrated embodiment, the flow cell 102 includes sidewalls 138, 125, and a flow cover 136 that is supported by the sidewalls 138, 125. The sidewalls 138, 125 are coupled to the sample surface 134 and extend between the flow cover 136 and the sidewalls 138, 125. In some embodiments, the sidewalls 138, 125 are formed from a curable adhesive layer that bonds the flow cover 136 to the sampling device 104.

The sidewalls 138, 125 are sized and shaped so that a flow channel 144 exists between the flow cover 136 and the sampling device 104. The flow cover 136 may include a material that is transparent to excitation light 101 propagating from an exterior of the biosensor 100 into the flow channel 144. In an example, the excitation light 101 approaches the flow cover 136 at a non-orthogonal angle.

Also shown, the flow cover 136 may include inlet and outlet ports 142, 146 that are configured to fluidically engage other ports (not shown). For example, the other ports may be from the cartridge or the workstation. The flow channel 144 is sized and shaped to direct a fluid along the sample surface 134. A height $H_1$ and other dimensions of the flow channel 144 may be configured to maintain a substantially even flow of a fluid along the sample surface 134. The dimensions of the flow channel 144 may also be configured to control bubble formation.

By way of example, the flow cover 136 (or the flow cell 102) may comprise a transparent material, such as glass or plastic. The flow cover 136 may constitute a substantially rectangular block having a planar exterior surface and a planar inner surface that defines the flow channel 144. The block may be mounted onto the sidewalls 138, 125. Alternatively, the flow cell 102 may be etched to define the flow cover 136 and the sidewalls 138, 125. For example, a recess may be etched into the transparent material. When the etched material is mounted to the sampling device 104, the recess may become the flow channel 144.

The sampling device 104 may be similar to, for example, an integrated circuit comprising a plurality of stacked substrate layers 120-126. The substrate layers 120-126 may include a base substrate 120, a solid-state imager 122 (*e.g.,* CMOS image sensor), a filter or light-management layer 124, and a passivation layer 126. It should be noted that the above is only illustrative and that other embodiments may include fewer or additional layers. Moreover, each of the substrate layers 120-126 may include a plurality of sub-layers. The sampling device 104 may be manufactured using processes that are similar to those used in manufacturing integrated circuits, such as CMOS image sensors and CCDs. For example, the substrate layers 120-126 or portions thereof may be grown, deposited, etched, and the like to form the sampling device 104.

The passivation layer 126 is configured to shield the filter layer 124 from the fluidic environment of the flow channel 144. In some cases, the passivation layer 126 is also configured to provide a solid surface (*i.e.,* the sample surface 134) that permits biomolecules or other analytes-of-interest to be immobilized thereon. For example, each of the reaction sites may include a cluster of biomolecules that are immobilized to the sample surface 134. Thus, the passivation layer 126 may be formed from a material that permits the reaction sites to be immobilized thereto. The passivation layer 126 may also comprise a material that is at least transparent to a desired fluorescent light. By way of example, the passivation layer 126 may include silicon nitride ($Si_2N_4$) and/or silica ($SiO_2$). However, other suitable material(s) may be used. In the illustrated embodiment, the passivation layer 126 may be substantially planar. However, in alternative embodiments, the passivation layer 126 may include recesses, such as pits, wells, grooves, and the like. In the illustrated embodiment, the passivation layer 126 has a thickness that is about 150-200 nm and, more particularly, about 170 nm.

The filter layer 124 may include various features that affect the transmission of light. In some embodiments, the filter layer 124 can perform multiple functions. For instance, the filter layer 124 may be configured to (a) filter unwanted light signals, such as light signals from an excitation light source; (b) direct emission signals from the reaction sites toward corresponding sensors 106, 108, 110, 112, and 114 that are configured to detect the emission signals from the reaction sites; or (c) block or prevent detection of unwanted emission signals from adjacent reaction sites. As such, the filter layer 124 may also be referred to as a light-management layer. In the illustrated embodiment, the filter layer 124 has a thickness that is about 1-5 $\mu$m and, more particularly, about 2-4 $\mu$m. In alternative embodiments, the filter layer 124 may include an array of microlenses or other optical components. Each of the microlenses may be configured to direct emission signals from an associated reaction site to a sensor.

In some embodiments, the solid-state imager 122 and the base substrate 120 may be provided together as a previously constructed solid-state imaging device (*e.g.*, CMOS chip). For example, the base substrate 120 may be a wafer of silicon and the solid-state imager 122 may be mounted thereon. The solid-state imager 122 includes a layer of semiconductor material (*e.g.*, silicon) and the sensors 106, 108, 110, 112, and 114. In the illustrated embodiment, the sensors are photodiodes configured to detect light. In other embodiments, the sensors comprise light detectors. The solid-state imager 122 may be manufactured as a single chip through a CMOS-based fabrication processes.

The solid-state imager 122 may include a dense array of sensors 106, 108, 110, 112, and 114 that are configured to detect activity indicative of a desired reaction from within or along the flow channel 144. In some embodiments, each sensor has a pixel area (or detection area) that is about 1-2 square micrometer ($\mu m^2$). The array can include 500,000 sensors, 5 million sensors, 10 million sensors, or even 120 million sensors. The sensors 106, 108, 110, 112, and 114 can be configured to detect a predetermined wavelength of light that is indicative of the desired reactions.

In some embodiments, the sampling device 104 includes a microcircuit arrangement, such as the microcircuit arrangement described in U.S. Patent No. 7,595,882. More specifically, the sampling device 104 may comprise an integrated circuit having a planar array of the sensors 106, 108, 110, 112, and 114. Circuitry formed within the sampling device 104 may be configured for at least one of signal amplification, digitization, storage, and processing. The circuitry may collect and analyze the detected fluorescent light and generate pixel signals (or detection signals) for communicating detection data to a signal processor. The circuitry may also perform additional analog and/or digital signal processing in the sampling device 104. Sampling device 104 may include conductive vias 130 that perform signal routing (*e.g.*, transmit the pixel signals to the signal processor). The pixel signals may also be transmitted through electrical contacts 132 of the sampling device 104.

The sampling device 104 is discussed in further details with respect to U.S. Nonprovisional Patent Application No. 16/874,599, titled "Systems and Devices for Characterization and Performance Analysis of Pixel-Based Sequencing," filed May 14, 2020 (Attorney Docket No. ILLM 1011-4/IP-1750-US). The sampling device 104 is not limited to the above constructions or uses as described above. In alternative embodiments, the sampling device 104 may take other forms. For example, the sampling device 104 may comprise a CCD device, such as a CCD camera, that is coupled to a flow cell or is moved to interface with a flow cell having reaction sites therein.

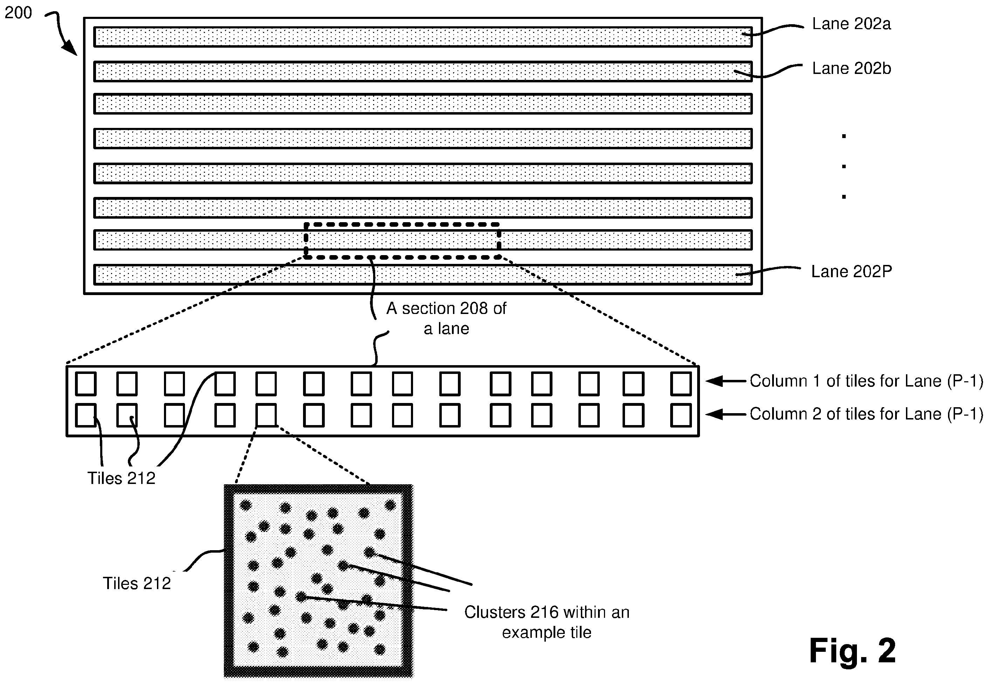

Fig. 2 depicts one implementation of a flow cell 200 that contains clusters in its tiles. The flow cell 200 corresponds to the flow cell 102 of Fig. 1, e.g., without the flow cover 136. Furthermore, the depiction of the flow cell 200 is symbolic in nature, and the flow cell 200 symbolically depicts various lanes and tiles therewithin, without illustrating various other components therewithin. Fig. 2 illustrates a top view of the flow cell 200.

In an embodiment, the flow cell 200 is divided or partitioned in a plurality of lanes, such as lanes 202a, 202b, ..., 202P, *i.e.,* P number of lanes. In the example of Fig. 2, the flow cell 200 is illustrated to include 8 lanes, *i.e.,* P = 8 in this example, although the number of lanes within a flow cell is implementation specific.

In an embodiment, individual lanes 202 are further partitioned into non-overlapping regions called "tiles" 212. For example, Fig. 2 illustrates a magnified view of a section 208 of an example lane. The section 208 is illustrated to comprise a plurality of tiles 212.

In an example, each lane 202 comprises one or more columns of tiles. For example, in Fig. 2, each lane 202 comprises two corresponding columns of tiles 212, as illustrated within the magnified section 208. A number of tiles within each column of tiles within each lane is implementation specific, and in one example, there can be 50 tiles, 60 tiles, 100 tiles, or another appropriate number of tiles in each column of tiles within each lane.

Each tile comprises a corresponding plurality of clusters. During the sequencing procedure, the clusters and their surrounding background on the tiles are imaged. For example, Fig. 2 illustrates example clusters 216 within an example tile.

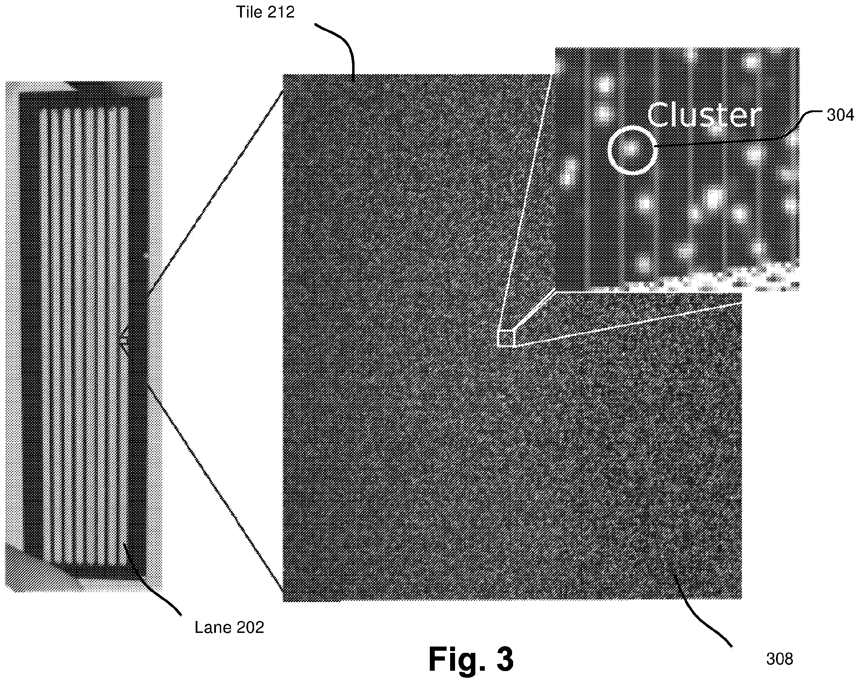

Fig. 3 illustrates an example Illumina GA-IIx™ flow cell with eight lanes, and also illustrates a zoom-in on one tile and its clusters and their surrounding background. For example, there are a hundred tiles per lane in Illumina Genome Analyzer II and sixty-eight tiles per lane in Illumina HiSeq2000. A tile 212 holds hundreds of thousands to millions of clusters. In Fig. 3, an image generated from a tile with clusters shown as bright spots is shown at 308 (*e.g.,* 308 is a magnified image view of a tile), with an example cluster 304 labelled. A cluster 304 comprises approximately one thousand identical copies of a template molecule, though clusters vary in size and shape. The clusters are grown from the template molecule, prior to the sequencing run, by bridge amplification of the input library. The purpose of the amplification and cluster growth is to increase the intensity of the emitted signal since the imaging device cannot reliably sense a single fluorophore. However, the physical distance of the DNA fragments within a cluster 304 is small, so the imaging device perceives the cluster of fragments as a single spot 304.

The clusters and the tiles are discussed in further details with respect to U.S. Nonprovisional Patent Application No. 16/825,987, titled "TRAINING DATA GENERATION FOR ARTIFICIAL INTELLIGENCE-BASED SEQUENCING," filed 20 March 2020 (Attorney Docket No. ILLM 1008-16/IP-1693-US).

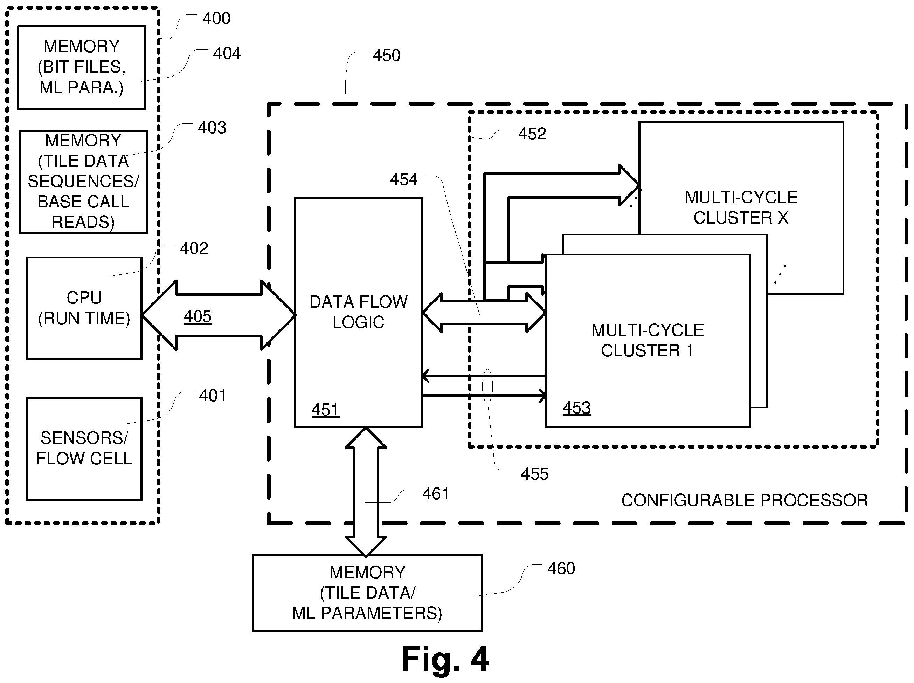

**Fig. 4** is a simplified block diagram of the system for analysis of sensor data from a sequencing system, such as base call sensor outputs (*e.g.*, see Fig. 1). In the example of Fig. 4, the system includes a sequencing machine 400 and a configurable processor 450. The configurable processor 450 can execute a neural network-based base caller in coordination with a runtime program executed by a host processor, such as a central processing unit (CPU) 402. The sequencing machine 400 comprises base call sensors and flow cell 401 (*e.g.*, discussed with respect to Figs. 1-3). The flow cell can comprise one or more tiles in which clusters of genetic material are exposed to a sequence of analyte flows used to cause reactions in the clusters to identify the bases in the genetic material, as discussed with respect to Figs. 1-3. The sensors sense the reactions for each cycle of the sequence in each tile of the flow cell to provide tile data. Examples of this technology are described in more detail below. Genetic sequencing is a data intensive operation, which translates base call sensor data into sequences of base calls for each cluster of genetic material sensed in during a base call operation.

The system in this example includes the CPU 402 which executes a runtime program to coordinate the base call operations, memory 403 to store sequences of arrays of tile data, base call reads produced by the base calling operation, and other information used in the base call operations. Also, in this illustration the system includes memory 404 to store a configuration file (or files), such as FPGA bit files, and model parameters for the neural network used to configure and reconfigure the configurable processor 450 and execute the neural network. The sequencing machine 400 can include a program for configuring a configurable processor and in some embodiments a reconfigurable processor to execute the neural network.

The sequencing machine 400 is coupled by a bus 405 to the configurable processor 450. The bus 405 can be implemented using a high throughput technology, such as in one example bus technology compatible with the PCIe standards (Peripheral Component Interconnect Express) currently maintained and developed by the PCI-SIG (PCI Special Interest Group). Also, in this example, a memory 460 is coupled to the configurable processor 450 by bus 461. The memory 460 can be on-board memory, disposed on a circuit board with the configurable processor 450. The memory 460 is used for high-speed access by the configurable processor 450 of working data used in the base call operation. The bus 461 can also be implemented using a high throughput technology, such as bus technology compatible with the PCIe standards.

Configurable processors, including Field Programmable Gate Arrays (FPGAs), Coarse Grained Reconfigurable Arrays (CGRAs), and other configurable and reconfigurable devices, can be configured to implement a variety of functions more efficiently or faster than might be achieved using a general-purpose processor executing a computer program. Configuration of configurable processors involves compiling a functional description to produce a configuration file, referred to sometimes as a bitstream or bit file, and distributing the configuration file to the configurable elements on the processor.

The configuration file defines the logic functions to be executed by the configurable processor, by configuring the circuit to set data flow patterns, use of distributed memory and other on-chip memory resources, lookup table contents, operations of configurable logic blocks and configurable execution units like multiply-and-accumulate units, configurable interconnects and other elements of the configurable array. A configurable processor is reconfigurable if the configuration file may be changed in the field, by changing the loaded configuration file. For example, the configuration file may be stored in volatile SRAM elements, in non-volatile read-write memory elements, and in combinations of the same, distributed among the array of configurable elements on the configurable or reconfigurable processor. A variety of commercially available configurable processors are suitable for use in a base calling operation as described herein. Examples include commercially available products such as Xilinx Alveo™ U200, Xilinx Alveo™ U250, Xilinx Alveo™ U280, Intel/Altera Stratix™ GX2800, Intel/Altera Stratix™ GX2800, and Intel Stratix™ GX10M. In some examples, a host CPU can be implemented on the same integrated circuit as the configurable processor.

Embodiments described herein implement the multi-cycle neural network using a configurable processor 450. The configuration file for a configurable processor can be implemented by specifying the logic functions to be executed using a high-level description language (HDL) or a register transfer level (RTL) language specification. The specification can be compiled using the resources designed for the selected configurable processor to generate the configuration file. The same or similar specification can be compiled for the purposes of generating a design for an application-specific integrated circuit which may not be a configurable processor.

Alternatives for the configurable processor, in all embodiments described herein, therefore include a configured processor comprising an application specific ASIC or special purpose integrated circuit or set of integrated circuits, or a system-on-a-chip SOC device, configured to execute a neural network based base call operation as described herein.

In general, configurable processors and configured processors described herein, as configured to execute runs of a neural network, are referred to herein as neural network processors.

The configurable processor 450 is configured in this example by a configuration file loaded using a program executed by the CPU 402, or by other sources, which configures the array of configurable elements on the configurable processor 454 to execute the base call function. In this example, the configuration includes data flow logic 451 which is coupled to the buses 405 and 461 and executes functions for distributing data and control parameters among the elements used in the base call operation.

Also, the configurable processor 450 is configured with base call execution logic 452 to execute a multi-cycle neural network. The logic 452 comprises a plurality of multi-cycle execution clusters (*e.g.*, 453) which, in this example, includes multi-cycle cluster 1 through multi-cycle cluster X. The number of multi-cycle clusters can be selected according to a trade-off involving the desired throughput of the operation, and the available resources on the configurable processor.

The multi-cycle clusters are coupled to the data flow logic 451 by data flow paths 454 implemented using configurable interconnect and memory resources on the configurable processor. Also, the multi-cycle clusters are coupled to the data flow logic 451 by control paths 455 implemented using configurable interconnect and memory resources for example on the configurable processor, which provide control signals indicating available clusters, readiness to provide input units for execution of a run of the neural network to the available clusters, readiness to provide trained parameters for the neural network, readiness to provide output patches of base call classification data, and other control data used for execution of the neural network.

The configurable processor is configured to execute runs of a multi-cycle neural network using trained parameters to produce classification data for sensing cycles of the base flow operation. A run of the neural network is executed to produce classification data for a subject sensing cycle of the base call operation. A run of the neural network operates on a sequence including a number N of arrays of tile data from respective sensing cycles of N sensing cycles, where the N sensing cycles provide sensor data for different base call operations for one base position per operation in time sequence in the examples described herein. Optionally, some of the N sensing cycles can be out of sequence if the needed according to a particular neural network model being executed. The number N can be any number greater than one. In some examples described herein, sensing cycles of the N sensing cycles represent a set of sensing cycles for at least one sensing cycle preceding the subject sensing cycle and at least one sensing cycle following the subject cycle in time sequence. Examples are described herein in which the number N is an integer equal to or greater than five.

The data flow logic 451 is configured to move tile data and at least some trained parameters of the model from the memory 460 to the configurable processor for runs of the neural network, using input units for a given run including tile data for spatially aligned patches of the N arrays. The input units can be moved by direct memory access operations in one DMA operation, or in smaller units moved during available time slots in coordination with the execution of the neural network deployed.

Tile data for a sensing cycle as described herein can comprise an array of sensor data having one or more features. For example, the sensor data can comprise two images which are analyzed to identify one of four bases at a base position in a genetic sequence of DNA, RNA, or other genetic material. The tile data can also include metadata about the images and the sensors. For example, in embodiments of the base calling operation, the tile data can comprise information about alignment of the images with the clusters such as distance from center information indicating the distance of each pixel in the array of sensor data from the center of a cluster of genetic material on the tile.

During execution of the multi-cycle neural network as described below, tile data can also include data produced during execution of the multi-cycle neural network, referred to as intermediate data, which can be reused rather than recomputed during a run of the multi-cycle neural network. For example, during execution of the multi-cycle neural network, the data flow logic can write intermediate data to the memory 460 in place of the sensor data for a given patch of an array of tile data. Embodiments like this are described in more detail below.

As illustrated, a system is described for analysis of base call sensor output, comprising memory (*e.g.*, 460) accessible by the runtime program storing tile data including sensor data for a tile from sensing cycles of a base calling operation. Also, the system includes a neural network processor, such as configurable processor 450 having access to the memory. The neural network processor is configured to execute runs of a neural network using trained parameters to produce classification data for sensing cycles. As described herein, a run of the neural network is operating on a sequence of N arrays of tile data from respective sensing cycles of N sensing cycles, including a subject cycle, to produce the classification data for the subject cycle. The data flow logic 451 is provided to move tile data and the trained parameters from the memory to the neural network processor for runs of the neural network using input units including data for spatially aligned patches of the N arrays from respective sensing cycles of N sensing cycles.

Also, a system is described in which the neural network processor has access to the memory, and includes a plurality of execution clusters, the execution logic clusters in the plurality of execution clusters configured to execute a neural network. The data flow logic has access to the memory and to execution clusters in the plurality of execution clusters, to provide input units of tile data to available execution clusters in the plurality of execution clusters, the input units including a number N of spatially aligned patches of arrays of tile data from respective sensing cycles, including a subject sensing cycle, and to cause the execution clusters to apply the N spatially aligned patches to the neural network to produce output patches of classification data for the spatially aligned patch of the subject sensing cycle, where N is greater than 1.

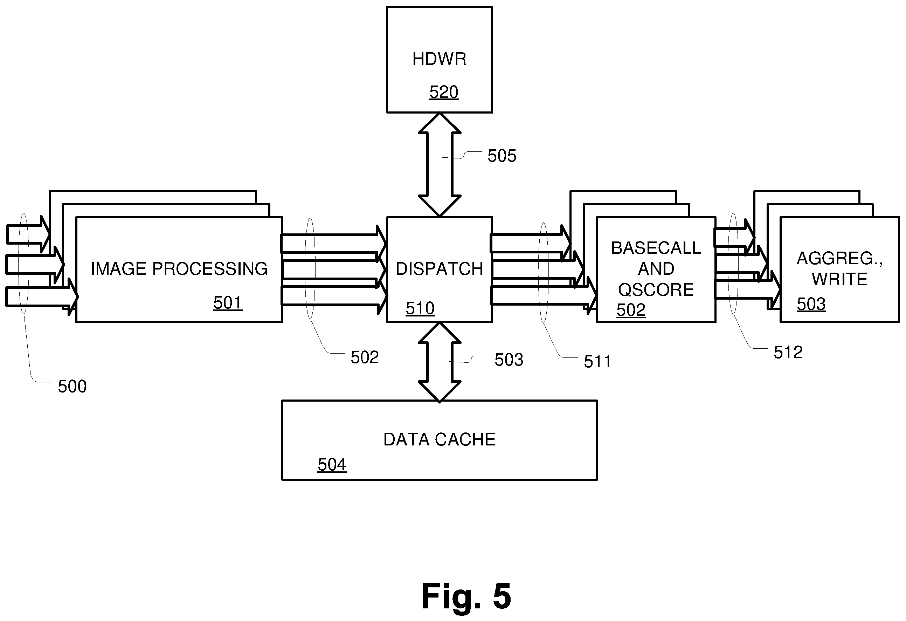

**Fig. 5** is a simplified diagram showing aspects of the base calling operation, including functions of a runtime program executed by a host processor. In this diagram, the output of image sensors from a flow cell (such as those illustrated in Figs. 1-2) are provided on lines 500 to image processing threads 501, which can perform processes on images such as resampling, alignment and arrangement in an array of sensor data for the individual tiles, and can be used by processes which calculate a tile cluster mask for each tile in the flow cell, which identifies pixels in the array of sensor data that correspond to clusters of genetic material on the corresponding tile of the flow cell. To compute a cluster mask, one example algorithm is based on a process to detect clusters which are unreliable in the early sequencing cycles using a metric derived from the softmax output, and then the data from those wells/clusters is discarded, and no output data is produced for those clusters. For example, a process can identify clusters with high reliability during the first N1 (*e.g.*, 25) base-calls, and reject the others. Rejected clusters might be polyclonal or very weak intensity or obscured by fiducials. This procedure can be performed on the host CPU. In alternative implementations, this information would potentially be used to identify the necessary clusters of interest to be passed back to the CPU, thereby limiting the storage required for intermediate data.

The outputs of the image processing threads 501 are provided on lines 502 to a dispatch logic 510 in the CPU which routes the arrays of tile data to a data cache 504 on a high-speed bus 503, or on high-speed bus 505 to the multi-cluster neural network processor hardware 520, such as the configurable processor of Fig. 4, according to the state of the base calling operation. The hardware 520 returns classification data output by the neural network to the dispatch logic 510, which passes the information to the data cache 504, or on lines 511 to threads 502 that perform base call and quality score computations using the classification data, and can arrange the data in standard formats for base call reads. The outputs of the threads 502 that perform base calling and quality score computations are provided on lines 512 to threads 503 that aggregate the base call reads, perform other operations such as data compression, and write the resulting base call outputs to specified destinations for utilization by the customers.

In some embodiments, the host can include threads (not shown) that perform final processing of the output of the hardware 520 in support of the neural network. For example, the hardware 520 can provide outputs of classification data from a final layer of the multi-cluster neural network. The host processor can execute an output activation function, such as a softmax function, over the classification data to configure the data for use by the base call and quality score threads 502. Also, the host processor can execute input operations (not shown), such as resampling, batch normalization or other adjustments of the tile data prior to input to the hardware 520.

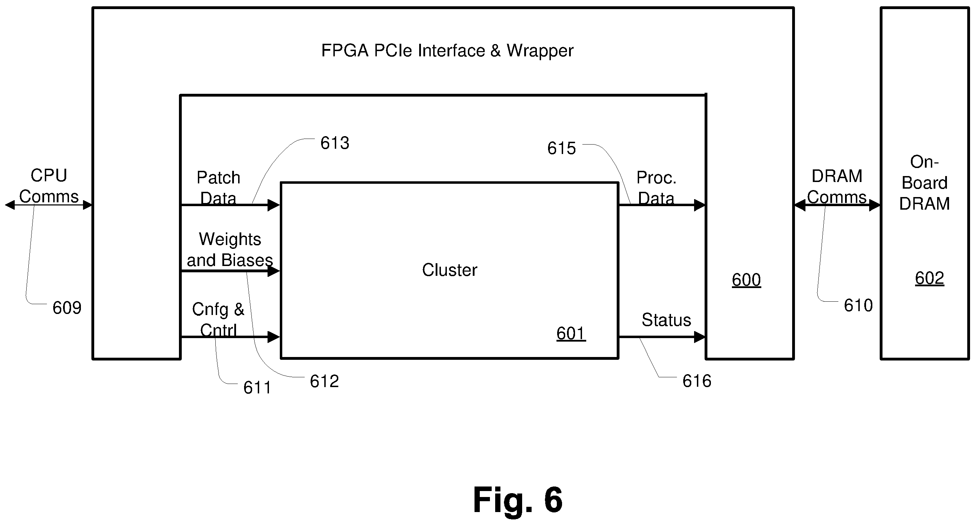

**Fig. 6** is a simplified diagram of a configuration of a configurable processor such as that of Fig. 4. In Fig. 6, the configurable processor comprises in FPGA with a plurality of high speed PCIe interfaces. The FPGA is configured with a wrapper 600 which comprises the data flow logic described with reference to Fig. 1. The wrapper 600 manages the interface and coordination with a runtime program in the CPU across the CPU communication link 609 and manages communication with the on-board DRAM 602 (*e.g.,* memory 460) via DRAM communication link 610. The data flow logic in the wrapper 600 provides patch data retrieved by traversing the arrays of tile data on the on-board DRAM 602 for the number N cycles to a cluster 601 and retrieves process data 615 from the cluster 601 for delivery back to the on-board DRAM 602. The wrapper 600 also manages transfer of data between the on-board DRAM 602 and host memory, for both the input arrays of tile data, and for the output patches of classification data. The wrapper transfers patch data on line 613 to the allocated cluster 601. The wrapper provides trained parameters, such as weights and biases on line 612 to the cluster 601 retrieved from the on-board DRAM 602. The wrapper provides configuration and control data on line 611 to the cluster 601 provided from, or generated in response to, the runtime program on the host via the CPU communication link 609. The cluster can also provide status signals on line 616 to the wrapper 600, which are used in cooperation with control signals from the host to manage traversal of the arrays of tile data to provide spatially aligned patch data, and to execute the multi-cycle neural network over the patch data using the resources of the cluster 601.

As mentioned above, there can be multiple clusters on a single configurable processor managed by the wrapper 600 configured for executing on corresponding ones of multiple patches of the tile data. Each cluster can be configured to provide classification data for base calls in a subject sensing cycle using the tile data of multiple sensing cycles described herein.

In examples of the system, model data, including kernel data like filter weights and biases can be sent from the host CPU to the configurable processor, so that the model can be updated as a function of cycle number. A base calling operation can comprise, for a representative example, on the order of hundreds of sensing cycles. Base calling operation can include paired end reads in some embodiments. For example, the model trained parameters may be updated once every 20 cycles (or other number of cycles), or according to update patterns implemented for particular systems and neural network models. In some embodiments including paired end reads in which a sequence for a given string in a genetic cluster on a tile includes a first part extending from a first end down (or up) the string, and a second part extending from a second end up (or down) the string, the trained parameters can be updated on the transition from the first part to the second part.

In some examples, image data for multiple cycles of sensing data for a tile can be sent from the CPU to the wrapper 600. The wrapper 600 can optionally do some pre-processing and transformation of the sensing data and write the information to the on-board DRAM 602. The input tile data for each sensing cycle can include arrays of sensor data including on the order of 4000 x 3000 pixels per sensing cycle per tile or more, with two features representing colors of two images of the tile, and one or two bytes per feature per pixel. For an embodiment in which the number N is three sensing cycles to be used in each run of the multi-cycle neural network, the array of tile data for each run of the multi-cycle neural network can consume on the order of hundreds of megabytes per tile. In some embodiments of the system, the tile data also includes an array of DFC data, stored once per tile, or other type of metadata about the sensor data and the tiles.

In operation, when a multi-cycle cluster is available, the wrapper allocates a patch to the cluster. The wrapper fetches a next patch of tile data in the traversal of the tile and sends it to the allocated cluster along with appropriate control and configuration information. The cluster can be configured with enough memory on the configurable processor to hold a patch of data including patches from multiple cycles in some systems, that is being worked on in place, and a patch of data that is to be worked on when the current patch of processing is finished using a ping-pong buffer technique or raster scanning technique in various embodiments.

When an allocated cluster completes its run of the neural network for the current patch and produces an output patch, it will signal the wrapper. The wrapper will read the output patch from the allocated cluster, or alternatively the allocated cluster will push the data out to the wrapper. Then the wrapper will assemble output patches for the processed tile in the DRAM 602. When the processing of the entire tile has been completed, and the output patches of data transferred to the DRAM, the wrapper sends the processed output array for the tile back to the host/CPU in a specified format. In some embodiments, the on-board DRAM 602 is managed by memory management logic in the wrapper 600. The runtime program can control the sequencing operations to complete analysis of all the arrays of tile data for all the cycles in the run in a continuous flow to provide real time analysis.

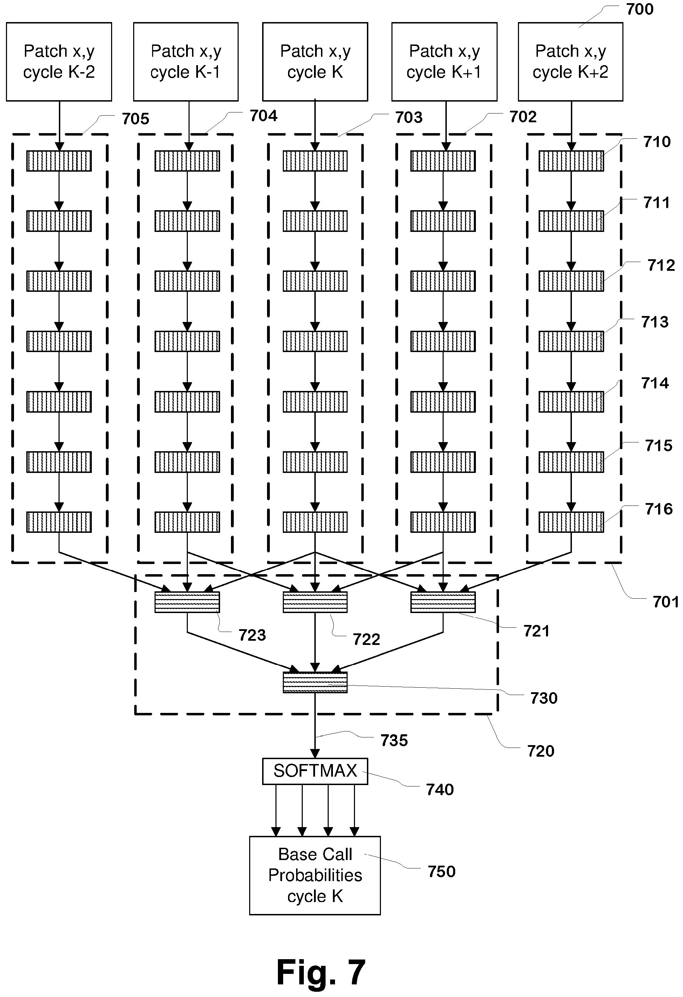

**Fig. 7** is a diagram of a multi-cycle neural network model which can be executed using the system described herein. The example shown in Fig. 7 can be referred to as a five-cycle input, one-cycle output neural network. The inputs to the multi-cycle neural network model include five spatially aligned patches (*e.g.*, 700) from the tile data arrays of five sensing cycles of a given tile. Spatially aligned patches have the same aligned row and column dimensions (x,y) as other patches in the set, so that the information relates to the same clusters of genetic material on the tile in sequence cycles. In this example, a subject patch is a patch from the array of tile data for cycle K. The set of five spatially aligned patches includes a patch from cycle K-2 preceding the subject patch by two cycles, a patch from cycle K-1 preceding the subject patch by one cycle, a patch from cycle K+1 following the patch from the subject cycle by one cycle, and a patch from cycle K+2 following the patch from the subject cycle by two cycles.

The model includes a segregated stack 701 of layers of the neural network for each of the input patches. Thus, stack 701 receives as input, tile data for the patch from cycle K+2, and is segregated from the stacks 702, 703, 704, and 705 so they do not share input data or intermediate data. In some embodiments, all of the stacks 710-705 can have identical models, and identical trained parameters. In other embodiments, the models and trained parameters may be different in the different stacks. Stack 702 receives as input, tile data for the patch from cycle K+1. Stack 703 receives as input, tile data for the patch from cycle K. Stack 704 receives as input, tile data for the patch from cycle K-1. Stack 705 receives as input, tile data for the patch from cycle K-2. The layers of the segregated stacks each execute a convolution operation of a kernel including a plurality of filters over the input data for the layer. As in the example above, the patch 700 may include three features. The output of the layer 710 may include many more features, such as 10 to 20 features. Likewise, the outputs of each of layers 711 to 716 can include any number of features suitable for a particular implementation. The parameters of the filters are trained parameters for the neural network, such as weights and biases. The output feature set (intermediate data) from each of the stacks 701-705 is provided as input to an inverse hierarchy 720 of temporal combinatorial layers, in which the intermediate data from the multiple cycles is combined. In the example illustrated, the inverse hierarchy 720 includes a first layer including three combinatorial layers 721, 722, 723, each receiving intermediate data from three of the segregated stacks, and a final layer including one combinatorial layer 730 receiving intermediate data from the three temporal layers 721, 722, 723.

The output of the final combinatorial layer 730 is an output patch of classification data for clusters located in the corresponding patch of the tile from cycle K. The output patches can be assembled into an output array classification data for the tile for cycle K. In some embodiments, the output patch may have sizes and dimensions different from the input patches. In some embodiments, the output patch may include pixel-by-pixel data that can be filtered by the host to select cluster data.

The output classification data can then be applied to a softmax function 740 (or other output activation function) optionally executed by the host, or on the configurable processor, depending on the particular implementation. An output function different from softmax could be used (*e.g.*, making a base call output parameter according to largest output, then use a learned nonlinear mapping using context/network outputs to give base quality).

Finally, the output of the softmax function 740 can be provided as base call probabilities for cycle K (750) and stored in host memory to be used in subsequent processing. Other systems may use another function for output probability calculation, *e.g.*, another nonlinear model.

The neural network can be implemented using a configurable processor with a plurality of execution clusters so as complete evaluation of one tile cycle within the duration of the time interval, or close to the duration of the time interval, of one sensing cycle, effectively providing the output data in real time. Data flow logic can be configured to distribute input units of tile data and trained parameters to the execution clusters, and to distribute output patches for aggregation in memory.

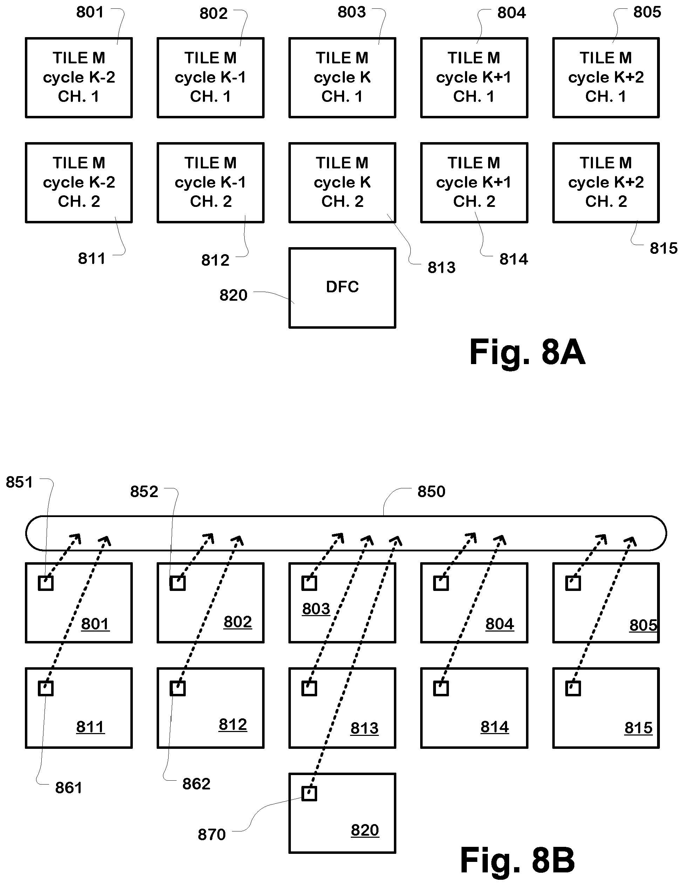

Input units of data for a five-cycle input, one-cycle output neural network like that of Fig. 7 are described with reference to Figs. 8A and 8B for a base call operation using two-channel sensor data. For example, for a given base in a genetic sequence, the base call operation can execute two flows of analyte and two reactions that generate two channels of signals, such as images, which can be processed to identify which one of four bases is located at a current position in the genetic sequence for each cluster of genetic material. In other systems, a different number of channels of sensing data may be utilized. For example, base calling can be performed utilizing one-channel methods and systems. U.S. Patent Application Publication No. 2013/0079232 discusses base calling using various number of channels, such as one-channel, two-channels, or four-channels.

**Fig. 8A** shows arrays of tile data for five cycles for a given tile, tile M, used for the purposes of executing a five-cycle input, one-cycle output neural network. The five-cycle input tile data in this example can be written to the on-board DRAM, or other memory in the system which can be accessed by the data flow logic and, for cycle K-2, includes an array 801 for channel 1 and an array 811 for channel 2, for cycle K-1, an array 802 for channel 1 and an array 812 for channel 2, for cycle K, an array 803 for channel 1 and an array 813 for channel 2, for cycle K+1, an array 804 for channel 1 and an array 814 for channel 2, for cycle K+2, an array 805 for channel 1 and an array 815 for channel 2. Also an array 820 of metadata for the tile can be written once in the memory, in this case a DFC file, included for use as input to the neural network along with each cycle.

Although Fig. 8A discusses two-channel base calling operations, using two channels is merely an example, and base calling can be performed using any other appropriate number of channels. For example, U.S. Patent Application Publication No. 2013/0079232 discusses base calling using various number of channels, such as one-channel, two-channels, or four-channels, or another appropriate number of channels.

The data flow logic composes input units, which can be understood with reference to Fig. 8B, of tile data that includes spatially aligned patches of the arrays of tile data for each execution cluster configured to execute a run of the neural network over an input patch. An input unit for an allocated execution cluster is composed by the data flow logic by reading spatially aligned patches (*e.g.*, 851, 852, 861, 862, 870) from each of the arrays 801-805, 811, 815, 820 of tile data for the five input cycles, and delivering them via data paths (schematically 850) to memory on the configurable processor configured for use by the allocated execution cluster. The allocated execution cluster executes a run of the five-cycle input/one-cycle output neural network, and delivers an output patch for the subject cycle K of classification data for the same patch of the tile in the subject cycle K.

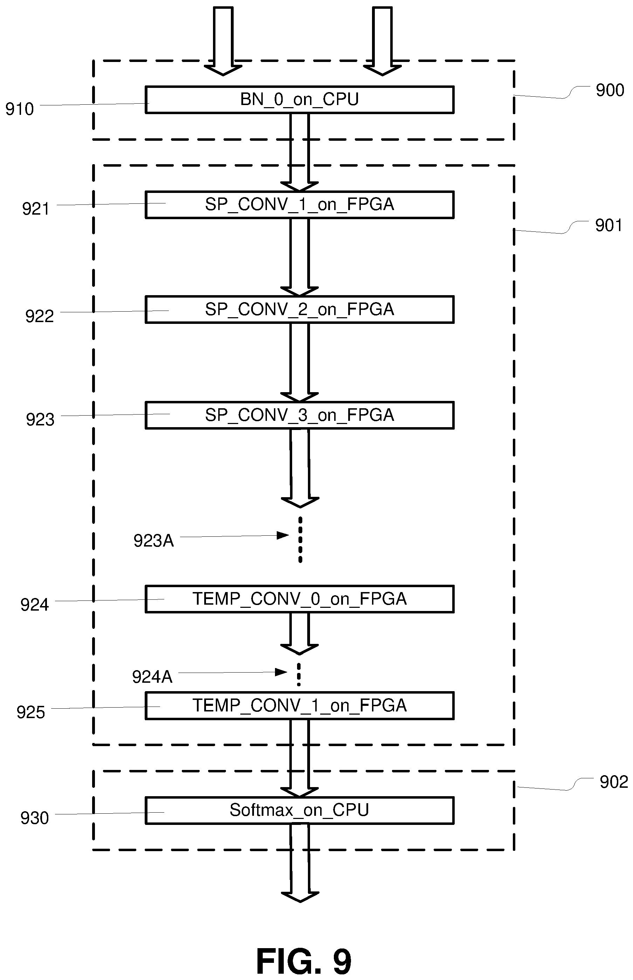

**Fig. 9** is a simplified representation of a stack of a neural network usable in a system like that of Fig. 7 (*e.g.,* 701 and 720). In this example, some functions of the neural network (*e.g.,* 900, 902) are executed on the host, and other portions of the neural network (*e.g.,* 901) are executed on the configurable processor.

In an example, a first function can be batch normalization (layer 910) formed on the CPU. However, in another example, batch normalization as a function may be fused into one or more layers, and no separate batch normalization layer may be present.

A number of spatial, segregated convolution layers are executed as a first set of convolution layers of the neural network, as discussed above on the configurable processor. In this example, the first set of convolution layers applies 2D convolutions spatially.

As shown in Fig. 9, a first spatial convolution 921 is executed, followed by a second spatial convolution 922, followed by a third spatial convolution 923, and so on for a number L/2 of spatially segregated neural network layers in each stack (L is described with reference to Figure 7). As indicated at 923A, the number of spatial layers can be any practical number, which for context may range from a few to more than 20 in different embodiments.

For SP_CONV_ 0, kernel weights are stored for example in a (1,6,6,3,L) structure since there are 3 input channels to this layer. In this example, the "6" in this structure is due to storing coefficients in the transformed Winograd domain (the kernel size is 3x3 in the spatial domain but expands in the transform domain).

For other SP_CONV layers, kernel weights are stored for this example in a (1,6,6 L) structure since there are K(=L) inputs and outputs for each of these layers.

The outputs of the stack of spatial layers are provided to temporal layers, including convolution layers 924, 925 executed on the FPGA. Layers 924 and 925 can be convolution layers applying 1D convolutions across cycles. As indicated at 924A, the number of temporal layers can be any practical number, which for context may range from a few to more than 20 in different embodiments.

The first temporal layer, TEMP_CONV_0 layer 824, reduces the number of cycle channels from 5 to 3, as illustrated in Fig. 7. The second temporal layer, layer 925, reduces the number of cycle channels from 3 to 1 as illustrated in Fig. 7, and reduces the number of feature maps to four outputs for each pixel, representing confidence in each base call.

The output of the temporal layers is accumulated in output patches and delivered to the host CPU to apply for example, a softmax function 930, or other function to normalize the base call probabilities.

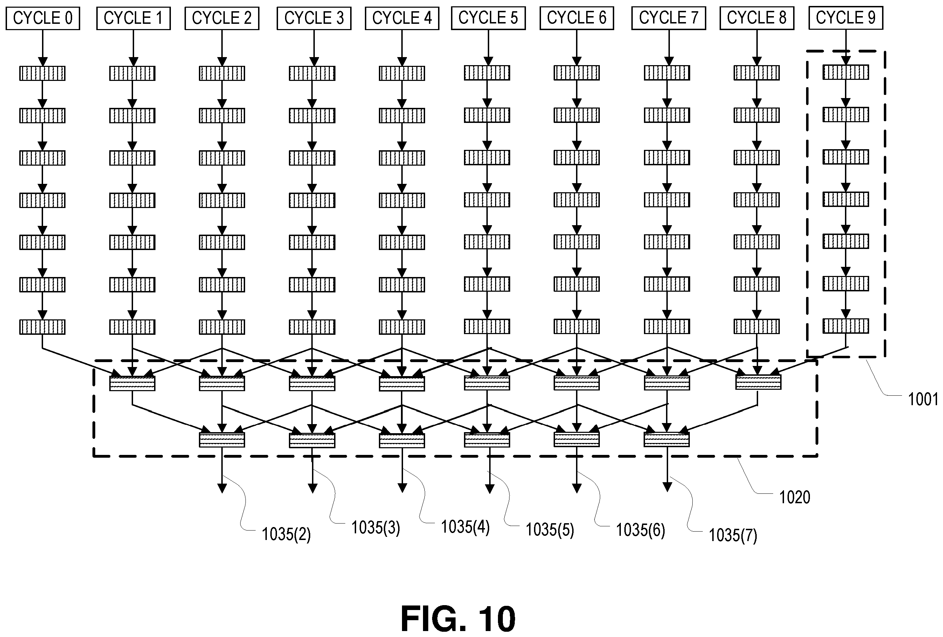

**Fig. 10** illustrates an alternative implementation showing a 10-input, six-output neural network which can be executed for a base calling operation. In this example, tile data for spatially aligned input patches from cycles 0 to 9 are applied to segregated stacks of spatial layers, such as stack 1001 for cycle 9. The outputs of the segregated stacks are applied to an inverse hierarchical arrangement of temporal stacks 1020, having outputs 1035(2) through 1035(7) providing base call classification data for subject cycles 2 through 7.

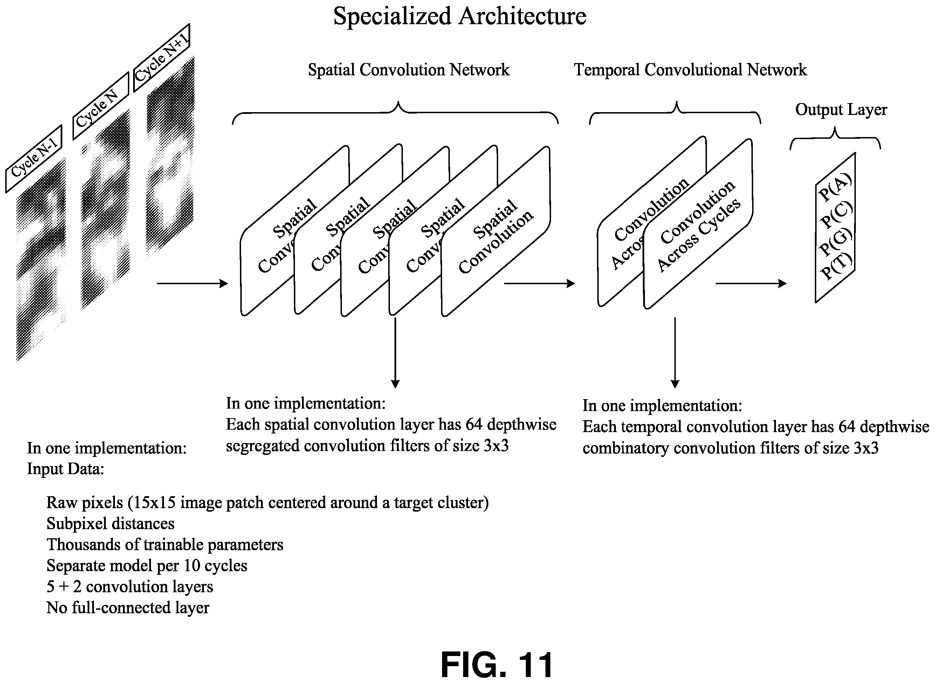

**Fig. 11** illustrates one implementation of the specialized architecture of the neural network-based base caller *(e.g.,* Fig. 7) that is used to segregate processing of data for different sequencing cycles. The motivation for using the specialized architecture is described first.

The neural network-based base caller processes data for a current sequencing cycle, one or more preceding sequencing cycles, and one or more successive sequencing cycles. Data for additional sequencing cycles provides sequence-specific context. The neural network-based base caller learns the sequence-specific context during training and base call them. Furthermore, data for pre and post sequencing cycles provides second order contribution of pre-phasing and phasing signals to the current sequencing cycle.

Images captured at different sequencing cycles and in different image channels are misaligned and have residual registration error with respect to each other. To account for this misalignment, the specialized architecture comprises spatial convolution layers that do not mix information between sequencing cycles and only mix information within a sequencing cycle.

Spatial convolution layers use so-called "segregated convolutions" that operationalize the segregation by independently processing data for each of a plurality of sequencing cycles through a "dedicated, non-shared" sequence of convolutions. The segregated convolutions convolve over data and resulting feature maps of only a given sequencing cycle, *i.e.,* intra-cycle, without convolving over data and resulting feature maps of any other sequencing cycle.

Consider, for example, that the input data comprises (i) current data for a current (time $t$) sequencing cycle to be base called, (ii) previous data for a previous (time $t$-1) sequencing cycle, and (iii) next data for a next (time $t$+1) sequencing cycle. The specialized architecture then initiates three separate data processing pipelines (or convolution pipelines), namely, a current data processing pipeline, a previous data processing pipeline, and a next data processing pipeline. The current data processing pipeline receives as input the current data for the current (time t) sequencing cycle and independently processes it through a plurality of spatial convolution layers to produce a so-called "current spatially convolved representation" as the output of a final spatial convolution layer. The previous data processing pipeline receives as input the previous data for the previous (time $t$-1) sequencing cycle and independently processes it through the plurality of spatial convolution layers to produce a so-called "previous spatially convolved representation" as the output of the final spatial convolution layer. The next data processing pipeline receives as input the next data for the next (time $t$+1) sequencing cycle and independently processes it through the plurality of spatial convolution layers to produce a so-called "next spatially convolved representation" as the output of the final spatial convolution layer.

In some implementations, the current pipeline, one or more previous pipeline(s), and one or more next processing pipeline(s) are executed in parallel.

In some implementations, the spatial convolution layers are part of a spatial convolutional network (or subnetwork) within the specialized architecture.

The neural network-based base caller further comprises temporal convolution layers that mix information between sequencing cycles, *i.e.,* inter-cycles. The temporal convolution layers receive their inputs from the spatial convolutional network and operate on the spatially convolved representations produced by the final spatial convolution layer for the respective data processing pipelines.

The inter-cycle operability freedom of the temporal convolution layers emanates from the fact that the misalignment property, which exists in the image data fed as input to the spatial convolutional network, is purged out from the spatially convolved representations by the stack, or cascade, of segregated convolutions performed by the sequence of spatial convolution layers.

Temporal convolution layers use so-called "combinatory convolutions" that groupwise convolve over input channels in successive inputs on a sliding window basis. In one implementation, the successive inputs are successive outputs produced by a previous spatial convolution layer or a previous temporal convolution layer.

In some implementations, the temporal convolution layers are part of a temporal convolutional network (or subnetwork) within the specialized architecture. The temporal convolutional network receives its inputs from the spatial convolutional network. In one implementation, a first temporal convolution layer of the temporal convolutional network groupwise combines the spatially convolved representations between the sequencing cycles. In another implementation, subsequent temporal convolution layers of the temporal convolutional network combine successive outputs of previous temporal convolution layers.

The output of the final temporal convolution layer is fed to an output layer that produces an output. The output is used to base call one or more clusters at one or more sequencing cycles.

During a forward propagation, the specialized architecture processes information from a plurality of inputs in two stages. In the first stage, segregated convolutions are used to prevent mixing of information between the inputs. In the second stage, combinatory convolutions are used to mix information between the inputs. The results from the second stage are used to make a single inference for the plurality of inputs.

This is different than the batch mode technique where a convolution layer processes multiple inputs in a batch at the same time and makes a corresponding inference for each input in the batch. In contrast, the specialized architecture maps the plurality of inputs to the single inference. The single inference can comprise more than one prediction, such as a classification score for each of the four bases (A, C, T, and G).

In one implementation, the inputs have temporal ordering such that each input is generated at a different time step and has a plurality of input channels. For example, the plurality of inputs can include the following three inputs: a current input generated by a current sequencing cycle at time step (t), a previous input generated by a previous sequencing cycle at time step (t-1), and a next input generated by a next sequencing cycle at time step (t+1). In another implementation, each input is respectively derived from the current, previous, and next inputs by one or more previous convolution layers and includes k feature maps.

In one implementation, each input can include the following five input channels: a red image channel (in red), a red distance channel (in yellow), a green image channel (in green), a green distance channel (in purple), and a scaling channel (in blue). In another implementation, each input can include k feature maps produced by a previous convolution layer and each feature map is treated as an input channel. In yet another example, each input can have merely one channel, two channels, or another different number of channels U.S. Patent Application Publication No 2013/0079232 discusses base calling using various number of channels, such as one-channel, two-channels, or four-channels.

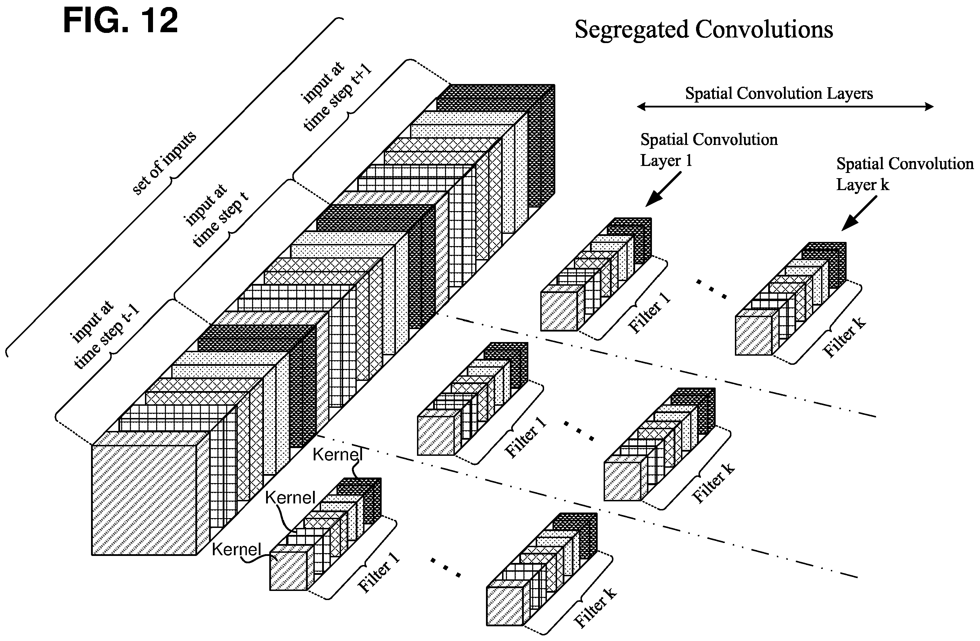

**Fig. 12** depicts one implementation of segregated layers, each of which can include convolutions. Segregated convolutions process the plurality of inputs at once by applying a convolution filter to each input in parallel. With the segregated convolutions, the convolution filter combines input channels in a same input and does not combine input channels in different inputs. In one implementation, a same convolution filter is applied to each input in parallel. In another implementation, a different convolution filter is applied to each input in parallel. In some implementations, each spatial convolution layer comprises a bank of k convolution filters, each of which applies to each input in parallel.

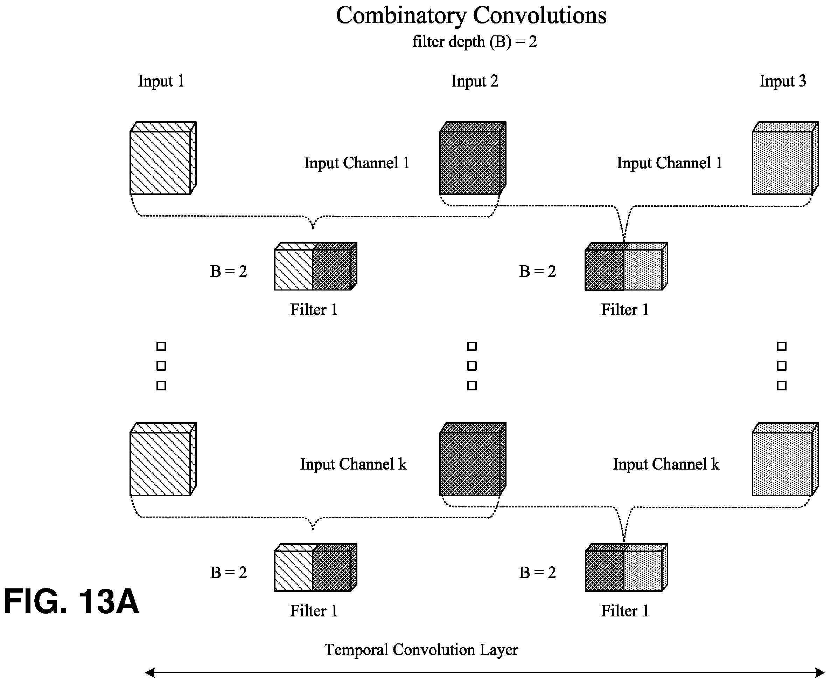

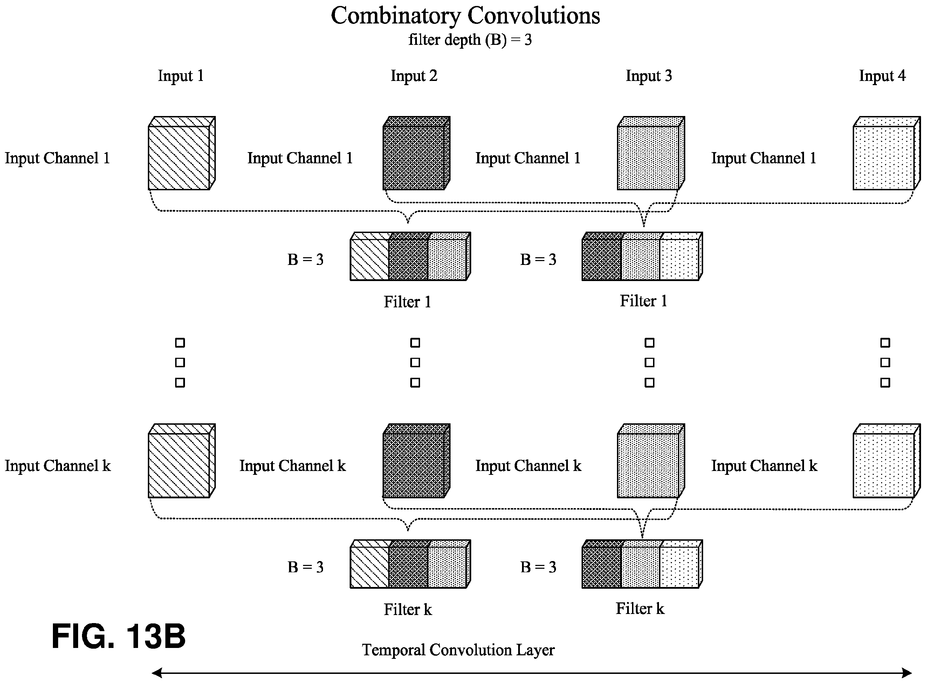

Fig. 13A depicts one implementation of combinatory layers, each of which can include convolutions. Fig. 13B depicts another implementation of the combinatory layers, each of which can include convolutions. Combinatory convolutions mix information between different inputs by grouping corresponding input channels of the different inputs and applying a convolution filter to each group. The grouping of the corresponding input channels and application of the convolution filter occurs on a sliding window basis. In this context, a window spans two or more successive input channels representing, for instance, outputs for two successive sequencing cycles. Since the window is a sliding window, most input channels are used in two or more windows.

In some implementations, the different inputs originate from an output sequence produced by a preceding spatial or temporal convolution layer. In the output sequence, the different inputs are arranged as successive outputs and therefore viewed by a next temporal convolution layer as successive inputs. Then, in the next temporal convolution layer, the combinatory convolutions apply the convolution filter to groups of corresponding input channels in the successive inputs.

In one implementation, the successive inputs have temporal ordering such that a current input is generated by a current sequencing cycle at time step ($t$), a previous input is generated by a previous sequencing cycle at time step ($t$-1), and a next input is generated by a next sequencing cycle at time step ($t$+1). In another implementation, each successive input is respectively derived from the current, previous, and next inputs by one or more previous convolution layers and includes $k$ feature maps.

In one implementation, each input can include the following five input channels: a red image channel (in red), a red distance channel (in yellow), a green image channel (in green), a green distance channel (in purple), and a scaling channel (in blue). In another implementation, each input can include k feature maps produced by a previous convolution layer and each feature map is treated as an input channel.

The depth $B$ of the convolution filter is dependent upon the number of successive inputs whose corresponding input channels are groupwise convolved by the convolution filter on a sliding window basis. In other words, the depth $B$ is equal to the number of successive inputs in each sliding window and the group size.

In Fig. 13A, corresponding input channels from two successive inputs are combined in each sliding window, and therefore $B$ = 2. In Fig. 13B, corresponding input channels from three successive inputs are combined in each sliding window, and therefore B = 3.

In one implementation, the sliding windows share a same convolution filter. In another implementation, a different convolution filter is used for each sliding window. In some implementations, each temporal convolution layer comprises a bank of $k$ convolution filters, each of which applies to the successive inputs on a sliding window basis.

Further detail of Figs. 4-10, and variations thereof, can be found in co-pending U.S. Nonprovisional Patent Application No. 17/176,147, titled "HARDWARE EXECUTION AND ACCELERATION OF ARTIFICIAL INTELLIGENCE-BASED BASE CALLER," filed February 15, 2021 (Attorney Docket No. ILLM 1020-2/IP-1866-US).

## Training of a base caller from scratch

A base calling system is trained to predict base calls of unknown analyte comprising base sequences. For example, the base calling system has a base caller comprising a neural network, which predicts base calls for the bases of the unknown analyte.

Training the neural network of the base calling system is challenging. This is especially true in absence of labelled training data to be used for training the base calling system. In some examples, a Real Time Analysis (RTA) System can be used to generate labelled training data, which may be used for training the base calling system. An example of the RTA system is discussed in U.S. Patent No. US10304189B2, titled "Data processing system and methods," issued 28 May 2019. However, if a system lacks an RTA or is not able to fully utilize the functionality of the RTA, generating initial labelled training data for training the neural network of the base calling system would be challenging.

This disclosure discusses a self-learned base caller, which generates initial labelled training data, trains itself using the labelled training data, generates further labelled training data using the at least partially trained base caller, trains itself using the further labelled training data, generates even further labelled training data, and iteratively repeats this process to adequately train the base caller. This iterative training and labelled training data generation process includes different stages, such as a single-oligo stage, multiple-oligo stages (such as a two-oligo stage, a three-oligo stage, and so on), followed by simple-organism stage, complex-organism stage, further complex-organism stage, and so on. Thus, a complexity and/or a length of the analyte used for the training and generation of labelled training data progressively and monotonically increases with the iterations, along with a complexity of the underlying neural network configuration of the base caller, as will be discussed in further detail herein in turn. Because the base caller is progressively self-trained, such a system obviates use of an RTA for generating labelled training data. Thus, although the base calling system discussed herein may include an RTA, the iterative training process discussed herein can be used in addition to, or instead of the RTA, to train the base caller.

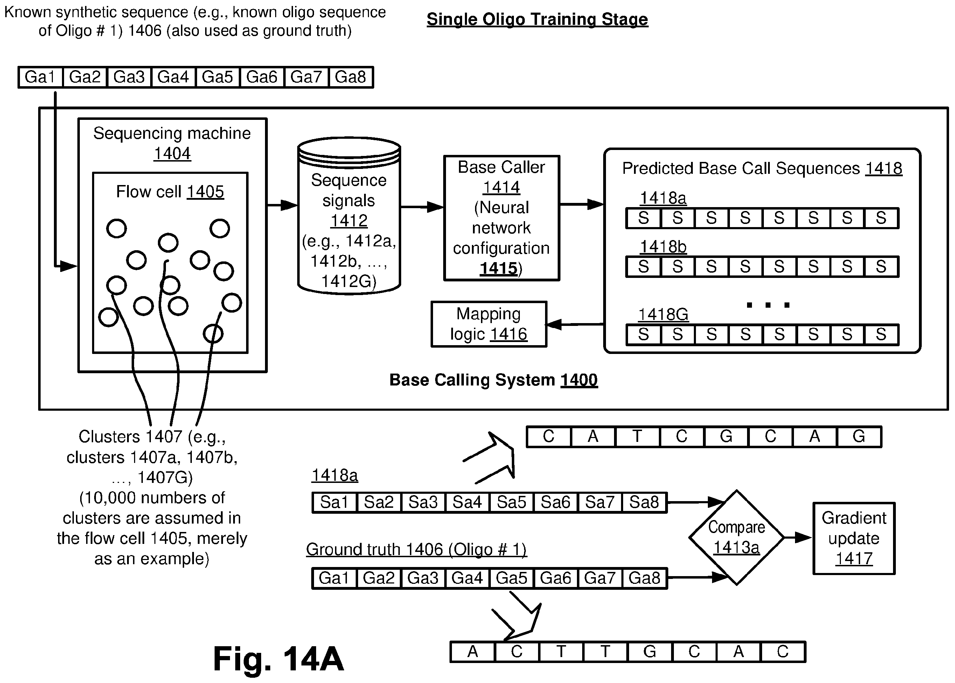

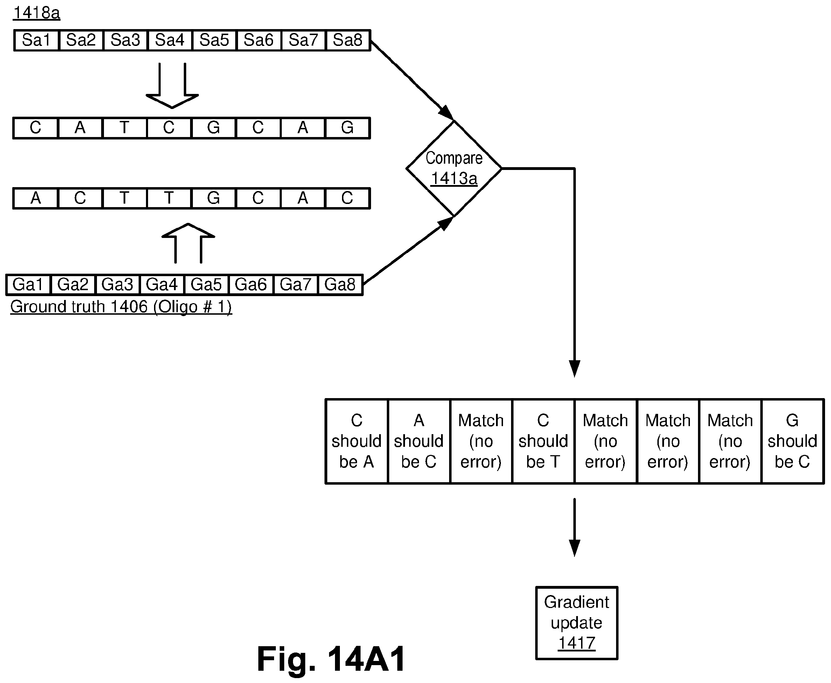

**Fig. 14A** illustrates a base calling system 1400 operating in a single-oligo training stage, to train a base caller 1414 comprising a neural network (NN) configuration 1415, using known synthetic sequence 1406.

In the example of Fig. 14A, the base calling system 1400 comprises a sequencing machine 1404, such as the sequencing machine 400 of Fig. 4. In an embodiment, the sequencing machine 1404 includes a biosensor (not illustrated in Fig. 14A) comprising a flow cell 1405, similar to the flow cell 102 of the biosensor 100 of Fig. 1.

As discussed with respect to Figs. 2, 3, and 6, the flow cell 1405 comprises a plurality of clusters 1407a, ..., 1407G. Specifically, in an example, the flow cell 1405 comprises a plurality of lanes of tiles, with each tile including a corresponding plurality of clusters, as discussed with respect to Fig. 2. In Fig. 14A, the flow cell 1405 is illustrated to include some such example clusters 1407a, ..., 1407G. During the base calling process, a base call (A, C, G, T) for every cluster at a specific cycle is predicted.

A typical flow cell 1405 can include multiple clusters 1407, such as thousands or even millions of clusters. Merely as an example, without limiting the scope of this disclosure, and for explaining some of the principles of this disclosure, is it assumed that there are 10,000 (or 10k) number of clusters 1407 in the flow cell 1405 (*i.e.*, G = 10,000), although a practical flow cell is likely to have a much higher number of such clusters.

In an example, the known synthetic sequence 1406 is used as analyte for the base calling operations during the single oligo training stage. In an example, the known synthetic sequence 1406 comprises a synthetically generated oligomer. Oligonucleotides are short DNA or RNA molecules, which are referred to as oligomers or simply as oligos, that have a wide range of applications in genetic testing, research, and forensics. Commonly made in the laboratory by solid-phase chemical synthesis, these small bits of nucleic acids can be manufactured as single-stranded molecules with any user-specified sequence, and so are vital for artificial gene synthesis, polymerase chain reaction (PCR), DNA sequencing, molecular cloning and as molecular probes. The length of the oligonucleotide is usually denoted by "-mer". For example, an oligonucleotide of six nucleotides (nt) is a hexamer, while one of 25 nt would usually be called a "25-mer". In an example, a size of the oligomer or oligo comprising the known synthetic sequence 1406 can have any appropriate number of bases, such as 8, 10, 12, or higher, and is implementation specific. Merely as an example, Fig. 14A illustrates the oligo of the known synthetic sequence 1406 comprising 8 bases.

The oligo referred to in Fig. 14A is labelled as Oligo # 1 (or Oligo number 1). As merely one unique oligo is used in Fig. 14A, the same Oligo # 1 is populated in individual clusters 1407. Thus, all the 10k clusters 1407 are populated with the same oligo sequence. That is, copies of the same oligo are populated in all the clusters 1407.

The sequencing machine 1404 generates sequence signals 1412a, ..., 1412G for corresponding ones of the plurality of clusters 1407a, ..., 1407G. For example, for a cluster 1407a, the sequencing machine 1404 generates corresponding sequence signal 1412a indicative of base sequences populated within the cluster 1407a for a series of sequencing cycles. Similarly, for a cluster 1407b, the sequencing machine 1404 generates corresponding sequence signal 1412b indicative of base sequences populated within the cluster 1407b for the series of sequencing cycles, and so on. The base caller 1414 receives the sequence signals 1412, and aims to call (*e.g.*, predict) the corresponding bases. In an example, the base caller 1414 comprising the NN configuration 1415 (and various other NN configurations discussed herein later) can be stored in the memories 404, 403, and/or 406, and executed on a host CPU (such as the CPU 402 of Fig. 4) and/or on configurable processor(s) (such as the configurable processor 450 of Fig. 4) that are local to the sequencing machine 400. In another example, the base caller 1414 can be stored remotely from the sequencing machine 400 (*e.g.*, stored in the cloud), and can be executed by remote processors (*e.g.*, executed in the cloud). For example, in the remote version of the base caller 1414, the base caller 1414 receives (*e.g.*, over a network, such as the Internet) the sequence signals 1412, performs base calling operations, and transmits the base calling results (*e.g.*, over the network, such as the Internet) to the sequencing machine 400.

In an example, the sequence signals 1412 comprise images captured by sensors (*e.g.,* light detectors, photodiodes), as discussed herein previously. Thus, at least some of the examples and embodiments discussed herein relate to iteratively training a base caller (such as the base caller 1414) that processes sequence signals comprising images. However, the principles of this disclosure are not limited to training any specific type of base callers that receives a specific type of sequence signals. For example, the iterative training discussed herein in this disclosure is independent of the type of base caller to be trained, or the type of sequence signals used. For example, the iterative training discussed herein in this disclosure can be used to train any other appropriate type of base callers, such as base callers configured to call bases based on sequence signals that does not comprise images. For example, the sequence signals can comprise electrical signals (*e.g.,* voltage signals, current signals), pH levels, and/or the like, and the iterative training methodology discussed herein can be applied to train a base caller receiving any such type of sequence signals.

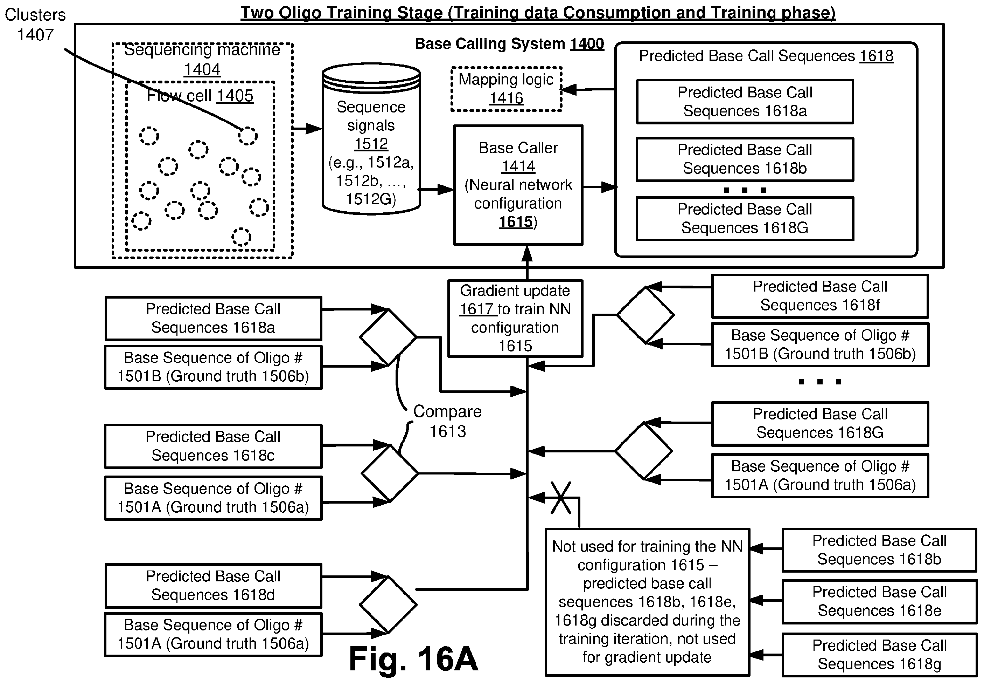

The neural network configuration 1415 is a convolution neural network (examples of which are illustrated in Figs. 7, 9, 10, 11, 12) that uses a relatively smaller number of layers and a relatively smaller number of parameters (*e.g.*, compared to some other neural network configurations discussed herein later, such as neural network configuration 1615 of Fig. 16A), as will be discussed in further detail herein.

The initially untrained base caller 1414 comprising the neural network configuration 1415 predicts base call sequences 1418a, ..., 1418G for corresponding ones of the plurality of clusters 1407a, ..., 1407G, based on the corresponding sequence signals 1412a, ..., 1412G, respectively. For example, for the cluster 1407a, the base caller 1414 predicts corresponding base call sequence 1418a including base calls for the cluster 1407a for the series of sequencing cycles, based on the corresponding sequence signal 1412a. Similarly, for the cluster 1407b, the base caller 1414 predicts corresponding base call sequence 1418b including base calls for the cluster 1407b for the series of sequencing cycles, based on the corresponding sequence signal 1412b, and so on. Thus, G base call sequences 1418a, ..., 1418G are predicted by the base caller 1414.

Assume that the Oligo # 1 has 8 bases labelled generally as GA1, ..., GA8. Merely as an example and without limiting the scope of this disclosure, assume that the 8 bases of the Oligo # are A, C, T, T, G, C, A, C. Initially, the base caller 1414 is untrained, and hence, is likely to make errors in base calls. For example, the predicted base call sequence 1418a (generally labelled as Sa1, ..., Sa8) are C, A, T, C, G, C, A, G, as illustrated in Fig. 14A. Thus, comparing the ground truth base sequence 1406 of Oligo # 1 (*i.e.*, A, C, T, T, G, C, A, C) and the predicted base sequence 1418a (*i.e.,* C, A, T, C, G, C, A, G), there is an error in base calls for base numbers 1, 2, 4, and 8. Thus, in Fig. 14A, the ground truth base sequence 1406 of Oligo # 1 and the predicted base sequence 1418a are compared at operation 1413a, and the error between these two base sequences are used in the backward pass of the neural network configuration 1415 of the base caller 1414 to train the neural network configuration 1415, such as used for updating gradients and weights of the neural network configuration 1415 (symbolically labelled as gradient update 1417 in Fig. 14A).

Fig. 14A1 illustrates a comparison operation between predicted base sequence 1418a and the ground truth base sequence 1406 of Oligo # 1 in further detail. For example, referring to Figs. 14A and 14A1, the predicted base sequence 1418a is C, A, T, C, G, C, A, G, and the ground truth base sequence 1406 of Oligo # 1 is A, C, T, T, G, C, A, C. Thus, comparing the ground truth base sequence 1406 of Oligo # 1 (*i.e.*, A, C, T, T, G, C, A, C) and the predicted base sequence 1418a (*i.e.,* C, A, T, C, G, C, A, G), there are errors in base calls for base numbers 1, 2, 4, and 8. For example, in Fig. 14A1, the error for base call for base number 1 is given by: "C should be A," *i.e.,* base call C should be base call A. Similarly, the error for base call for base number 2 is given by: "A should be C," *i.e.,* base call A should be base call B, and so on. There are no errors for base calls for base numbers 3, 5, 6, and 7 (illustrated as "Match (no error)" in Fig. 14A1). Thus, in Fig. 14A1, during the comparison, each base call of the predicted base call sequences 1418a is compared to a corresponding base call of the corresponding ground truth sequence (*e.g.*, base sequence 1406 of Oligo # 1), to generate a corresponding comparison result, as illustrated in Fig. 14A1.

Referring again to Fig. 14A, the base calling system 1400 also includes mapping logic 1416, the functionality of which will be discussed herein later. In an example, the mapping logic 1416 can be stored in the memories 404, 403, and/or 406, and the mapping logic 1416 can be executed on a host CPU (such as the CPU 402 of Fig. 4) and/or on configurable processor(s) (such as the configurable processor 450 of Fig. 4) that are local to the sequencing machine 400. In another example, the mapping logic 1416 can be stored remotely from the sequencing machine 400 (*e.g.*, stored in the cloud), and can be executed by remote processors (*e.g.*, executed in the cloud). For example, in the remote version of the mapping logic 1416, the mapping logic receives (*e.g.*, over a network, such as the Internet) data to be mapped from the sequencing machine 400, performs mapping operations, and transmits the mapping results (*e.g.*, over the network, such as the Internet) to the sequencing machine 400. Mapping operations have been discussed in further detail herein later.

Fig. 14A and various other figures, examples, and embodiments of this disclosure refer to a base caller predicting base call sequences. Various example of such prediction of base call sequence have been discussed herein. Further examples of base call prediction can be found in co-pending in U.S. Provisional Patent Application No. 63/217,644, titled "IMPROVED ARTIFICIAL INTELLIGENCE-BASED BASE CALLING OF INDEX SEQUENCES," filed July 1, 2021 (Attorney Docket No. ILLM 1046-1/IP-2135-PRV).

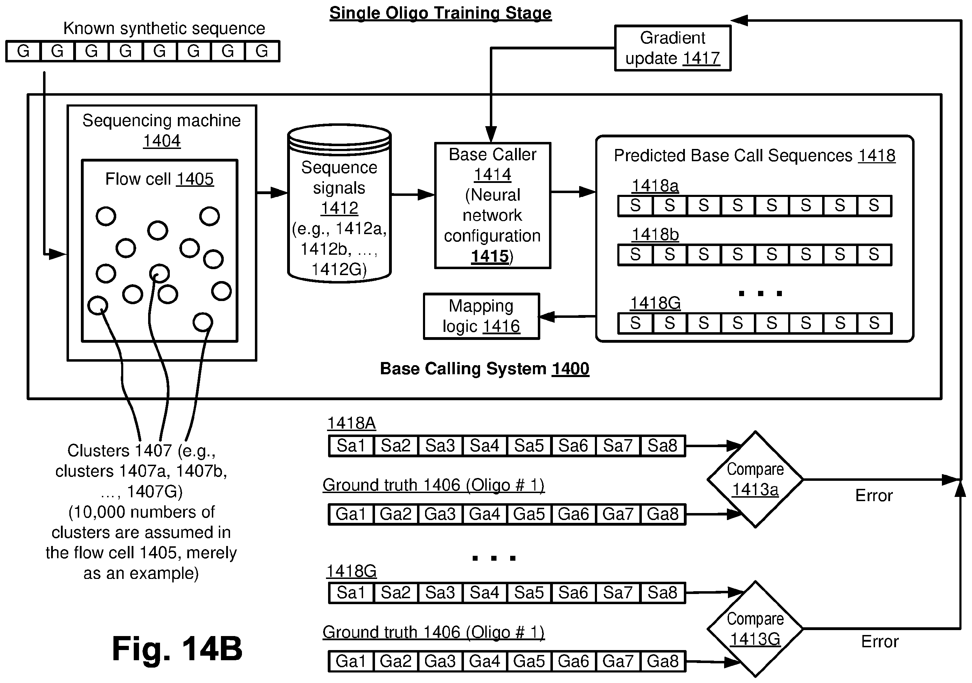

**Fig. 14B** illustrates further details of the base calling system 1400 of Fig. 14A operating in the single-oligo training stage, to train the base caller 1414 comprising the neural network configuration 1415, using the known synthetic sequence 1406. For example, Fig. 14B illustrates using the predicted base call sequences 1418a, ..., 1418G for training the base caller 1414. For example, individual ones of the predicted base call sequences 1418a, ..., 1418G are compared to the ground truth base sequence 1406 of Oligo # 1 (see comparison operations 1413a, ..., 1413G), and the resultant errors are used for gradient update and consequent updating of parameters (such as weights and biases) of the neural network configuration 1415 (symbolically labelled as gradient update 1417 in Fig. 14A) by the backpropagation section of the neural network configuration 1415.

Thus, the neural network configuration 1415 is being trained using base call sequences 1418 predicted by the neural network configuration 1415, and using the ground truth base sequence 1406 of Oligo # 1. Because the training discussed with respect to Figs. 14A and 14B uses a single oligo, this training stage is also referred to as "Single Oligo Training Stage" and Figs. 14A and 14B have been labelled accordingly.

In an example, the process of Figs. 14A and 14B can be repeated iteratively. For example, at a first iteration of Fig. 14A, the NN configuration 1415 is at least partially trained. The at least partially trained NN configuration 1415 is used again during a second iteration to re-generate predicted base call sequences from the sequence signals 1412 (*e.g.,* as discussed with respect to Fig. 14A), and the resultant predicted base call sequences are again compared to the ground truth 1406 (*i.e.,* oligo # 1) to generate error signals, which are used to further train the NN configuration 1415. This process may be iteratively repeated multiple times, until the NN configuration 1415 is adequately trained. In an example, this process may be iteratively repeated for a specific number of times. In another example, this process may be iteratively repeated until there is a saturation in a number of errors (*e.g.*, the errors in consecutive iterations do not significantly decrease).

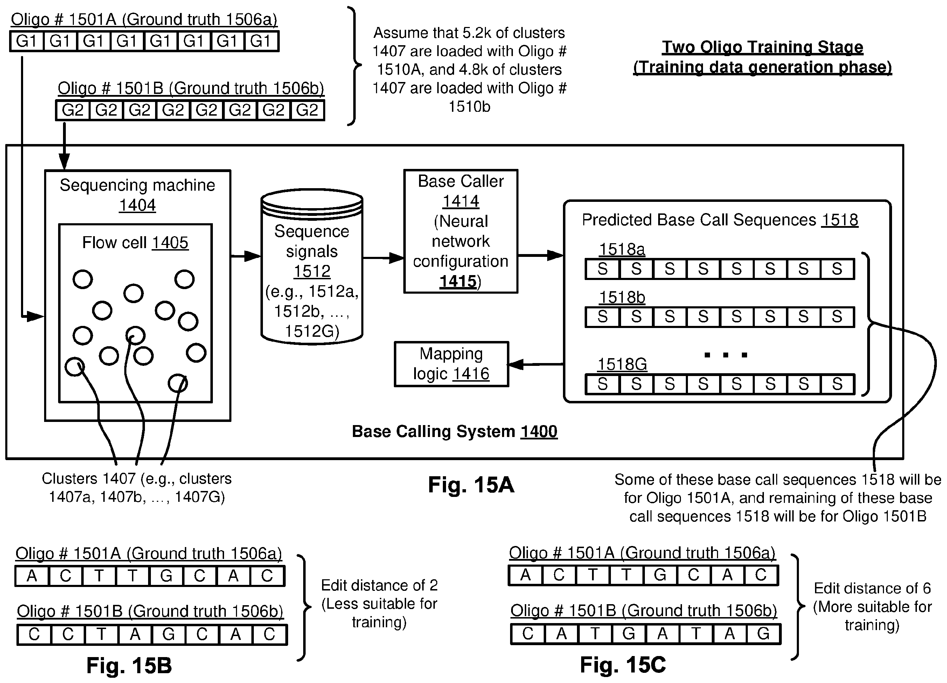

**Fig. 15A** illustrates the base calling system 1400 of Fig. 14A operating in a training data generation phase of a two-oligo training stage, to generate labelled training data using two known synthetic sequences 1501A and 1501B.

The base calling system 1400 of Fig. 15A is the same as the base calling system of Fig. 14A, and in both figures the base calling system 1400 uses the neural network configuration 1415. Furthermore, two different unique oligo sequences 1501A and 1501B are loaded in various clusters of the flow cell 1405. Merely as an example and without limiting the scope of this disclosure, assume that out of the 10,000 clusters 1407, about 5,200 clusters are populated with oligo sequences 1501A and remaining 4,800 clusters are populated with oligo sequences 1501B (although in another example, the two oligos can be substantially equally divided among the 10,000 clusters).

The sequencing machine 1404 generates sequence signals 1512a, ..., 1512G for corresponding ones of the plurality of clusters 1407a, ..., 1407G. For example, for a cluster 1407a, the sequencing machine 1404 generates corresponding sequence signal 1512a indicative of bases for the cluster 1407a for a series of sequencing cycles. Similarly, for a cluster 1407b, the sequencing machine 1404 generates corresponding sequence signal 1512b indicative of bases for the cluster 1407b for the series of sequencing cycles, and so on.

The base caller 1414 comprising the at least partly trained neural network configuration 1415 (*e.g.*, which is trained by iteratively repeating operations of Figs. 14A and 14B) predicts base call sequences 1518a, ..., 1518G for corresponding ones of the plurality of clusters 1407a, ..., 1407G, based on the corresponding sequence signals 1512a, ..., 1512G, respectively. For example, for the cluster 1407a, the base caller 1414 predicts corresponding base call sequence 1518a including base calls for the cluster 1407a for the series of sequencing cycles, based on the corresponding sequence signal 1512a. Similarly, for the cluster 1407b, the base caller 1414 predicts corresponding base call sequence 1518b including base calls for the cluster 1407b for the series of sequencing cycles, based on the corresponding sequence signal 1512b, and so on. Thus, G base call sequences 1518a, ..., 1518G are predicted by the base caller 1414. Note that the neural network configuration 1415 of Fig. 15A was trained earlier during the iterations of the single oligo training stage discussed with respect to Figs. 14A and 14B. Accordingly, the predicted base call sequences 1518a, ..., 1518G would be somewhat accurate, but not very highly accurate (as the base caller 1414 is not fully trained).

In an embodiment, the oligo sequences 1501A and 1501B are selected to have sufficient edit distance between the bases of the two oligos. **Figs. 15B** and **15C** illustrate two corresponding example selections of the oligo sequences 1501A and 1501B of Fig. 15A. For example, in Fig. 15B, oligo 1501A is selected to have bases A, C, T, T, G, C, A, C, whereas oligo 1501B is selected to have bases C, C, T, A, G, C, A, C. Thus, the first base and the fourth base in the two oligos 1510A and 1510B are different, resulting in an edit distance of two between the two oligos 1510A and 1510B.

In contrast, in Fig. 15B, oligo 1501A is selected to have bases A, C, T, T, G, C, A, C, whereas oligo 1501B is selected to have bases C, A, T, G, A, T, A, G. Thus, in the example of Fig. 15B, the first, second, fourth, fifth, sixth, and eighth bases in the two oligos 1510A and 1510B are different, resulting in an edit distance of six between the two oligos 1510A and 1510B.

In an example, the two oligos 1501A and 1501B are selected such that the two oligos are separated by at least a threshold edit distance. Merely as an example, the threshold edit distance can be 4 bases, 5 bases, 6 bases, 7 bases, or even 8 bases. Thus, the two oligos 1501A and 1501B are selected such that the two oligos are sufficiently different from each other.

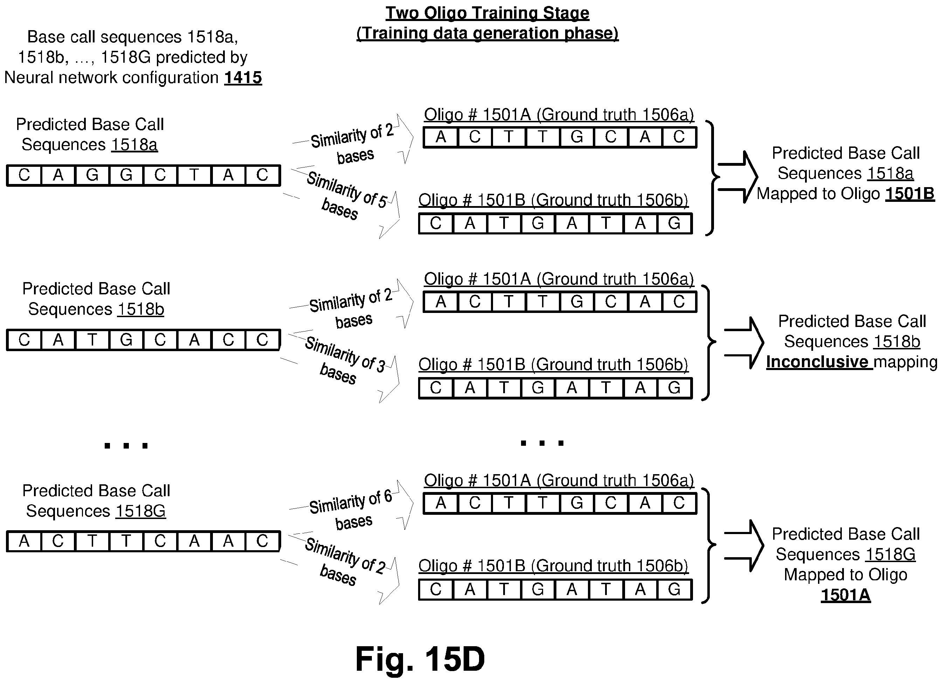

Referring again to Fig. 15A, the base caller 1414 is unaware as to which oligo sequence is populated in which cluster. Thus, the base caller 1414 is unaware of a mapping between the known oligo sequences 1501A, 1501B and the various clusters. In an example, the mapping logic 1416 receives the predicted base call sequences 1518, and maps each predicted base call sequence 1518 to either the oligo 1501A or to the oligo 1501B, or declares inconclusiveness in mapping the predicted base call sequence to either of the two oligos. **Fig. 15D** illustrates example mapping operations to either (i) map a predicted base call sequence to either of the oligo 1501A or to the oligo 1501B, or (ii) declare inconclusiveness in mapping the predicted base call sequence to either of the two oligos.

In an example, the higher the edit distance between the two the oligos, the easier it is (or the more accurate it is) to map individual predictions to either of the two oligos. For example, referring to Fig. 15B, as the edit distance between the two oligos 1501A and 1501B is only two, the two oligos are almost similar, and it may be relatively difficult to map a base call prediction to either of the two oligos. However, as the edit distance between the two oligos 1501A and 1501B in Fig. 15C is six, the two oligos are highly dissimilar, and it may be relatively easy to map a prediction to either of the two oligos. Accordingly, Fig. 15B with the edit distance of two is labelled as "Less suitable for training," and Fig. 15C with the edit distance of six is labelled as "More suitable for training." Thus, in an example, the oligos 1501A and 1501B in accordance with Fig. 15C (and not in accordance with Fig. 15B) are generated and used for training, as will be discussed herein in further detail in turn.

Referring again to Fig. 15D, illustrated are example predicted base call sequences 1518a, 1518b, and 1518G. Also illustrated are example bases of the two oligos 1501A and 1501B (the example bases of the two oligos correspond to the bases illustrated in Fig. 15C).

Because the neural network configuration 1415 is somewhat trained, but not fully trained, the neural network configuration 1415 may be able to make base call predictions, but such base call predictions will be prone to errors.

The predicted base call sequence 1518a comprises C, A, G, G, C, T, A, C. This is compared to the base call sequence A, C, T, T, G, C, A, C of oligo 1501A, and also compared to the base call sequence C, A, T, G, A, T, A, G of Oligo 1501B. The predicted base call sequence 1518a has the seventh and eight bases matching with the corresponding seventh and eight bases of the oligo 1501A, and has the first, second, fourth, sixth, and seventh bases matching with the corresponding bases of the oligo 1501B. Thus, as illustrated in Fig. 15D, the predicted base call sequence 1518a has similarity of 2 bases with oligo 1501A, and the predicted base call sequence 1518a has similarity of 5 bases with oligo 1501B.

If indeed the predicted base call sequence 1518a is for the oligo 1501B (*e.g.,* as the predicted base call sequence 1518a has similarity of 5 bases with oligo 1501B), this means that the neural network configuration 1415 was able to correctly predict five bases of the 8-base sequence (*i.e.,* was able to correctly predict the first, second, fourth, sixth, and seventh bases matching with the corresponding bases of the oligo 1501B). However, as the neural network configuration 1415 isn't fully trained, the neural network configuration 1415 made errors in predicting the remaining three bases (*i.e.*, the third, fifth, and eighth bases).

The mapping logic 1416 can use appropriate logic to map a predicted base call sequence to a corresponding oligo. For example, assume that a predicted base call sequence has SA number of similarities with oligo 1501A, and SB number of similarities with oligo 1501B. In an example, the mapping logic 1416 maps a predicted base call sequence to oligo 1501A if SA > ST, and SB < ST, where ST is a threshold number. That is, the mapping logic 1416 maps the predicted base call sequence to oligo 1501A if the similarity level with the oligo 1501A is higher than the threshold, and if the similarity level with the oligo 1501B is lower than the threshold.

Similarly, in another example, the mapping logic 1416 maps a predicted base call sequence to oligo 1501B if SB > ST, and SA < ST.

In yet another example, the mapping logic 1416 declares a predicted base call sequence to be inconclusive if both SA and SB are less than the threshold ST, or if both SA and SB are greater than the threshold ST.

The above discussion can be written in an equation form as:

For a predicted base call sequence:

$$\text{if } SA > ST, \text{ and } SB < ST, \text{ then map to oligo 1501A;} \qquad \text{Equation 1}$$

$$\text{if } SB > ST, \text{ and } SA < ST, \text{ then map to oligo 1501B;} \qquad \text{Equation 2}$$

$$\text{if both } SA, SB < ST, \text{ then declare inconclusive mapping; or} \qquad \text{Equation 3}$$

$$\text{if both } SA, SB > ST, \text{ then declare inconclusive mapping.} \qquad \text{Equation 4}$$

The threshold ST depends on a number of bases in the oligos (which is 8 in the example use case illustrated in the figures), a desired accuracy, and/or is implementation specific. Merely as an example, the threshold ST is assumed to be 4 in the example use case illustrated in the Fig. 15D. Note that the threshold ST of 4 is a mere example, and the selection of the threshold ST can be implementation specific. Merely as an example, during initial iterations of the training, the threshold ST can have a relatively lower value (*e.g.,* 4); and the threshold ST can have a relatively higher value (*e.g.,* 6 or 7) during later iterations of the training (the training iterations have been discussed herein later). Thus, as and when the NN configurations are better trained during later training iterations, the threshold ST can be gradually increased. However, in another example, the threshold ST can have the same value throughout all iterations of the training. Although the threshold ST is selected as 4 in the example of Fig. 15D, the threshold ST can be, for example, 5, 6, or 7 in other example implementations. In an example, the threshold ST can also be represented as a percentage. For example, when the threshold ST is 4 and total number of bases is 8, the threshold ST can be expressed as $(4/8) \times 100$, *i.e.,* 50%. The threshold ST can be a user selectable parameter, and can be selected to be between 50% and 95% in an example.

Now referring again to Fig. 15D, as discussed above, the predicted base call sequence 1518a has similarity of 2 bases with oligo 1501A, and the predicted base call sequence 1518a has similarity of 5 bases with oligo 1501B. Thus, SA = 2, and SB = 5. Assuming the threshold ST of 4, in accordance with equation 2, the predicted base call sequence 1518a is mapped to oligo 1501B.

Referring now to the predicted base call sequence 1518b, the predicted base call sequence 1518b has similarity of 2 bases with oligo 1501A, and the predicted base call sequence 1518b has similarity of 3 bases with oligo 1501B. Thus, SA = 2, and SB = 3. Assuming the threshold ST of 4, in accordance with equation 3, the predicted base call sequence 1518b is declared inconclusive for mapping to either of the oligo sequences.

Referring now to the predicted base call sequence 1518G, the predicted base call sequence 1518G has similarity of 6 bases with oligo 1501A, and the predicted base call sequence 1518G has similarity of 3 bases with oligo 1501B. Thus, SA = 6, and SB = 3. Assuming the threshold ST of 4, in accordance with equation 2, the predicted base call sequence 1518G is mapped to oligo 1501A.

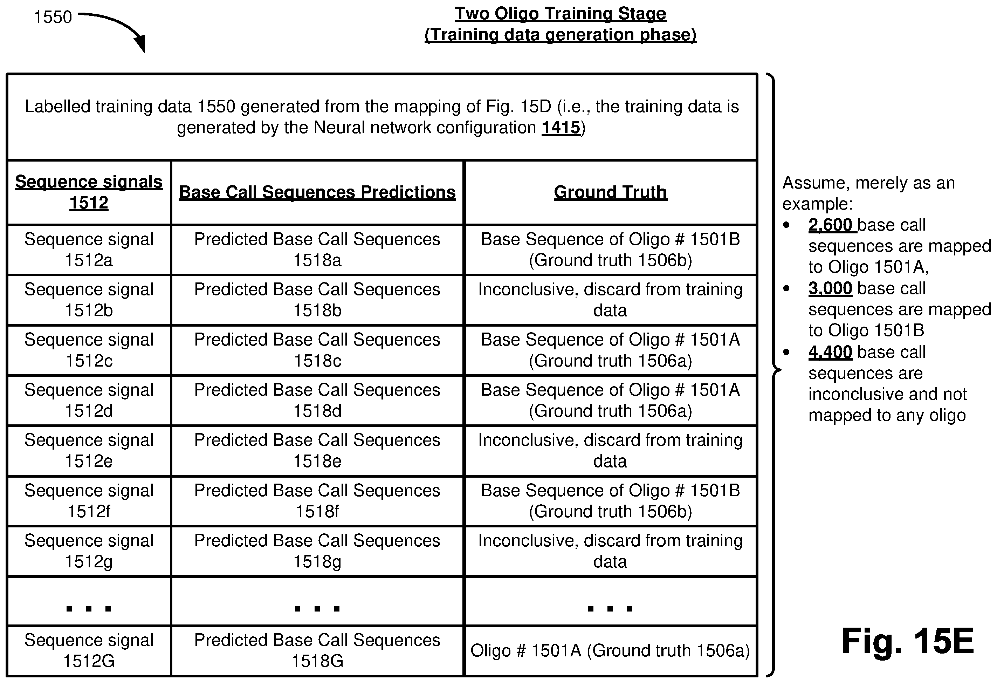

**Fig. 15E** illustrates labelled training data 1550 generated from the mapping of Fig. 15D, where the labelled training data 1550 is used by another neural network configuration 1615 (*e.g.,* illustrated in Fig. 16A, where the other neural network configuration 1615 is different from, and more complex relative to the neural network configuration 1415 of Figs. 14A, 14B, 15A).

As illustrated in Fig. 15E, some of the predicted base call sequences 1518 and corresponding sequence signals are mapped to the base sequence of oligo 1501A (*i.e.,* ground truth 1506a), some other predicted base call sequences 1518 and corresponding sequence signals are mapped to the base sequence of oligo 1501B (*i.e.,* ground truth 1506b), and mapping of remaining of the predicted base call sequences 1518 and corresponding sequence signals are inconclusive.

For example, the predicted base call sequences 1518c, 1518d, 1518G and corresponding sequence signals 1512c, 1512d, 1512G are mapped to the base sequence of oligo 1501A (*i.e.,* ground truth 1506a); the predicted base call sequences 1518a, 1518f and corresponding sequence signals 1512a, 1512f are mapped to the base sequence of oligo 1501B (*i.e.,* ground truth 1506b); and mapping of remaining of the predicted base call sequences 1518b, 1518e, 1518g and corresponding sequence signals 1512b, 1512e, 1512g are inconclusive.

Assume, merely as an example, that 2,600 base call sequences of the training data 1550 are mapped to oligo 1501A and 3,000 base call sequences of the training data 1550 are mapped to oligo 1501B. Remaining 4,400 base call sequences are inconclusive and not mapped to any of the two oligos, as illustrated in Fig. 15E.

Note that Figs. 15A, 15D and 15E are referred to as "training data generation phase" of "two oligo training stage," as the labelling training data 1550 are generated using sequences from two oligos and using the neural network configuration 1415.

**Fig. 16A** illustrates the base calling system 1400 of Fig. 14A operating in a "training data consumption and training phase" of the "two-oligo training stage," to train the base caller 1414 comprising another neural network configuration 1615 (that is different from, and more complex, relative to the neural network configuration 1415 of Fig. 14A), using the two known synthetic sequences 1501A and 1501B.

The base calling system 1400 of Fig. 16A is same as the base calling system of Fig. 14A. However, unlike Fig. 14A (where neural network configuration 1415 was used in the base caller 1414), the base caller 1414 in Fig. 16A uses a different neural network configuration 1615. The neural network configuration 1615 of Fig. 16A is different from the neural network configuration 1415 of Fig. 14A. For example, the neural network configuration 1615 is a convolution neural network (examples of which are illustrated in Figs. 7, 9, 10, 11, 12) that uses a larger number of layers and parameters (such as weights and biases) than the neural network configuration 1415. In another example, the neural network configuration 1615 is a convolution neural network that uses a larger number of convolution filters than the neural network configuration 1415. The configuration, topology, and number of layers and/or filters of the two neural network configurations 1415 and 1615 may be different in some examples.

In the "training data consumption and training phase" of the "two-oligo training stage" illustrated in Fig. 16A, the base caller 1414 comprising the neural network configuration 1615 receives the sequence signals 1512, which were previously generated during the "training data generation phase" of Fig. 15A. That is, the base caller 1414 comprising the neural network configuration 1615 reuses the previously generated sequence signals 1512. Accordingly, as the previously generated sequence signals 1512 are reused in the "training data consumption and training phase" of the "two-oligo training stage" illustrated in Fig. 16A, the sequencing machine 1404 and components there within do not play a role, and hence, are illustrated using dotted lines. Similarly, the mapping logic 1416 also does not play any role (as no mapping is being performed in Fig. 16A), and hence, the mapping logic 1416 is also illustrated using dotted lines.

Thus, in Fig. 16A, the base caller 1414 comprising the neural network configuration 1615 receives the previously generated sequence signals 1512, and predicts base call sequences 1618 from the sequence signals 1512. The predicted base call sequences 1618 comprise predicted base call sequences 1618a, 1618b, ..., 1618G. For example, the sequence signal 1512a is used to predict base call sequence 1618a, the sequence signal 1512b is used to predict base call sequence 1618b, the sequence signal 1512G is used to predict base call sequence 1618G, and so on.

The neural network configuration 1615 is not yet trained, and hence, the predicted base call sequences 1618a, 1618b, ..., 1618G would have many errors. The mapped training data 1550 of Fig. 15E is now used to train the neural network configuration 1615. For example, from the training data 1550, the base caller 1414 knows that:

(i) sequence signals 1512c, 1512d, 1512G are for the base sequence of oligo 1501A (*i.e.,* ground truth 1506a);
(ii) sequence signals 1512a, 1512f are for the base sequence of oligo 1501B (*i.e.,* ground truth 1506b); and
(iii) mapping of sequence signals 1512b, 1512e, 1512g are inconclusive.

Thus, the sequence signals 1512 and predicted base call sequences 1518 are culled in three categories: (i) a first category comprising sequence signals 1512c, 1512d, 1512G (and corresponding predicted base call sequences 1518c, 1518d, 1518G) that can be mapped to the base sequence of oligo 1501A (*i.e.,* ground truth 1506a); (i) a second category comprising sequence signals 1512a, 1512f (and corresponding predicted base call sequences 1518a, 1518f) that can be mapped to the base sequence of oligo 1501B (*i.e.,* ground truth 1506b); and (iii) a third category comprising sequence signals 1512b, 1512e, 1512g (and corresponding predicted base call sequences 1518b, 1518e, 1518g) that cannot mapped to any of the base sequences of oligos 1501A or 1501B.

Hence, based on (iii) above, the predicted base call sequences 1618b, 1618e, and 1618g (*e.g.,* corresponding to the sequence signals 1512b, 1512e, 1512g) are not used for training the neural network configuration 1615. Thus, the predicted base call sequences 1618b, 1618e, and 1618g are discarded during the training iteration and not used for gradient update (symbolically illustrated in Fig. 16A using a "X" or "cross sign" between the predicted base call sequences 1618b, 1618e, and 1618g and the gradient update box 1617).

Based on (i) above, the base caller 1414 knows that the predicted base call sequences 1618c, 1618d, 1618G (*e.g.*, corresponding to the sequence signals 1512c, 1512d, 1512G) are likely to be for oligo 1501A. That is, the base sequence of oligo 1501A is likely to be the ground truth for these predicted base call sequences 1618c, 1618d, 1618G, although the untrained neural network configuration 1615 may have erroneously predicted at least some bases of these predicted base call sequences. Accordingly, the neural network configuration compares each of the predicted base call sequences 1618c, 1618d, and 1618G to the ground truth 1506a (which is the base sequence of oligo 1501A) using comparison functions 1613, and uses the generated errors for gradient update 1617 and resultant training of the neural network configuration 1615.

Similarly, based on (ii) above, the base caller knows that the predicted base call sequences 1618a and 1618f (*e.g.*, corresponding to the sequence signals 1512a and 1512f, respectively) are likely to be for oligo 1501B. That is, the base sequence of oligo 1501B is likely to be the ground truth for these predicted base call sequences 1618a and 1618f, although the untrained neural network configuration 1615 may have erroneously predicted at least some bases of these predicted base call sequences. Accordingly, the neural network configuration compares each of the predicted base call sequences 1618a and 1618f to the ground truth 1506b (which is the base sequence of oligo 1501B) using comparison functions 1613, and uses the generated errors for gradient update 1617 and resultant training of the neural network configuration 1615.

At the end of the training data consumption and training phase of Fig. 16A, the NN configuration 1615 is at least partially trained.

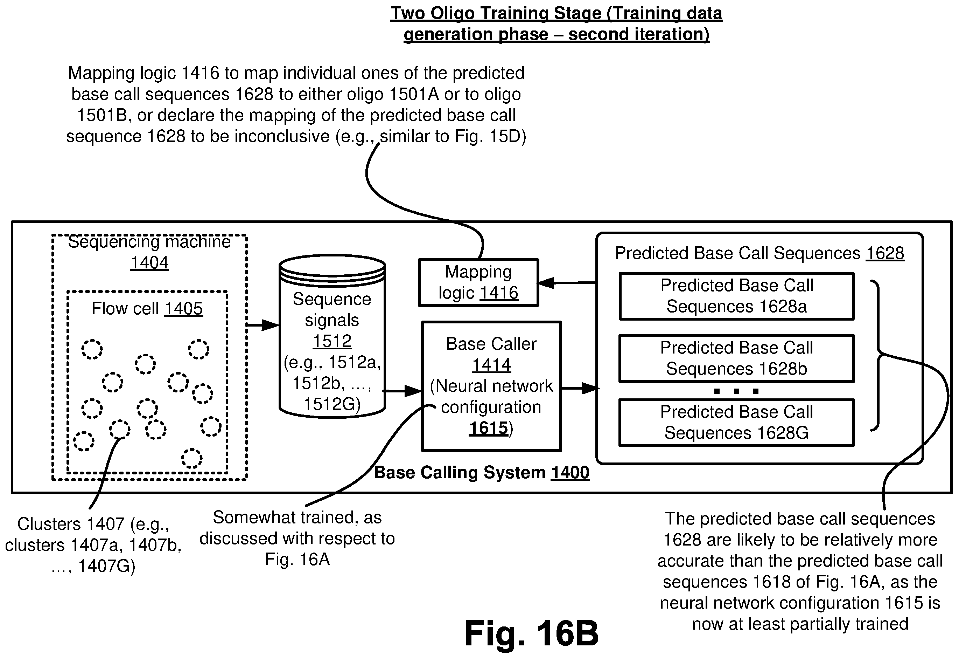

**Fig. 16B** illustrates the base calling system 1400 of Fig. 14A operating in a second iteration of the training data generation phase of the two-oligo training stage. For example, in Fig. 16A, the neural network configuration 1615 was being trained using the training data 1550. In Fig. 16B, the somewhat or at least partially trained neural network configuration 1615 is used to generate further training data. For example, the at least partially trained neural network configuration 1615 uses the previously generated sequence signals 1512 to predict base call sequences 1628. The predicted base call sequences 1628 of Fig. 16B are likely to be relatively more accurate than the predicted base call sequences 1618 of Fig. 16A, as the predicted base call sequences 1618 of Fig. 16A were generated using the untrained neural network configuration 1615, whereas the predicted base call sequences 1628 of Fig. 16B are generated using the at least partially neural network configuration 1615.

Furthermore, the mapping logic 1416 maps individual ones of the predicted base call sequences 1628 to either oligo 1501A or to oligo 1501B, or declares the mapping of the predicted base call sequence 1628 to be inconclusive (*e.g.*, similar to the discussion with respect to Fig. 15D).

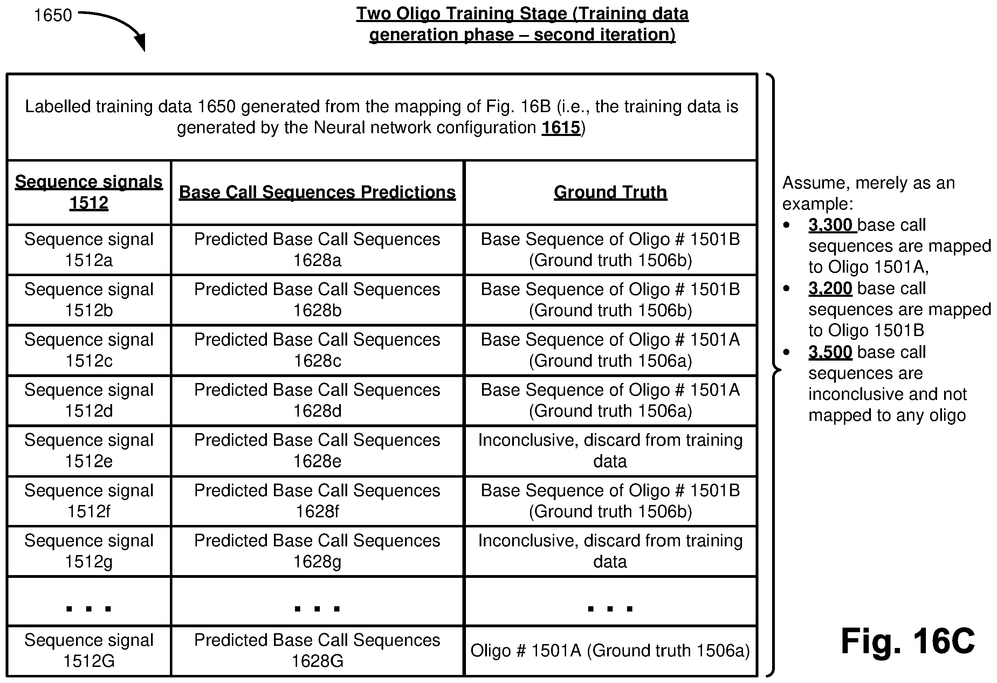

**Fig. 16C** illustrates labelled training data 1650 generated from the mapping of Fig. 16B, where the training data 1650 is to be used for further training.

As illustrated in Fig. 16C, some of the predicted base call sequences 1628 and corresponding sequence signals 1512 are mapped to the base sequence of oligo 1501A (*i.e.*, ground truth 1506a), some other predicted base call sequences 1628 and corresponding sequence signals 1512 are mapped to the base sequence of oligo 1501B (*i.e.*, ground truth 1506b), and mapping of the remaining predicted base call sequences 1628 and corresponding sequence signals 1512 are inconclusive.

For example, the predicted base call sequences 1628 are culled in three categories - (i) the predicted base call sequences 1628c, 1628d, and 1628G and corresponding sequence signals 1512c, 1512d, and 1512G are mapped to the base sequence of oligo 1501A (*i.e.*, ground truth 1506a); (ii) the predicted base call sequences 1628a, 1628b,and 1628f and corresponding sequence signals 1512a, 1512b, and 1512f are mapped to the base sequence of oligo 1501B (*i.e.*, ground truth 1506b); and (iii) mapping of the remaining predicted base call sequences 1628e and 1628g and corresponding sequence signals 1512e and 1512g are inconclusive.

Assume, merely as an example, that 3,300 base call sequences of the training data 1650 are mapped to oligo 1501A and 3,200 base call sequences of the training data 1650 are mapped to oligo 1501B. The remaining 3,500 base call sequences are inconclusive and not mapped to any of the two oligos, as illustrated in Fig. 16C.

Comparing the number of unmapped (or inconclusive) sequences of base calls between the training data of Figs. 15E and 16C, it is observed that this number is 4,400 in Fig. 15E and 3,500 in Fig. 16C. This is because the at least partially trained neural network configuration 1615 of Fig. 16B (that was used to generate the mapping of the training data 1650) may be relatively more accurate and/or more trained than the at least partially trained neural network configuration 1415 of Fig. 15A (that was used to generate the mapping of the training data 1550). Accordingly, the number of inconclusive sequences of base calls gradually decreases, as base calls are getting relatively more accurate (*e.g.*, less error prone) and hence, are now relatively more correctly mapped.

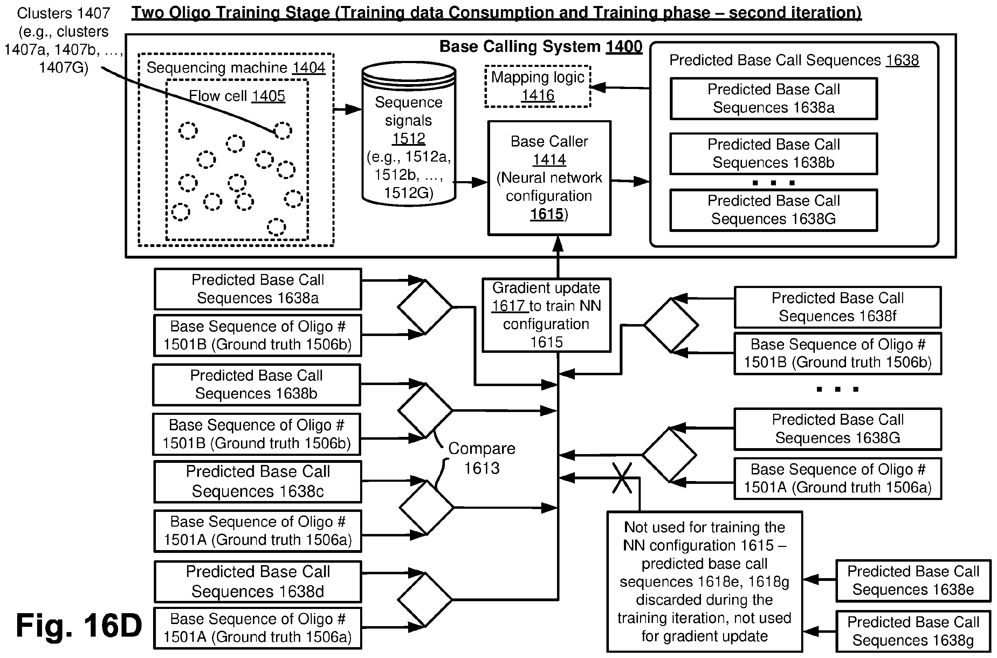

**Fig. 16D** illustrates the base calling system 1400 of Fig. 14A operating in a second iteration of the "training data consumption and training phase" of the "two-oligo training stage," to train the base caller 1414 comprising the neural network configuration 1615 of Fig. 16A, using the two known synthetic sequences 1501A and 1501B.

Figs. 16A and 16D are at least in part similar. For example, Figs. 16A and 16D are used to train the neural network configuration 1615 using training data 1550 of Fig. 15E and training data 1650 of Fig. 16C, respectively. Note that at the initial stage of Fig. 16A, the neural network configuration 1615 was totally untrained; whereas at the initial stage of Fig. 16D, the neural network configuration 1615 is at least partially trained.

In Fig. 16D, the base caller 1414 comprising the at least partially trained neural network configuration 1615 receives the sequence signals 1512, which were previously generated during the "training data generation phase" of Fig. 15A, and predicts base call sequences 1638 from the sequence signals 1512. The predicted base call sequences 1638 comprise predicted base call sequences 1638a, 1638b, ..., 1638G. For example, the sequence signal 1512a is used to predict base call sequence 1638a, the sequence signal 1512b is used to predict base call sequence 1638b, the sequence signal 1512G is used to predict base call sequence 1638G, and so on.

The neural network configuration 1615 is not fully trained, and hence, the predicted base call sequences 1638a, 1638b, ..., 1638G would include some errors, although the errors in the predicted base call sequences 1638 of Fig. 16D are likely to be less than the errors in the predicted base call sequences 1618 of Fig. 16A and 1628 of Fig. 16B. The mapped training data 1650 of Fig. 16C is now used to further train the neural network configuration 1615. For example, from the training data 1650, the base caller 1414 knows that:

(i) sequence signals 1512c, 1512d, 1512G are for the base sequence of oligo 1501A (*i.e.*, Ground truth 1506a);
(ii) sequence signals 1512a, 1512b, 1512f are for the base sequence of oligo 1501B (*i.e.*, Ground truth 1506b); and
(iii) mapping of sequence signals 1512e, 1512g are inconclusive.

Hence, based on (iii) above, the predicted base call sequences 1638e and 1638g (*e.g.*, corresponding to the sequence signals 1512e and 1512g, respectively) in Fig. 16D are not used for training the neural network configuration 1615. Thus, these predicted base call sequences 1638e and 1638g are discarded from the training data and not used for gradient update (symbolically illustrated using a "X" or "cross sign" between the predicted base call sequences 1618e, 1618g and the gradient update box 1617 in Fig. 16D).

Based on (i) above, the base caller 1414 knows that the predicted base call sequences 1638c, 1638d, and 1638G (*e.g.*, corresponding to the sequence signals 1512c, 1512d, and 1512G, respectively) are likely to be for oligo 1501A. That is, the base sequence of oligo 1501A is likely to be the ground truth for these predicted base call sequences 1638c, 1638d, 1638G, although the partially neural network configuration 1615 may have erroneously predicted at least some bases of these predicted base call sequences. Accordingly, the neural network configuration compares each of the predicted base call sequences 1638c, 1638d, 1638G to the ground truth 1506a (which is the base sequence of oligo 1501A) using comparison functions 1613, and uses the generated errors for gradient update 1617 and resultant training of the neural network configuration 1615. For example, during the comparison, each base call of the predicted base call sequences 1638c is compared to a corresponding base call of the corresponding ground truth sequence, to generate a corresponding comparison result, *e.g.*, as discussed with respect to Fig. 14A1.

Similarly, based on (ii) above, the base caller knows that the predicted base call sequences 1638a, 1638b, and 1638f (*e.g.*, corresponding to the sequence signals 1512a, 1512b, and 1512f, respectively) are likely to be for oligo 1501B. That is, the base sequence of oligo 1501A is likely to be the ground truth for these predicted base call sequences 1638a, 1638b, and 1638f, although the partially neural network configuration 1615 may have erroneously predicted at least some bases on these predicted base call sequences. Accordingly, the neural network configuration compares each of the predicted base call sequences 1638a, 1638b, and 1638f to the ground truth 1506b (which is the base sequence of oligo 1501B) using comparison functions 1613, and uses the generated errors for gradient update 1617 and resultant training of the neural network configuration 1615.

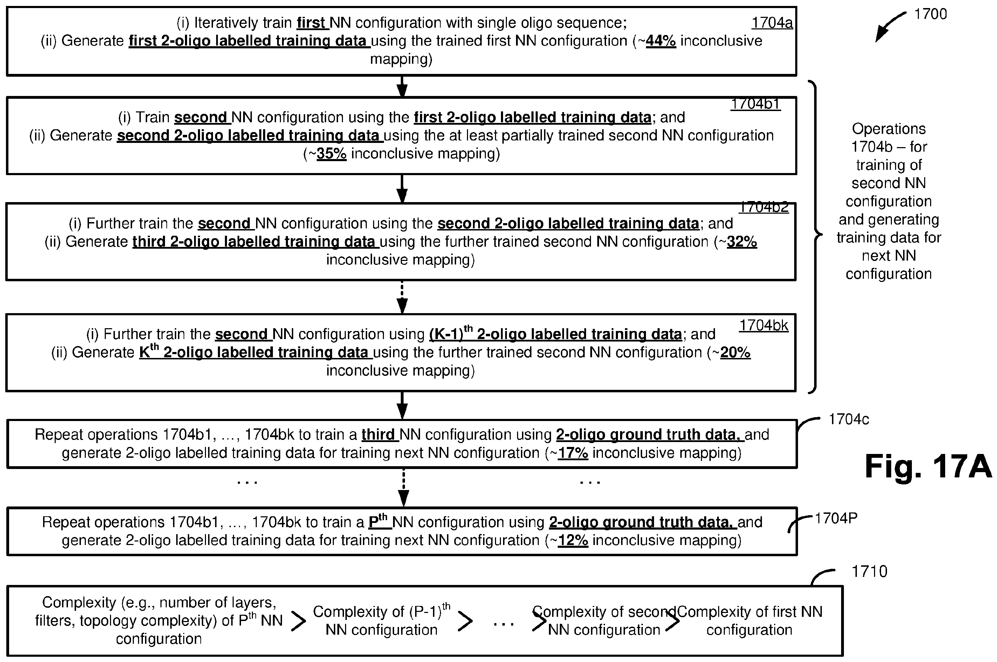

**Fig. 17A** illustrates a flowchart depicting an example method 1700 for iteratively training neural network configurations for base calling using single-oligo and two-oligo sequences. The method 1700 progressively trains NN configurations that are progressively and monotonically complex in nature. Increasing a complexity of NN configurations can include increasing a number of layers of the NN configuration, increasing a number of filters of the NN configuration, increasing a topology complexity in the NN configuration, and/or the like. For example, the method 1700 refers to a first NN configuration (which is the NN configuration 1415 discussed herein earlier with respect to Fig. 14A and other figures), a second NN configuration (which is the NN configuration 1615 discussed herein earlier with respect to Fig. 16A and other figures), a $P^{th}$ NN configuration (which haven't been specifically discussed with respect to Figs. 14A-16D), and so on. In an example, complexity of the $P^{th}$ NN configuration is higher than that of the $(P-1)^{th}$ NN configuration, which is higher than that of the $(P-2)^{th}$ NN configuration, and so on, and complexity of the second NN configuration is higher than that of the first NN configuration, as symbolically illustrated within box 1710 of Fig. 17A. Thus, the complexity of NN configurations increase monotonically (*i.e.*, a NN configuration of a later stage at least have similar complexity or more complexity than a NN configuration of an earlier stage).

Note that in method 1700, operations 1704a is for iteratively training the first NN configuration and generating labelled training data for the second NN configuration, operations 1704b1-1704bk are for training the second NN configuration and generating labelled training data for a third NN configuration, operations 1704c are for training the third NN configuration and generating labelled training data for a fourth NN configuration. This process continues, and operations 1704P is for training a $P^{th}$ NN configuration and generating labelled training data for a subsequent NN configuration. Thus, generally speaking, in the method 1700, operations 1704i are for training an $i^{th}$ NN configuration and generating labelled training data for a $(i+1)^{th}$ NN configuration, where i = 1, ..., P.

The method 1700 comprises, at 1704a, (i) iteratively training a first NN configuration with a single oligo sequence and (ii) generating first 2-oligo labelled training data using the trained first NN configuration. As discussed, the first NN configuration is the NN configuration 1415 of Fig. 14A, and the single oligo sequence comprises oligo # 1 discussed with respect to Figs. 14A, 14B. Iterative training of the first NN configuration with the single oligo sequence is discussed with respect to Figs. 14A, 14B. Generation of the first 2-oligo labelled training data using the trained first NN configuration is discussed with respect to Figs. 15A, 15D, 15E, where the first 2-oligo labelled training data is training data 1550 of Fig. 15E.

The method 1700 then proceeds from 1704a to 1704b. As illustrated, operations 1704b are for training the second NN configuration (*e.g.*, using the first 2-oligo labelled training data generated from operations 1704a), and using the trained second NN configuration to generate further 2-oligo labelled training data for training the third NN configuration. The operations 1704b comprises sub-operations at blocks 1704b1-1704bk.

At block 1704b1, (i) the second NN configuration is trained using the first 2-oligo labelled training data generated at 1704a and (ii) second 2-oligo labelled training data is generated using the at least partially trained second NN configuration. As discussed, the second NN configuration is the NN configuration 1615 of Fig. 16A. Training of the second NN configuration using the first 2-oligo labelled training data is also illustrated in Fig. 16A. Generation of the second 2-oligo labelled training data (*e.g.,* which is the training data 1650 of Fig. 16C) using the at least partially trained second NN configuration is discussed with respect to in Figs. 16B and 16C.

The method 1700 then proceeds from 1704b1 to 1704b2. At block 1704b2, (i) the second NN configuration is further trained using the second 2-oligo labelled training data and (ii) third 2-oligo labelled training data is generated using the further trained second NN configuration. Training of the second NN configuration using the second 2-oligo labelled training data is illustrated in Fig. 16D. Generation of the third 2-oligo labelled training data using the further trained second NN configuration is not illustrated, but would be similar to the discussion with respect to Figs. 16B and 16C.

Note that block 1704b1 is a first iteration of training the second NN configuration, block 1704b2 is a second iteration of training the second NN configuration, and so on, and finally block 1704bk is a $k^{th}$ iteration of training the second NN configuration. As discussed, operations of block 1704b1 are discussed in detail with respect to Figs. 16A, 16B, 16C.

Operations of subsequent blocks 1704b2, ..., 1704bk would be similar to the discussion for block 1704b1.

Note that the same second NN configuration is used in all of the iterations 1704b1, ..., 1704bk. Thus, these k iterations are aimed at iteratively training the same second NN configuration, without increasing a complexity of the second NN configuration.

The training of the second NN configuration progresses with each iteration of blocks 1704b1, 1704b2, ..., 1704bk. As the second neural network is gradually trained at each step of the iterations 1704b1, ..., 1704bk, the second neural network progressively makes relatively less errors in predicting the base call sequence. For example, as indicated in block 1704a and as also illustrated in Fig. 15E, the first 2-oligo labelled training data (*i.e.,* training data 1550) generated using the trained first NN configuration has 44% (*i.e.,* 4,400 out of 10,000) inconclusive mappings. As indicated in block 1704b1 and as also illustrated in Fig. 16C, the second 2-oligo labelled training data (*i.e.,* training data 1650) generated using the partially trained second NN configuration has 35% (*i.e.,* 3,500 out of 10,000) inconclusive mappings. As indicated in block 1704b2 and merely as an example, the third 2-oligo labelled training data generated using the further trained second NN configuration may have 32% (*i.e.,* 3,200 out of 10,000) inconclusive mappings. The percentage of inconclusive mappings may gradually decrease with each iteration, until it reaches about 20%, for example, at block 1704bk.

The number of iterations "k" for training the second NN configuration may be based on satisfaction of one or more convergence conditions. Once a converge condition is satisfied, the iterations for training the second NN configuration can end. The converge condition is implementation specific, and dictates a number of iterations to undergo for training the second NN configuration. In an example, satisfying the converge condition is an indication that further iterations may not significantly help in further training of the second NN configuration, and hence, the training iterations for the second NN configuration can be terminated. Discussed herein are some examples of converge condition and satisfaction thereof. For example, the second NN configuration may be iteratively trained, until the percentage of inconclusive mappings is less than a threshold percentage. Here, the convergence condition is satisfied once the percentage of inconclusive mappings becomes less than the threshold percentage. For example, for the second NN configuration, this threshold can be about 20%, merely as an example. Thus, at iteration k once the threshold is met, the convergence condition is satisfied, and training of the second NN configuration ends. Accordingly, the method proceeds to 1704c where the $K^{th}$ 2-oligo labelled training data generated at block 1704bk is used to train a third NN configuration that is more complex than the second NN configuration.

In another example, the iterations for the second NN configuration continues until the inconclusive mapping percentage somewhat saturates (*i.e.,* does not decrease significantly with consecutive iterations), which satisfies the convergence condition. That is, in this example, saturation below a threshold level indicates sufficient convergence of the iterative training (*e.g.,* is indicative of satisfaction of the convergence condition), and further iteration cannot significantly improve the model - hence, the iterations for the current model can end. For example, assume that at iteration (k-2) (*e.g.,* at block 1704b(k-2)), the inconclusive mapping percentage is 21%; at iteration (k-1) (*e.g.,* at block 1704b(k-2)), the inconclusive mapping percentage is 20.4%; and at iteration k (*e.g.,* at block 1704bk), the inconclusive mapping percentage is 20%. Thus, the decrease in the inconclusive mapping percentage is relatively low (*e.g.,* 0.6% and 0.4%, respectively) for the last two iterations, implying that the training has almost saturated and further training cannot significantly improve the second NN configuration. Here, saturation is measured as difference between inconclusive mapping percentages during two consecutive iterations. That is, if two consecutive iterations have almost same inconclusive mapping percentages, then further iterations may not be helpful in further reduction of this percentage, and hence, the training iterations can be terminated. Accordingly, at this stage, the iterations for the second NN configuration is terminated, and the method 1700 proceeds to 1704c for the third NN configuration.

In yet another example, the number of iterations "k" is prespecified, and completing the k number of iterations satisfies the convergence condition, so that the training for the current NN configuration can end and next NN configuration can commence.

Thus, at the end of the iterations for the second NN configuration (*i.e.,* at the end of block 1704k), the method 1700 proceeds to block 1704c, where the third NN configuration is iteratively trained. The training of the third NN configuration would also include iterations similar to those discussed with respect to operations 1704b1, ..., 1704bk, and hence, is not discussed in further detail.

This process of progressively training more complex NN configurations continues, until at 1704P of the method 1700, a $P^{th}$ NN configuration is trained, and 2-oligo training data is generated for training the next NN configuration.

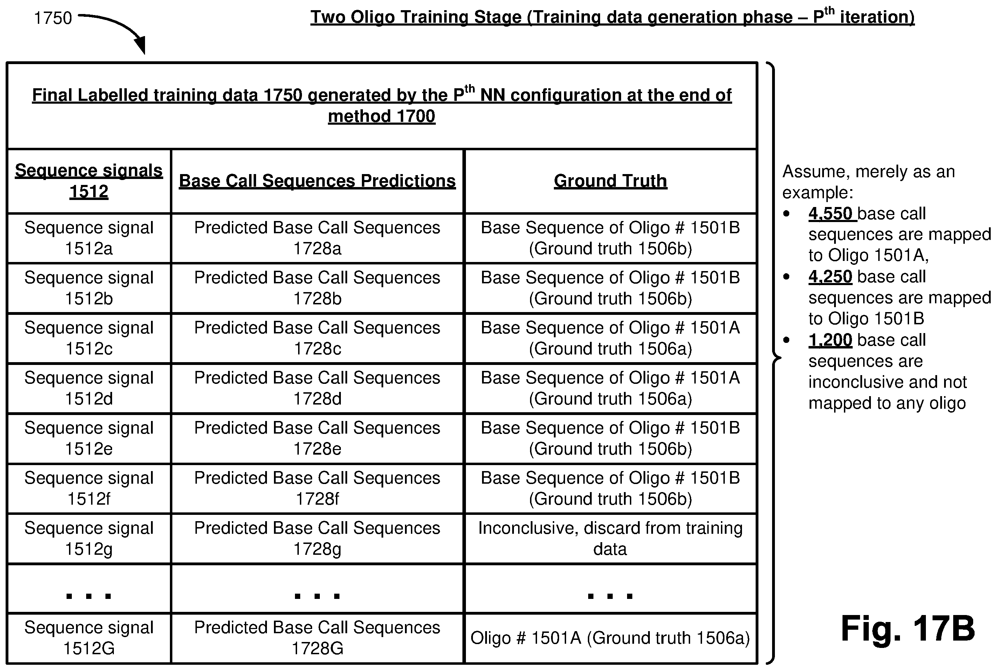

Note that in an example and as discussed herein, the same 2-oligo sequences may be used for all the iterations of blocks 1704b1, ..., 1704bk, 1704c, ..., 1704P. However, in some other examples and although not discussed herein, different 2-oligo sequences may also be used for different iterations of the method 1700 of Fig. 17.

As discussed, the more complex a model is, the better the model can be trained to predict base calls. For example, at the end of training the second NN configuration, the final labelled training data generated by the second NN configuration has 20% inconclusive mappings. The percentage of inconclusive mapping decreases further at the end of training of the third NN configuration. For example, during a first training iteration of the third NN configuration, the percentage of inconclusive mapping can be 36% (*e.g.,* as the third NN configuration is barely trained during the first iteration), and this percentage can gradually decrease with subsequent training iterations of the third NN configuration. Assume, as illustrated in Fig. 17A, for example, at the end of training the third NN configuration, the final labelled training data generated by the third NN configuration has 17% inconclusive mappings. This percentage of inconclusive mapping further decrease with progression of the iterations of Fig. 17A, and for example, at the end of training the $P^{th}$ NN configuration, the final labelled training data generated by the $P^{th}$ NN configuration has 12% inconclusive mappings. Note that the training ends at 12% inconclusive mapping, *e.g.*, when the convergence conditions (discussed herein earlier) are satisfied for the $P^{th}$ NN configuration. Thus, P number of NN configurations are trained in the method 1700. The number "P" can be three, four, five, or higher, and is implementation specific, and can also be based on satisfaction of corresponding one or more convergence conditions. For example, if $(P-1)^{th}$ NN configuration results in 12.05% inconclusive mapping and if $P^{th}$ NN configuration results in 12% inconclusive mapping, then there is a marginal improvement of 0.05% inconclusive mapping between the two NN configurations. This indicates that the training of new NN configurations with the 2-oligo sequence is saturating. Here, saturation refers to a difference in inconclusive mapping percentages between two consecutive NN configurations. If the saturation is at or below a threshold (such as 0.1%), the training of the 2-oligo sequence training is terminated. In another example, the number "P" of the NN configurations can be pre-specified by a user to be, for example, three, four, or a higher number. As will be discussed herein later in turn, once the training with the P number of NN configurations using 2-oligo sequences are completed, further complex analyte (such as 3-oligo sequence) may be used for training.

**Fig. 17B** illustrates example final labelled training data 1750 generated by the $P^{th}$ NN configuration at the end of method 1700 of Fig. 17A. As discussed, at the end of training the $P^{th}$ NN configuration, the final labelled training data generated by the $P^{th}$ NN configuration has 12% (or 1,200 out of 10,000) inconclusive mappings. The predicted base call sequences are culled in three categories: (i) a first category comprising predicted base call sequences mapped to the oligo 1501A, (ii) a second category comprising predicted base call sequences mapped to the oligo 1501B, and (iii) a third category comprising predicted base call sequences mapped to neither of the oligos 1501A or 1501B. The training data 1750 of Fig. 17B would be evident based on the discussion with respect to the training data of Figs. 15E and 16C.

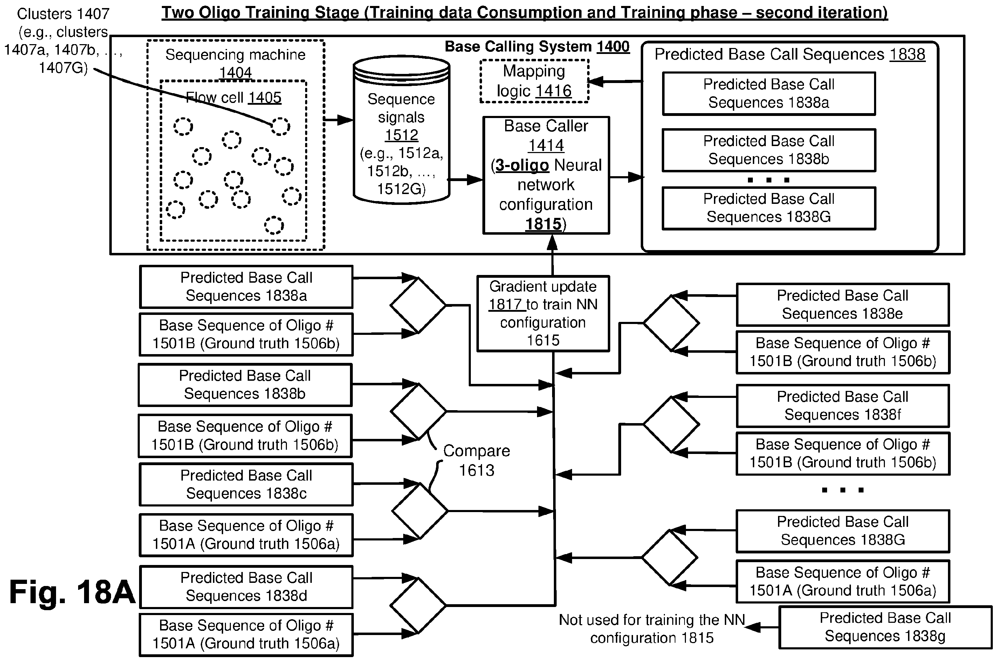

**Fig. 18A** illustrates the base calling system 1400 of Fig. 14A operating in a first iteration of a "training data consumption and training phase" of a "three-oligo training stage," to train the base caller 1414 comprising a 3-oligo neural network configuration 1815. The reasons for labelling the neural network configuration 1815 as a "3-oligo" neural network configuration 1815 would be apparent herein later. Fig. 18A is at least in part similar to Fig. 16D. However, unlike Fig. 15D, labelled training data 1750 (see Fig. 17B) generated at the end of method 1700 (*e.g.*, by the $P^{th}$ NN configuration that used 2-oligo based training) is used during the training in Fig. 18A.

For example, in Fig. 18A, the base caller 1414 comprising the 3-oligo neural network configuration 1815 predicts base call sequences 1838a, 1838b, ..., 1838G. The mapped training data 1750 of Fig. 17B is now used to further train the 3-oligo neural network configuration 1815, similar to the training discussed with respect to Fig. 16D.

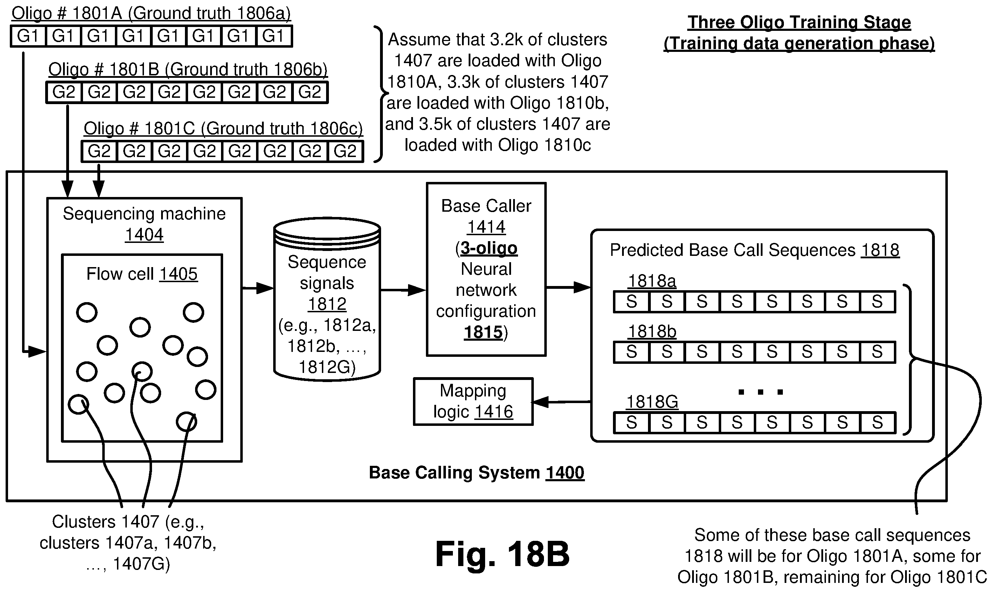

**Fig. 18B** illustrates the base calling system 1400 of Fig. 14A operating in a "training data generation phase" of the "three-oligo training stage," to train the base caller 1414 comprising the 3-oligo neural network configuration 1815 of Fig. 18A.

In Fig. 18B, three different oligo sequences 1801A, 1801B, and 1801C are loaded in various clusters of the flow cell 1405. Merely as an example and without limiting the scope of this disclosure, assume that out of the 10,000 clusters 1407, about 3,200 clusters include oligo sequences 1801A, about 3,300 clusters include oligo sequences 1801B, and remaining 3,500 clusters include oligo sequences 1501C (although in another example, the three oligos can be substantially equally divided among the 10,000 clusters).

The sequencing machine 1404 generates sequence signals 1812a, ..., 1812G for corresponding ones of the plurality of clusters 1407a, ..., 1407G. For example, for a cluster 1407a, the sequencing machine 1404 generates corresponding sequence signal 1812a indicative of bases for the cluster 1407a for a series of sequencing cycles. Similarly, for a cluster 1407b, the sequencing machine 1404 generates corresponding sequence signal 1812b indicative of bases for the cluster 1407b for a series of sequencing cycles, and so on.

The base caller 1414 comprising the neural network configuration 1815 predicts base call sequences 1818a, ..., 1818G for corresponding ones of the plurality of clusters 1407a, ..., 1407G, based on the corresponding sequence signals 1812a, ..., 1812G, respectively, *e.g.*, as discussed with respect to Fig. 15A.

In an embodiment, the oligo sequences 1801A, 1801B, and 1801C are selected to have sufficient edit distances between the bases of the three oligos, *e.g.,* as will be evident based on the discussion with respect to Figs. 15B and 15C. For example, any of the three oligo sequences 1801A, 1801B, and 1801C is to be separated from another of the three oligo sequences 1801A, 1801B, and 1801C by at least a threshold edit distance. Merely as an example, the threshold edit distance can be 4 bases, 5 bases, 6 bases, 7 bases, or even 8 bases. Thus, the three oligos are selected such that the three oligos are sufficiently different from each other.

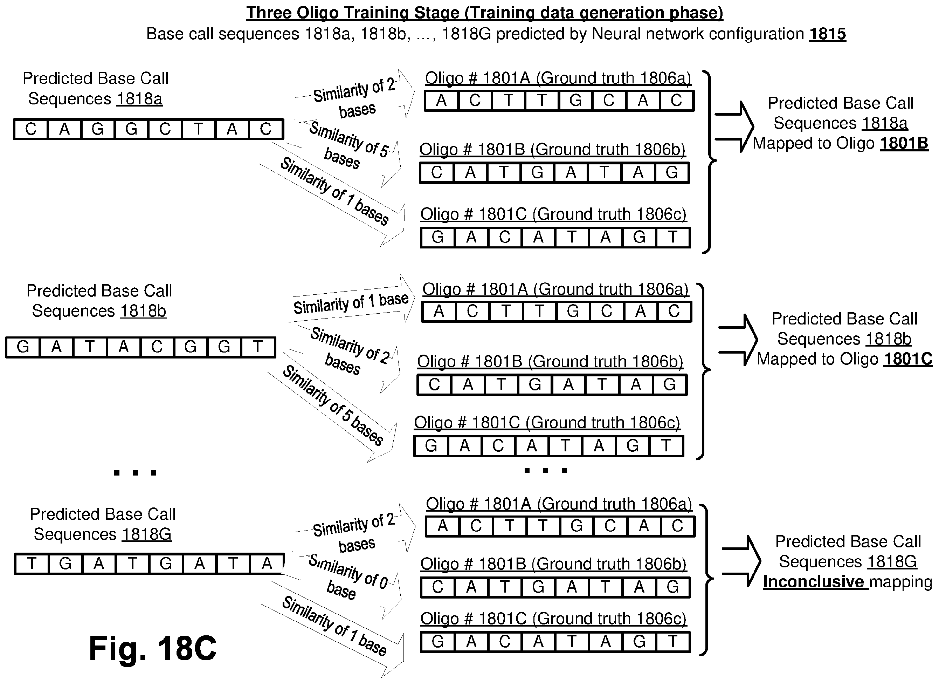

Referring again to Fig. 18B, in an example, the base caller 1414 is unaware as to which oligo sequence is populated in which cluster. Thus, the base caller 1414 is unaware of a mapping between the known oligo sequences 1801A, 1801B, and 1801C and the various clusters. The mapping logic 1416 receives the predicted base call sequences 1818, and maps each predicted base call sequence 1818 to one of the oligos 1801A, 1801B, or 1801C, or declares inconclusiveness in mapping the predicted base call sequence to any of the three oligos. **Fig. 18C** illustrates mapping operations to either (i) map a predicted base call sequence to any of the three oligos 1801A, 1801B, 1801C, or (ii) declare the mapping of the predicted base call sequence to any of the three oligos to be inconclusive.

As illustrated in Fig. 18C, the predicted base call sequence 1818a has similarity of 2 bases with oligo 1801A, has similarity of 5 bases with oligo 1801B, and has similarity of 1 base with oligo 1801C. Assuming a threshold similarity ST of 4 (*e.g.*, discussed with respect to equations 1-4), the predicted base call sequence 1818a is mapped to oligo 1801B.

Similarly, in the example of Fig. 18C, the predicted base call sequence 1818b is mapped to oligo 1801C, and the mapping of the predicted base call sequence 1818a is declared inconclusive by the mapping logic 1416 of FIG. 18B.

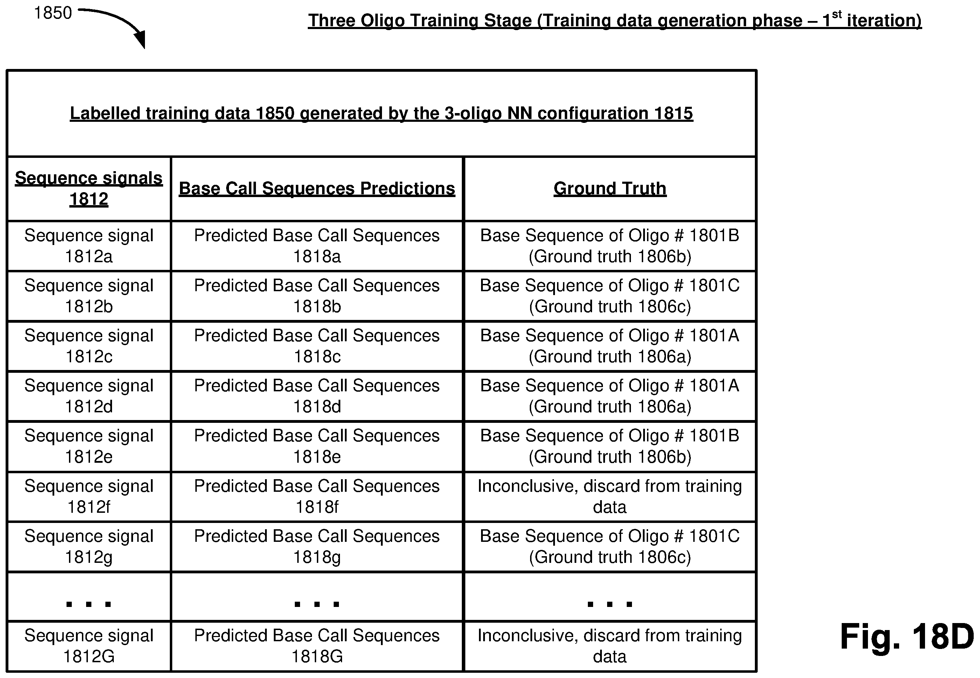

**Fig. 18D** illustrates labelled training data 1850 generated from the mapping of Fig. 18C, where the training data 1850 is used to train another neural network configuration. As illustrated in Fig. 18D, some of the predicted base call sequences 1818 and corresponding sequence signals are mapped to the base sequence of oligo 1801A (*i.e.*, ground truth 1806a), some of the predicted base call sequences 1818 and corresponding sequence signals are mapped to the base sequence of oligo 1801B (*i.e.,* ground truth 1806b), some of the predicted base call sequences 1818 and corresponding sequence signals are mapped to the base sequence of oligo 1801C (*i.e.,* Ground truth 1506c), and mapping of remaining of the predicted base call sequences 1818 and corresponding sequence signals are inconclusive. The training data 1850 of Fig. 18D will be apparent based on the discussion with respect to the training data 1550 of Fig. 15E herein earlier.

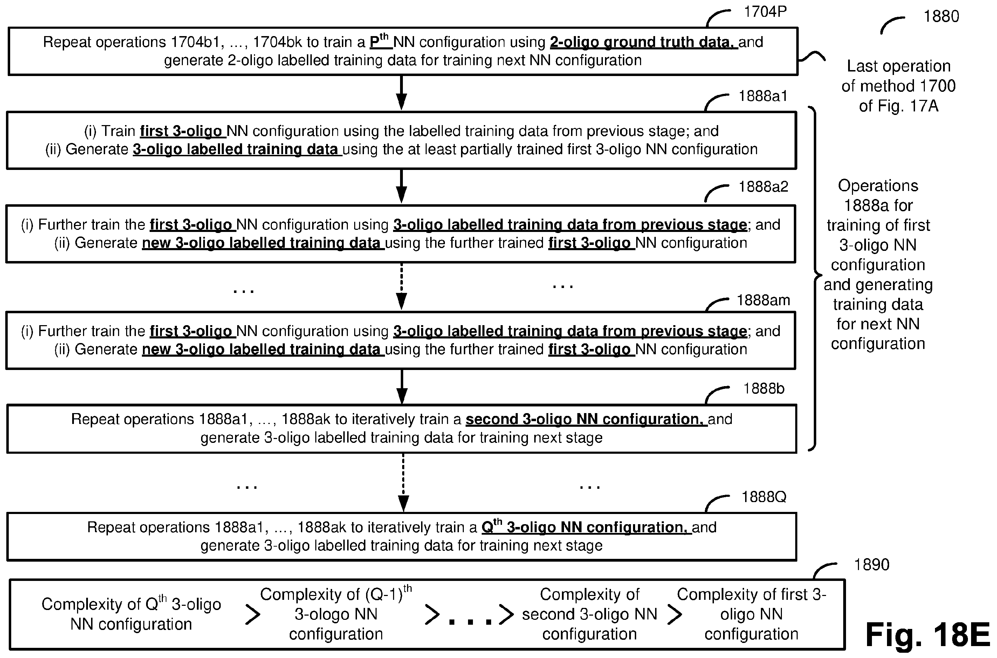

**Fig. 18E** illustrates a flowchart depicting an example method 1880 for iteratively training neural network configurations for base calling using 3-oligo ground truth sequences. The method 1800 progressively trains 3-oligo NN configurations that are progressively and monotonically complex in nature. Increasing a complexity of NN configurations can include increasing a number of layers of the NN configuration, increasing a number of filters of the NN configuration, increasing a topology complexity in the NN configuration, and/or the like, as also discussed with respect to Fig. 17A. For example, the method 1880 refers to a first 3-oligo NN configuration (which is the 3-ologo NN configuration 1815 discussed herein earlier with respect to Fig. 18A), a second 3-oligo NN configuration, a $Q^{th}$ NN configuration, and so on. In an example, complexity of the $Q^{th}$ 3-oligo NN configuration is higher than that of the $(Q-1)^{th}$ 3-oligo NN configuration, which is higher than that of the $(Q-2)^{th}$ 3-oligo NN configuration, and so on, and complexity of the second 3-oligo NN configuration is higher than that of the first 3-oligo NN configuration, as symbolically illustrated within box 1890 of Fig. 18E.

Note that in method 1880 of Fig. 18E, operations 1704P is from the last block of method 1700 of Fig. 17A, operations 1888a1-1888am are for iteratively training the first 3-oligo NN configuration and generating labelled training data for a second 3-oligo NN configuration, and operations 1888b are for iteratively training the second 3-oligo NN configuration and generating labelled training data for a third 3-oligo NN configuration, and so on. This process continues, and operations 1888Q is for training a $Q^{th}$ 3-oligo NN configuration and generating labelled training data for training a subsequent NN configuration. Thus, generally speaking, in the method 1880, operations 1888i are for training an $i^{th}$ 3-oligo NN configuration and generating labelled training data for a $(i+1)^{th}$ 3-oligo NN configuration, where i = 1, ..., Q.

The method 1880 comprises, at 1704P, repeating operations 1704b1, ..., 1704bk to train a $P^{th}$ NN configuration using 2-oligo ground truth data, and generating 2-oligo labelled training data for training next NN configuration, which is the last block of method 1700 of Fig. 17A.

The method 1880 then proceeds from 1704P to 1888a1. As illustrated, operations 1888a are for training the first 3-oligo NN configuration (*e.g.,* 3-oligo Neural network configuration 1815) using the labelled training data (*e.g.,* training data 1750 of Fig. 17B) generated from the previous block (*e.g.,* block 1704P), and using the trained first 3-oligo NN configuration to generate further 3-oligo labelled training data for subsequent training of the second 3-oligo NN configuration. The operations 1888a comprises sub-operations at blocks 1888a1-1888am.

At block 1888a1, (i) the first 3-oligo NN configuration (*e.g.*, 3-oligo NN configuration 1815 of Fig. 18A) is trained using the labelled training data generated at 1704P, and (ii) 3-oligo labelled training data is generated using the at least partially trained first 3-oligo NN configuration (such as training data 1850 of Fig. 18D).

The method 1880 then proceeds from 1888a1 to 1888a2. At block 1888a2, (i) the first 3-oligo NN configuration is further trained using the 3-oligo labelled training data generated at the previous stage (*e.g.,* generated at block 1888a1), and (ii) new 3-oligo labelled training data is generated using the further trained first 3-oligo NN configuration.

The operations discussed with respect to block 1888a2 (and block 1888a2) are iteratively repeated at 1888a3, ..., 1888am. Note that blocks 1888a1, ..., 1888am are all for training the first 3-oligo NN configuration. The number of iterations "m" can be implementation specific, and example criteria used to select the number of iterations for training a specific NN model has been discussed with respect to the method 1700 of Fig. 17A (*e.g.,* selection of the number of iterations "k" in this method).

After the first 3-oligo NN configuration is adequately or satisfactorily trained at 1888am, the method 1888 proceeds to block 1888b, where a second 3-oligo NN configuration is iteratively trained. The training of the second 3-oligo NN configuration would also include iterations similar to those discussed with respect to operations 1888a1, ..., 1888am, and hence, is not discussed in further detail.

This process of progressively training more complex NN configurations continues, until at 1888Q of the method 1888, a $Q^{th}$ 3-ologo NN configuration is trained, and corresponding 3-oligo training data is generated for training the next NN configuration.

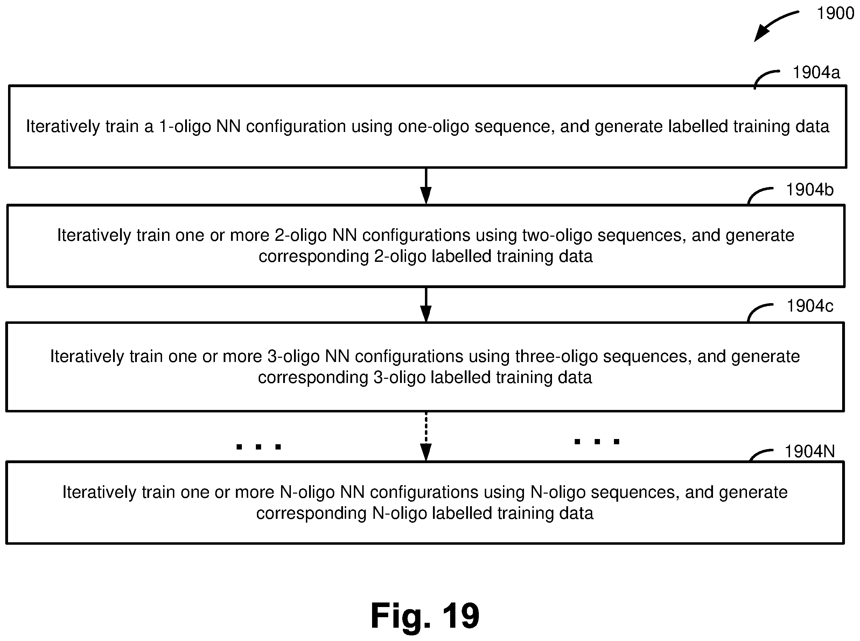

**Fig. 19** illustrates a flowchart depicting an example method 1900 for iteratively training neural network configurations for base calling using multiple-oligo ground truth sequences. Fig. 19, in essence, summarizes the discussion with respect to Figs. 14A-18E. For example, Fig. 19 illustrates iterative training and labelled training data generation process using different oligo stages, such as a single-oligo stage, a two-oligo stage, a three-oligo stage, and so on. Thus, a complexity and/or a length of the analyte used for the training and generation of labelled training data progressively and monotonically increases with the iterations, along with a complexity of the underlying neural network configuration of the base caller.

The method 1900 comprises, at 1904a, iteratively training a 1-oligo NN configuration, and generating labelled training data, *e.g.,* as discussed with respect to Figs. 14A and 14B and block 1704a of method 1700 of Fig. 17A.

The method 1900 further comprises, at 1904b, iteratively training one or more 2-oligo NN configurations using two-oligo sequences, and generating labelled 2-oligo training data, *e.g.,* as discussed with respect to blocks 1704b1-1704P of method 1700 of Fig. 17A.

The method 1900 further comprises, at 1904c, iteratively training one or more 3-oligo NN configurations using three-oligo sequences, and generating labelled 3-oligo training data, *e.g.,* as discussed with respect to blocks 1888a1-1888Q of method 1880 of Fig. 18E.

This process continues and higher number of oligo sequences may be progressively used. Finally, at 1904N, one or more N-oligo NN configurations are trained using N-oligo sequences, and corresponding N-oligo labelled training data are generated, where N can be an appropriate positive integer that is greater than or equal to 2. The operations at 1904N would be apparent based on the discussion with respect to operations at 1904b and 1904c.

Figs. 14A-19 are associated with training NN models with synthetically sequenced simple oligo sequences. For example, an oligo sequence used in these figures is likely to have fewer number of bases compared to sequences found in DNA of organisms. In an embodiment, the oligo-based training discussed with respect to Figs. 14A-19 are used to train progressively complex NN models and to generate progressive rich labelled training data set. For example, Fig. 19 outputs N-oligo labelled training data set using N-oligo NN configuration, where the N-oligo labelled training data set may have much richer, diverse, and larger labelled training data set than labelled training data set associated with "less than N" number of oligos.

However, in practice the sequencing machine 1404 and the base caller 1414 are to base call sequences that are far more complex than simple oligos sequences. For example, in practice the sequencing machine 1404 and the base caller 1414 are to base call organism sequences that are far more complex than simple oligos sequences. Accordingly, the base caller 1414 has to be trained on base sequences found in organism DNAs and RNAs that are more complex than oligo sequences.

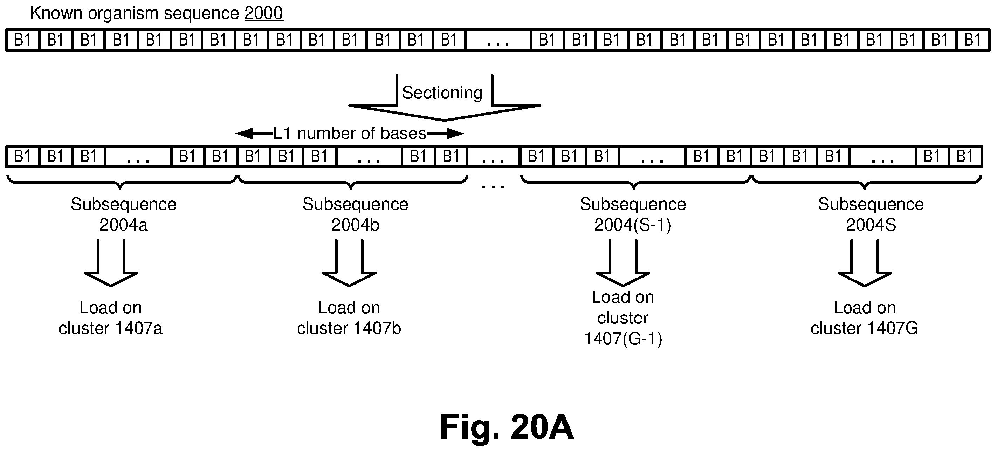

**Fig. 20A** illustrates an organism sequence 2000 to be used to train the base caller 1414 of Fig. 14A. The organism sequence can be of an organism that has relatively fewer bases, such as phix (also referred to as phi X). The phix bacteriophage is a single-stranded DNA (ssDNA) virus. The phix 174 bacteriophage is a ssDNA virus that infects Escherichia coli, and the first DNA-based genome to be sequenced in year 1977. The phix (such as ΦX174) virus particle has also been successfully assembled *in vitro.* In an embodiment, after training the base caller 1414 with the oligo sequences (as discussed with respect to Figs. 14A-19), the base caller 1414 can be further trained with simple organism DNA, such as phix DNA, although this does not limit the scope of this disclosure. For example, instead of phix, a more complex organism, such as a bacterium (such as Escherichia coli or E-coli bacteria) can be used. Thus, the organism sequence 2000 can be phix, or another relatively simple organism DNA. The organism sequence 2000 is pre-sequenced, *i.e.,* the base sequence of the organism sequence 2000 is known *a-priori* (e.g., sequenced by a sequencing machine and an already trained base caller that are different from those illustrated in Fig. 14A).

As illustrated in Fig. 20A, when loading the organism sequence 2000 in the sequencing machine 1404 of Fig. 14A, the organism sequence 2000 is partitioned or sectioned in multiple subsequences 2004a, 2004b, ..., 2004N. Each subsequence is loaded in corresponding one or more clusters. Thus, each cluster 1407 is populated with a corresponding subsequence 2004 and synthesized copies thereof. Any appropriate criteria can be used to section the organism sequence 2000, *e.g.,* a maximum size of a subsequence that a cluster can be populated with. For example, if individual clusters of the flow cell can be populated with subsequences having a maximum of about 150 bases, then the sectioning can be performed accordingly such that individual ones of the subsequence 2004 has at most 150 bases. In an example, individual subsequences 2004 can have substantially equal number of bases; whereas in another example, individual subsequences 2004 can have different number of bases. The subsequence 2004b, which is used as an example to discuss the teachings of this disclosure, is assumed to have L1 number of bases. Merely as an example, the number L1 can be between 100 and 200, although it can have any other appropriate value and is implementation specific.

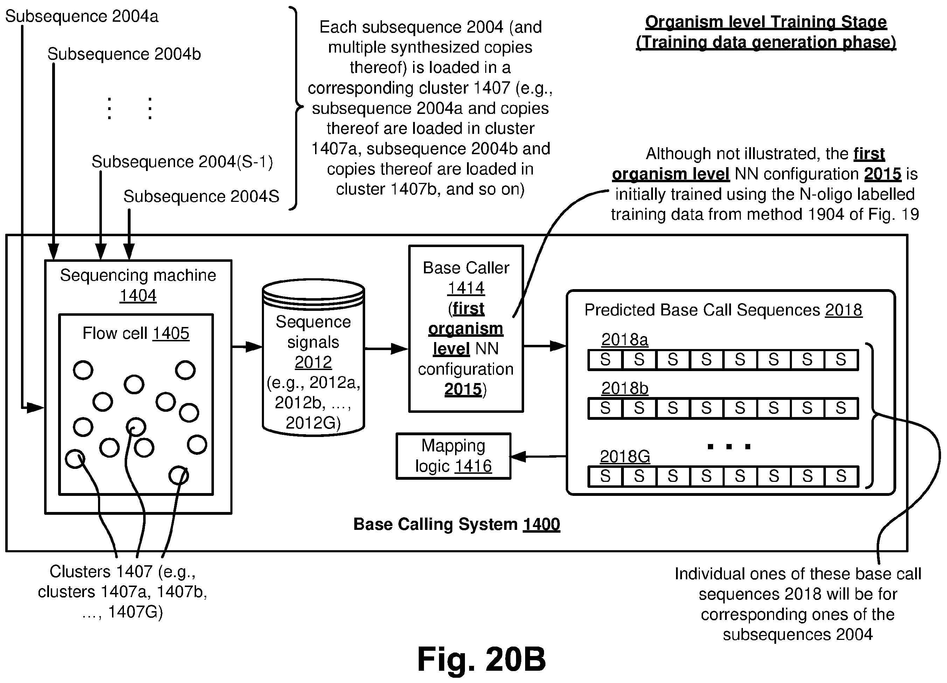

**Fig. 20B** illustrates the base calling system 1400 of Fig. 14A operating in a training data generation phase of a first organism training stage, to train the base caller 1414 comprising a first organism level neural network configuration 2015, using subsequences 2004a, ..., 2004S of a first organism sequence 2000 of Fig. 20A.

Note that although not illustrated in Fig. 20B, the first organism level NN configuration 2015 is initially trained using the N-oligo labelled training data from method 1904 of Fig. 19. Thus, the first organism level NN configuration 2015 is at least partially pre-trained. The base calling system 1400 of Fig. 20B is the same as the base calling system of Fig. 14A, although in the two figures the base calling system 1400 uses different neural network configurations and different analytes.

As discussed, the subsequences 2004a, ..., 2004S are loaded into corresponding clusters 1407. For example, subsequence 2004a is loaded in the cluster 1407a, subsequence 2004b is loaded in the cluster 1407b, and so on. Note that each cluster 1407 will include multiple sequenced copies of the same subsequence 2004. For example, a subsequence loaded in a cluster will be synthetically replicated, such that the cluster has multiple copies of the same subsequence, which helps in generating a corresponding sequence signal 2012 for the cluster.

Note that the base caller 1414 is unaware of which cluster is populated with which subsequence. For example, if the subsequence 2004a and synthesized copies thereof are loaded in a specific cluster, the base caller 1414 would be unaware of the cluster that is populated by the subsequence 2004a. As will be discussed herein later, the mapping logic 1416 aims to map individual subsequences 2004 to corresponding clusters 1407, to facilitate the training process.

The sequencing machine 1404 generates sequence signals 2012a, ..., 2012G for corresponding ones of the plurality of clusters 1407a, ..., 1407G. For example, for a cluster 1407a, the sequencing machine 1404 generates corresponding sequence signal 2012a indicative of bases for the cluster 1407a for a series of sequencing cycles. Similarly, for a cluster 1407b, the sequencing machine 1404 generates corresponding sequence signal 2012b indicative of bases for the cluster 1407b for a series of sequencing cycles, and so on.

In an example, although individual subsequences 2004 are loaded in corresponding clusters 1407, the base caller 1414 is unaware of which subsequence in loaded in which cluster. Thus, the base caller 1414 is unaware of a mapping between the subsequences 2004 and the clusters 1407. As each cluster 1407 generates a corresponding sequence signal 2012, the base caller 1414 is unaware of a mapping between the subsequences 2004 and the sequence signals 2012.

The base caller 1414 comprising the neural network configuration 2015 predicts base call sequences 2018a, ..., 2018G for corresponding ones of the plurality of clusters 1407a, ..., 1407G, based on the corresponding sequence signals 2012a, ..., 2012G, respectively. For example, for the cluster 1407a, the base caller 1414 predicts corresponding base call sequence 2018a including base calls for the cluster 1407a for the series of sequencing cycles, based on the corresponding sequence signal 2012a. Similarly, for the cluster 1407b, the base caller 1414 predicts corresponding base call sequence 2018b including base calls for the cluster 1407b for the series of sequencing cycles, based on the corresponding sequence signal 2012b, and so on.

Note that the neural network configuration 2015 is merely partially trained, and not fully trained. So, it may not be possible for the neural network configuration 2015 to correctly predict some or most of the bases of individual subsequences.

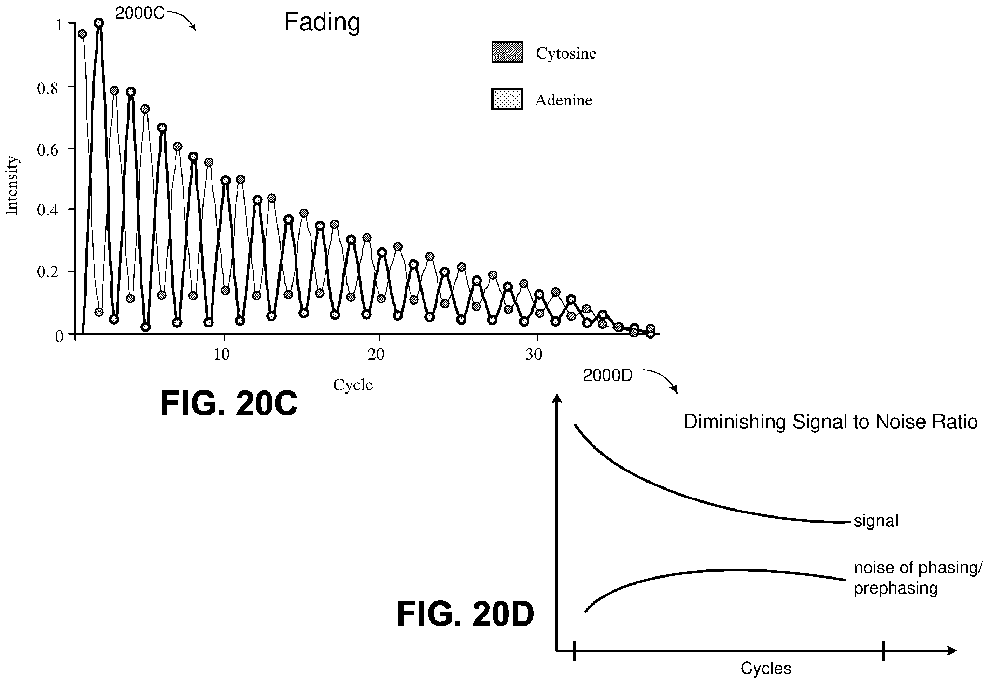

Furthermore, as base calling in a subsequence progresses, bases are increasingly difficult to call, *e.g.,* due to fading and/or noise of phasing or pre-phasing. **Fig. 20C** illustrates an example of fading, in which signal intensity is decreased as a function of cycle number is a sequencing run of a base calling operation. Fading is an exponential decay in fluorescent signal intensity as a function of cycle number. As the sequencing run progress, the analyte strands are washed excessively, exposed to laser emissions that create reactive species, and subject to harsh environmental conditions. All of these lead to a gradual loss of fragments in each analyte, decreasing its fluorescent signal intensity. Fading is also called dimming or signal decay. Fig. 20C illustrates one example of fading 2000C. In Fig. 20C, the intensity values of analyte fragments with AC microsatellites show exponential decay.

**Fig. 20D** conceptually illustrates a decreasing signal-to-noise ratio as cycles of sequencing progress. For example, as sequencing proceeds, accurate base calling becomes increasingly difficult, because signal strength decreases and noise increases, resulting in a substantially decreased signal-to-noise ratio. Physically, it was observed that later synthesis steps attach tags in a different position relative to the sensor than earlier synthesis steps. When the sensor is below a sequence that is being synthesized, signal decay results from attaching tags to strands further away from the sensor in later sequencing steps than in earlier steps. This causes signal decay with progression of sequencing cycles. In some designs, where the sensor is above the substrate that holds the cluster, the signal could increase, instead of decaying, as sequencing proceeds.

In the flow cell design investigated, while the signal decays, noise grows. Physically, phasing and pre-phasing increase noise as sequencing proceeds. Phasing refers to steps in sequencing in which tags fail to advance along the sequence. Pre-phasing refers to sequencing steps in which tags jump two positions forward instead of one, during a sequencing cycle. Phasing and pre-phasing are both relatively infrequent, on the order of once in 500 to 1000 cycles. Phasing is slightly more frequent than pre-phasing. Phasing and pre-phasing impact individual strands in a cluster that is producing intensity data, so the intensity noise distribution from a cluster accumulates in a binomial, trinomial, quadrinomial, etc. expansion as sequencing proceeds.

Further detail of fading, signal decay, and decrease in signal-to-noise ratio, and Figs. 20C and 20D can be found in U.S. Nonprovisional Patent Application No. 16/874,599, titled "Systems and Devices for Characterization and Performance Analysis of Pixel-Based Sequencing," filed May 14, 2020 (Attorney Docket No. ILLM 1011-4/IP-1750-US).

Thus, during base calling, reliability or predictability of the base calling decreases as the sequencing cycles progress. For example, referring to a specific subsequence, such as subsequence 2004b of Fig. 20A, in general, a calling of bases 1 to 10 of the subsequence 2004b may be more reliable than calling bases 10-20 or calling bases 50-60. Put differently, the first few bases of the L1 bases of the subsequence 2004b are likely to be predicted relatively more correctly than the remaining bases of the L1 bases of the subsequence 2004b.

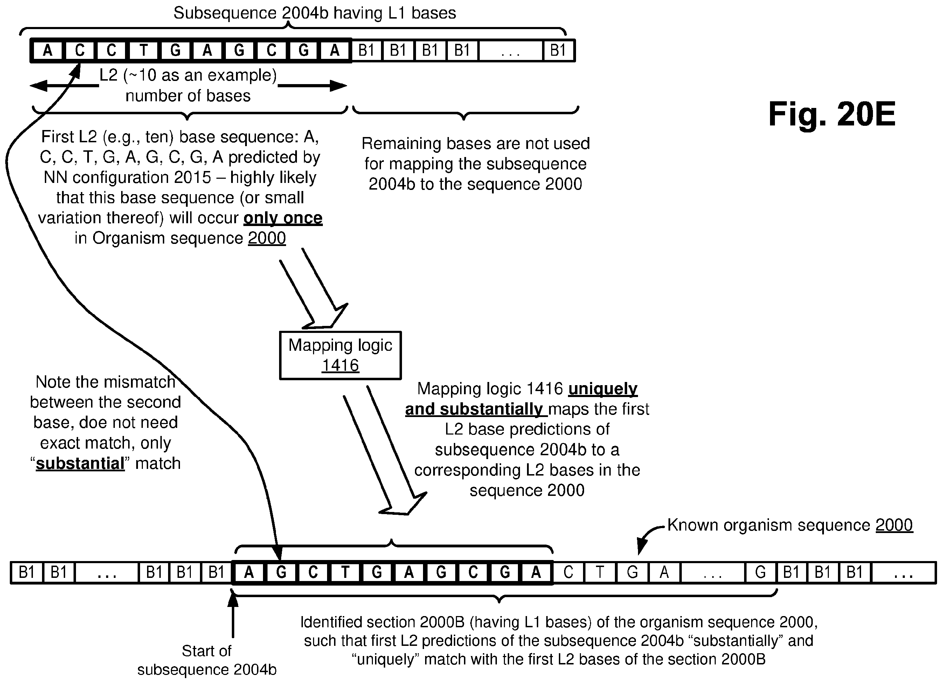

**Fig. 20E** illustrates base calling of a first L2 number of bases of the L1 number of bases of a subsequence 2004b, where the first L2 number of bases of the subsequence 2004b is used to map the subsequence 2004b to the sequence 2000.

For example, referring to Figs. 20A, 20B, and 20E, the sequencing machine 1404 generates sequence signals 2012b corresponding to the subsequence 2004b (*i.e.,* assuming that the subsequence 2004b is populated in cluster 1407b). But the base caller 1414 does not know where the subsequence corresponding to the sequence signal 2012b fits in the sequence 2000. That is, the base caller 1414 does not know that specifically subsequence 2004b is loaded in the cluster 1407b.

As illustrated in Fig. 20E, the partially trained NN configuration 2015 (*e.g.*, which is trained using the N-oligo labelled training data from method 1904 of Fig. 19) receives the sequence signals 2012b, and predicts the L1 bases indicated by the sequence signals 2012b. The prediction of the L1 bases includes prediction of the first L2 bases, where the prediction of the first L2 number of bases of the subsequence 2004b is used to map the subsequence 2004b to the sequence 2000.

In an example, the number L2 is 10. The number L2 can be any appropriate number, such as 8, 10, 12, 13, or the like, as long as L2 is relatively smaller than L1. For example, L2 is less than 10% of L1, less than 25% of L1, or the like.

For example, the first L2 bases of the subsequence 2004b predicted by the NN configuration 2015 is A, C, C, T, G, A, G, C, G, A, as illustrated in Fig. 20E. The prediction of the remining (L1-L2) bases are generically illustrated as B1, ..., B1 in Fig. 20E.

Now, there is a possibility that the NN configuration 2015 has correctly predicted the first L2 number of bases, or there may be one or more errors in these L2 number of base predictions. The mapping logic 1416 tries to map the first L2 number of base predictions to corresponding consecutive L2 bases in the organism sequence 2000. Put differently, the mapping logic 1416 tries to match the first L2 number of base predictions to consecutive L2 bases in the organism sequence 2000, such that the subsequence 2004b within the organism sequence 2000 can be identified.

As illustrated in Fig. 20E, the mapping logic 1416 is able to find a "substantial" and "unique" match between the first L2 number of bases predicted for the subsequence 2004b and consecutive L2 bases in the organism sequence 2000. Note that "substantial" match implies that the match may not be 100%, and there may be one or more errors in the match. For example, the first L2 number of bases of the subsequence 2004b predicted by the NN configuration 2015 is A, C, C, T, G, A, G, C, G, A, whereas the corresponding substantial matching sequential L2 bases in the organism sequence 2000 is A, G, C, T, G, A, G, C, G, A. Thus, the second base in these two L2 base sequences do not match, but the remaining bases match. As long as such number of mismatches is less than a threshold percentage, the mapping logic 1416 declares the two L2 number of base fragments to be matching. The threshold percentage of mismatch may be 10%, or 20%, or some similar percentage of the number L2. So, in an example, L2 is 10 and the matching logic 1416 can tolerate up to 2 mismatches (or 20% mismatch). Thus, the mapping logic 1416 aims to map the first L2 number of bases predicted for the subsequence 2004b, or a slight variation thereof (*e.g.*, where the variation implies an error tolerance during the matching) to consecutive L2 bases in the organism sequence 2000. The value of the threshold percentage can be implementation specific, and can be user configurable. Merely as an example, during initial iterations of the training, the threshold percentage can have a relatively high (such as 20%); and the threshold percentage can have a relatively lower value (such as 10%) during later iterations of the training. Thus, at early stages of the training iterations, the threshold percentage can be relatively high, because of relatively high likelihood of errors in base calling predictions. As and when the NN configurations are better trained, they are likely to make better base calling predictions and hence, the threshold percentage can be gradually lowered. However, in another example, the threshold percentage can be the same throughout all iterations of the training.

Also, in an example, the matching between two L2 number of bases has to be unique for proper mapping, and non-unique matching may result in the matching and mapping being declared as being inconclusive. Thus, the first L2 number of bases predicted for the subsequence 2004b (or slight variation thereof) can occur only once in the organism sequence 2000, for the matching and mapping to be valid. Usually, for practical base sequences of simpler organisms, there is a high likelihood that consecutive L2 bases (or small variation thereof) will occur only once in the organism sequence 2000.

For example, referring to the example of Fig. 20E, if there is an occurrence of consecutive bases A, G, C, T, G, A, G, C, G, A in one section of the organism sequence 2000 and there is another occurrence of consecutive bases A, C, A, T, G, A, G, C, G, A in another section of the organism sequence 2000, arguably, both sections of the organism sequence 2000 can be matched to the first L2 number of bases of the subsequence 2004b predicted by the NN configuration 2015 (which is A, C, C, T, G, A, G, C, G, A). Thus, in this example, the matching is not unique, and the mapping logic 1416 does not know which of the two sections of the organism sequence 2000 to be mapped to the L2 number of bases on the subsequence 2004b. In such a scenario, the mapping logic 1416 declares no reliable matching (*i.e.*, declares inconclusive mapping).

Referring to the example of Fig. 20E, as illustrated, the first L2 number of bases of the subsequence 2004b predicted by the NN configuration 2015 "substantially" and "uniquely" match with corresponding L2 number of consecutive bases of the organism sequence 2000. Also assume a section 2000B (which has L1 bases) of the organism sequence 2000, where the first L2 predictions of the subsequence 2004b "substantially" and "uniquely" matches with the first L2 bases of the section B of the organism sequence 2000. Thus, most likely, the subsequence 2004b is actually the section 2000B of the organism sequence 2000. Put differently, most likely, section 2000B of the organism sequence 2000 was sectioned in Fig. 20A to form the subsequence 2004b.

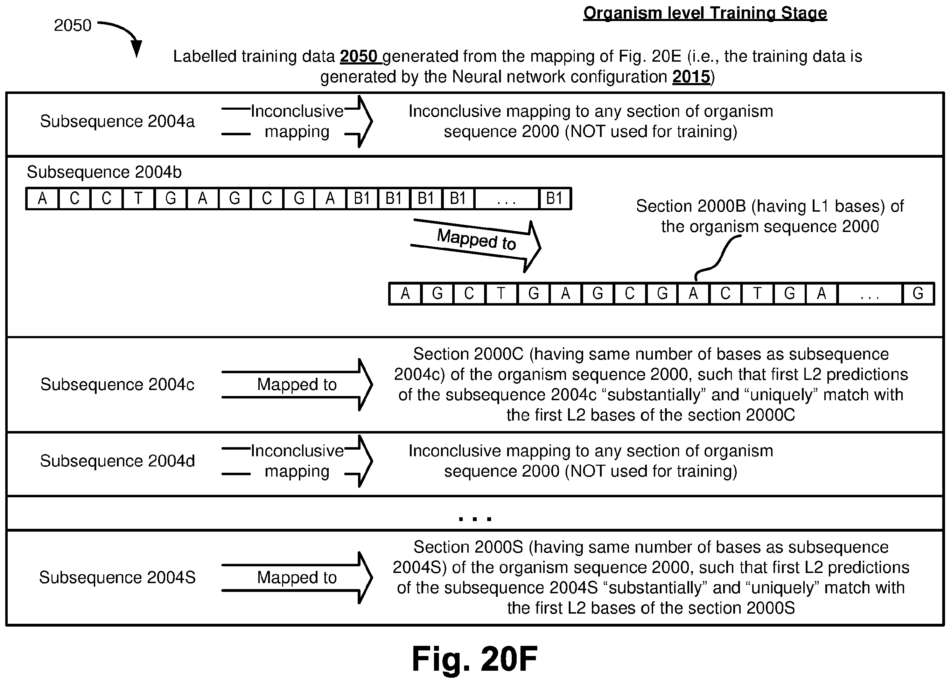

Accordingly, the section 2000B of the organism sequence 2000 acts as a ground truth for the sequence signal 2012b corresponding to the subsequence 2004b. **Fig. 20F** illustrates labelled training data 2050 generated from the mapping of Fig. 20E, wherein the labelled training data 2050 includes sections of organism sequence 2000 of Fig. 20A as ground truth.

In the labelled training data 2050 of Fig. 20F, merely as an example, subsequences 2004a, 2004d are not mapped to any section of the organism sequence 2000, due to inconclusive mapping. For example, as discussed with respect to Fig. 20E, there has to be substantial and unique matching between first L2 bases of a subsequence and a corresponding section of the organism sequence 2000, for the mapping logic 1416 to declare a conclusive mapping. The NN configuration 2015 may have made relatively higher number of errors in the first L2 bases of each of the subsequences 2004a, 2004d, as a result of which these subsequences cannot be mapped to any corresponding section of the organism sequence 2000.

In the labelled training data 2050 of Fig. 20F, subsequence 2004b (and hence, the sequence signal 2012b) is mapped to the section 2000B of the organism sequence 2000, as discussed with respect to Fig. 20E. Similarly, subsequence 2004c is mapped to a section 2000C of the organism sequence 2000 and subsequence 2004S is mapped to a section 2000S of the organism sequence 2000. For example, subsequence 2004c is mapped to a section 2000C (*e.g.*, having the same number of bases as subsequence 2004c) of the organism sequence 2000, such that first L2 base predictions of the subsequence 2004c "substantially" and "uniquely" match with the first L2 bases of the section 2000C.

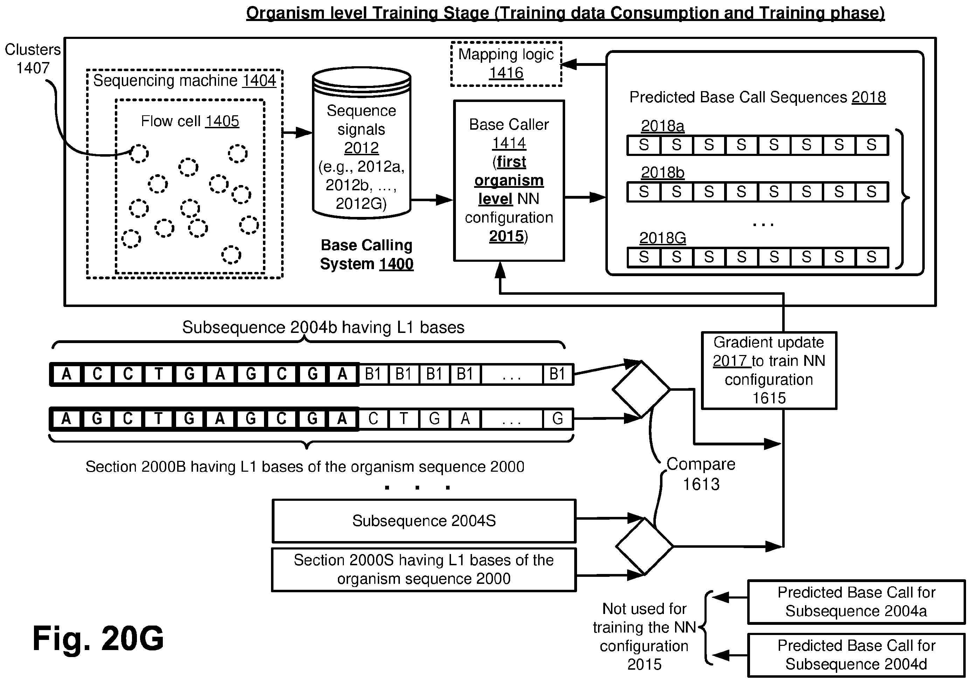

**Fig. 20G** illustrates the base calling system 1400 of Fig. 14A operating in a "training data consumption and training phase" of the "organism level training stage," to train the base caller 1414 comprising the first organism level neural network configuration 2015. For example, the labelled training data 2050 of Fig. 20F are used in the training of Fig. 20G.

For example, the L1 bases of the subsequence 2004b predicted by the base caller 1414 is compared to the section 2000B of the organism sequence 2000. Note that the L1 bases of the subsequence 2004b predicted by the base caller 1414 has the first L2 bases that were compared with the organism sequence 2000 to generate the mapping of Fig. 20F. The remaining (L1-L2) bases were not compared while generating the mapping of Fig. 20F, as the remaining (L1-L2) bases were likely to include many errors. This is because, as discussed with respect to Figs. 20C and 20D, the bases occurring later in a subsequence have higher chances of being predicted erroneously, due to fading, phasing and/or pre-phasing. In Fig. 20G, the entire L1 bases of the subsequence 2004b predicted by the base caller 1414 are compared to corresponding L1 bases on the section 2000B of the organism sequence 2000.

Thus, the mapping of Fig. 20F specifies a portion of the organism sequence 2000 (*i.e.*, the section 2000B) with which the subsequence 2004b is to be compared to in Fig. 20G. Once the mapping is completed and labelled training data 2050 are generated, the labelled training data 2050 are used in Fig. 20G for comparison and generation of error signals, which are used for gradient update 2017 in the backward pass of the NN configuration 2015 and resultant training of the NN configuration 2015.

Note that some of the subsequences (such as subsequences 2004a and 2004d, see Fig. 20F) were not conclusively matched to corresponding sections of the organism sequence 2000, and hence, base call predictions corresponding to these subsequences are not used in the training of Fig. 20G.

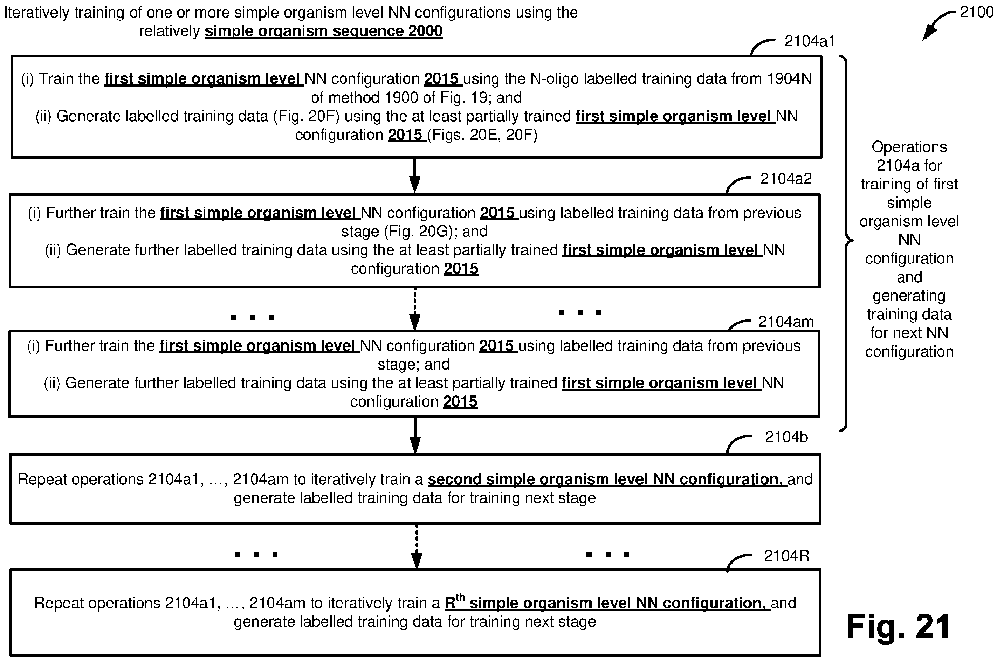

**Fig. 21** illustrates a flowchart depicting an example method 2100 for iteratively training neural network configurations for base calling using the simple organism sequence 2000 of Fig. 20A. The method 2100 progressively trains NN configurations that are monotonically complex in nature. As previously discussed herein, increasing a complexity of NN configurations can include increasing a number of layers of the NN configurations, increasing a number of filters of the NN configurations, increasing a topology complexity in the NN configurations, and/or the like. For example, the method 2100 refers to a first organism level NN configuration (which is the NN configuration 2015 discussed herein earlier with respect to Fig. 20B, 20G and other figures), a second organism level NN configuration, a $R^{th}$ organism level NN configuration, and so on. In an example, complexity of the $R^{th}$ organism level NN configuration is higher than that of the $(R-1)^{th}$ organism level NN configuration, which is higher than that of the $(R-2)^{th}$ organism level NN configuration, and so on, and complexity of the second organism level NN configuration is higher than that of the first organism level NN configuration.

Note that in method 2100, operations 2104a (which comprises blocks 2104a1, ..., 2104am) are for training the first organism level NN configuration and generating labelled training data for the second organism level NN configuration, operations 2104b are for training the second organism level NN configuration and generating labelled training data for a third organism level NN configuration, and so on. This process continues, and finally operations 2104R are for training the $R^{th}$ organism level NN configuration and generating labelled training data for next stage NN configuration. Thus, generally speaking, in the method 2100, operations 2104i are for training an $i^{th}$ organism level NN configuration and generating labelled training data for a $(i+1)^{th}$ organism level NN configuration, where i = 1, ..., R.

The method 2100 comprises, at 2104a1, (i) training the first organism level NN configuration (*e.g.*, the organism level NN configuration 2015 of Fig. 20B, although the training of this NN configuration is not illustrated in Fig. 20B) using the N-oligo labelled training data from 1904N of method 1900 of Fig. 19, and (ii) generating labelled training data using the at least partially trained first organism level NN configuration 2015. The labelled training data is illustrated in Fig. 20F, generation of which is discussed with respect to Figs. 20E and 20F.

The method 2100 then proceeds from 2104a1 to 2014a2, during which a second iteration of training the first organism level NN configuration 2015 is performed. For example, at 2104a2, (i) the first organism level NN configuration 2015 is further trained using labelled training data from previous stage, *e.g.,* as discussed with respect to Fig. 20G; and (ii) further labelled training data is generated using the at least partially trained first organism level NN configuration 2015 (*e.g.*, similar to the discussion with respect to Figs. 20E and 20F).

The training and generation operations are iteratively repeated, and finally at 2104am, the training of the first organism level NN configuration 2015 is completed. Note that block 2014a1 is a first iteration of training the first organism level NN configuration 2015, block 2104a2 is a second iteration of training the first organism level NN configuration 2015, and so on, and finally block 2104am is a $m^{th}$ iteration of training the first organism level NN configuration 2015. The number of iterations can be based on one or more factors, such as those previously discussed herein with respect to the method 1700 of Fig. 17A (*e.g.*, where criteria for selecting number of iterations "k" were discussed). The complexity of the first organism level NN configuration 2015 does not change during the iterations of 2104a1, ..., 2104am.

At the end of the iterations for the first organism level NN configuration 2015 (*i.e.*, at the end of block 2104am), the method 2100 proceeds to block 2104b, where the second organism level NN configuration is now iteratively trained. The training of the second organism level NN configuration and associated generation of training labelled data would also include iterations similar to those discussed with respect to operations 2104a1, ...., 2104am, and hence, is not discussed in further detail.

This process of progressively training more complex NN configurations associated generation of training labelled data continues, until at 2104R of the method 2100, a $R^{th}$ organism level NN configuration is trained, and corresponding labelled training data is generated for training next NN configuration.

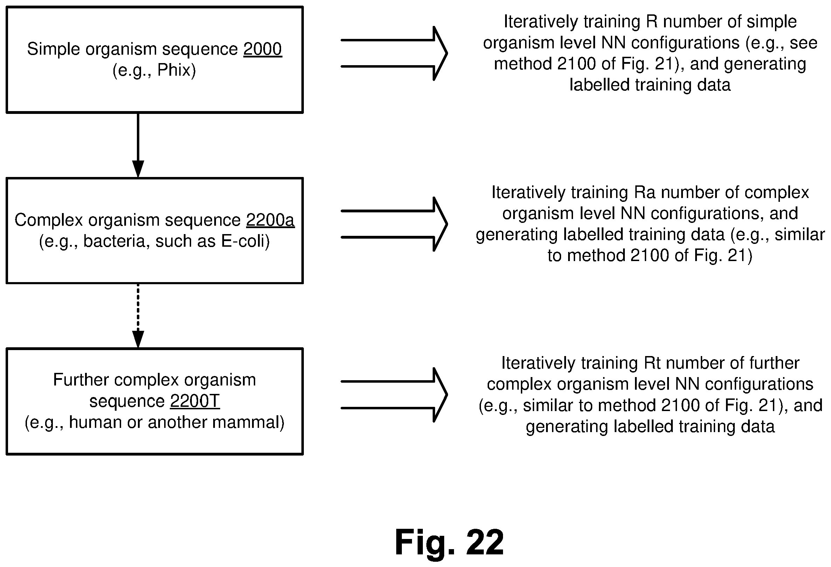

**Fig. 22** illustrates usage of complex organism sequences for training of corresponding NN configurations for the base caller 1414 of Fig. 14A. For example, as discussed with respect to Figs. 20A-21, relatively simple organism sequence 2000 comprising about L1 number of bases per subsequence are used to iteratively train R number of simple organism level NN configurations, and generate corresponding labelled training data. For example, method 2100 of Fig. 21 illustrates such iterative training and generation of the labelled training data using the simple organism sequence 2000. As discussed, the simple organism sequence 2000 can be Phix or another organism that has relatively simple (or relatively small) genetic sequence.

Also illustrated in Fig. 22 is use of relatively complex organism sequence 2200a. The organism sequence 2200a is more complex than the organism sequence 2000 as, for example, a number of bases in the complex organism sequence 2200a is higher than a number of bases in the organism sequence 2000. Merely as an example, the organism sequence 2000 can have about 1 million bases and the complex organism sequence 2200a can have 4 million bases. In another example, each subsequence sectioned from the complex organism sequence 2200a has a higher number of bases than that of each subsequence sectioned from the organism sequence 2000. In yet another example, a number of subsequences sectioned from the complex organism sequence 2200a is higher than a number of subsequences sectioned from the organism sequence 2000. For example, when sectioning the complex organism sequence 2200a and the organism sequence 2000, the number of subsequences sectioned from the complex organism sequence 2200a would be higher than the number of subsequences sectioned from the organism sequence 2000, because (i) the complex organism sequence 2200a has a higher number of bases than the organism sequence 2000 and (ii) each subsequence can have at most a threshold number of bases. In an example, the complex organism sequence 2200a comprises genetic material from bacteria, such as E-coli, or another appropriate organism sequence that is more complex than the organism sequence 2000.

As illustrated in Fig. 22, the complex organism sequence 2200a is used to iteratively train Ra number of complex organism level NN configurations and generate labelled training data. The training and generation of the labelled training data are similar to those discussed with respect to method 2100 of Fig. 21 (the difference being that the method 2100 is specifically directed towards organism sequence 2000, whereas here complex organism sequence 2200a is used).

This iterative process continues, and finally relatively further complex organism sequence 2200T is used. The further complex organism sequence 2200T is more complex than the organism sequences 2000 and 2200a. For example, a number of bases in the further complex organism sequence 2200T is higher than a number of bases in each of the organism sequences 2000 and 2200a. In another example, each subsequence sectioned from the further complex organism sequence 2200T has a higher number of bases than each subsequence sectioned from the organism sequences 2000 or 2200a. In yet another example, a number of subsequences sectioned from the further complex organism sequence 2200T is higher than a number of subsequences sectioned from the organism sequences 2000 or 2200a. In an example, the further complex organism sequence 2200T comprises genetic material from complex species, such as genetic material from human or other mammals.

As illustrated in Fig. 22, the organism sequence 2200T is used to iteratively train RT number of further complex organism level NN configurations and generate labelled training data. The training and generation of the labelled training data are similar to those discussed with respect to method 2100 of Fig. 21 (the difference being that the method 2100 is specifically directed towards organism sequence 2000, whereas here organism sequence 2000T is used).

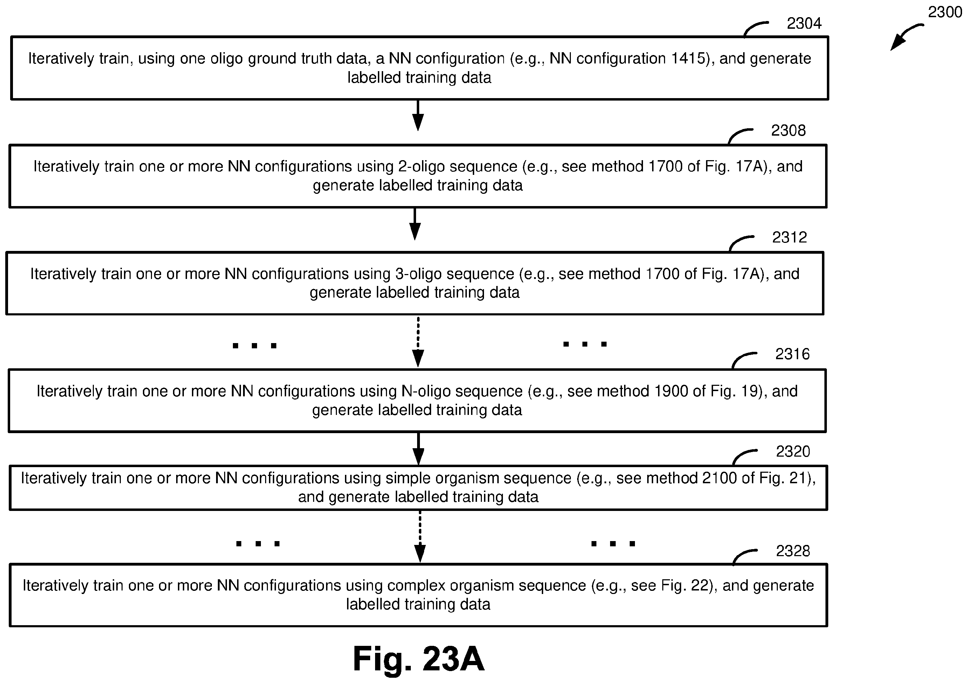

**Fig. 23A** illustrates a flowchart depicting an example method 2300 for iteratively training neural network configurations for base calling. The method 2300 summarizes at least some of the embodiments and examples discussed herein with respect to Figs. 14A-22. The method 2300 trains NN configurations that are monotonically complex in nature, as discussed herein. The method 2300 also monotonically uses complex genetic sequences as analytes. The method 2300 is used to train the base caller 1414 of various figures discussed herein.

The method 2300 commences at 2304, where the base caller 1414 comprising the NN configuration 1415 (*e.g.,* see Fig. 14A) is iteratively trained using a single oligo ground truth data, as discussed with respect to block 1704 of method 1700 of Fig. 17A. The at least partially trained NN configuration 1415 of Fig. 14A is used to generate labelled training data, as also discussed with respect to block 1704 of method 1700 of Fig. 17A.

The method 2300 then proceeds from 2304 to 2308, where one or more NN configurations are iteratively trained using 2-oligo sequences, and corresponding labelled training data are generated, *e.g.,* as discussed with respect to method 1700 of Fig. 17A.

The method 2300 then proceeds from 2308 to 2312, where one or more NN configurations are iteratively trained using 3-oligo sequences, and corresponding labelled training data are generated, *e.g.,* as discussed with respect to method 1900 of Fig. 19.

This process of training NN configurations using progressively higher number of oligos continue, until at 2316, one or more NN configurations are iteratively trained using N-oligo sequences, and corresponding labelled training data are generated, *e.g.*, as discussed with respect to method 1900 of Fig. 19.

The method 2300 then transitions to 2320, where the training and labelled training data generation involves organisms. At 2320, a simple organism sequence, such as simple organism sequence 2000 of Fig. 20A, is used. One or more NN configurations are trained using the simple organism sequence (*e.g.,* see method 2100 of Fig. 21), and labelled training data are generated.

As the method 2300 proceeds from 2320, increasingly complex organism sequences are used, *e.g.,* as discussed with respect to Fig. 22. Finally, at 2328, one or more NN configurations are iteratively trained using complex organism sequence (*e.g.*, further complex organism sequence 2200T of Fig. 22), and corresponding labelled training data are generated.

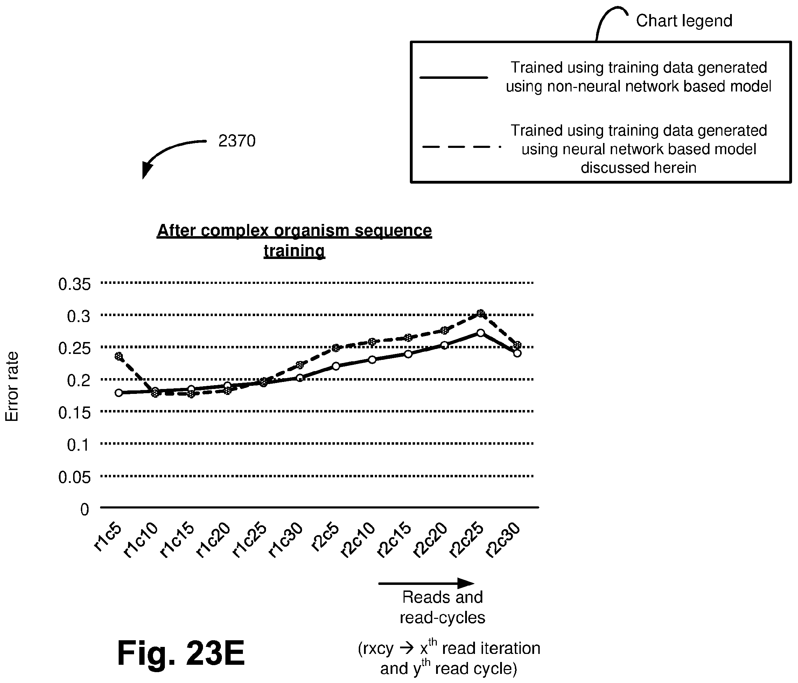

Thus, the method 2300 is continued until the base caller 1414 is "adequately trained." "Adequately trained" may imply that the base caller 1414 can now make base calls with error rates that are less than a target error rate. As discussed, the training process can be continued iteratively, until the adequate training and target error rate of base calling are achieved (*e.g.*, see "error rate" chart of Fig. 23E). At the end of method 2300, the base caller 1414 comprising the last NN configuration of the method 2300 is now adequately trained. Accordingly, the trained base caller 1414 comprising the last NN configuration of the method 2300 can now be used for inference, *e.g.*, used to sequence unknown genetic sequences.

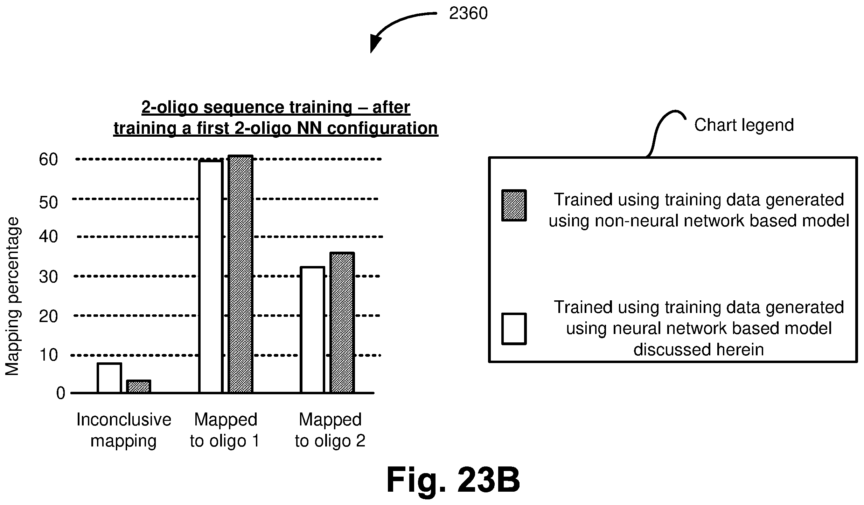

Figs. 23B-23E illustrate various charts illustrating effectiveness of the base caller training process discussed in this disclosure. Referring to Fig. 23B, illustrated is a chart 2360 that depicts mapping percentage of training data generated by (i) a first 2-oligo NN configuration, such as the NN configuration 1615, trained using the neural network-based training data generation techniques discussed herein, and (ii) a NN configuration trained using conventional 2-oligo training data generation techniques. The white bars in the chart 2360 illustrate mapping data from the first 2-oligo NN configuration that is trained using training data, which is generated using the neural network-based models discussed herein. Thus, the white bars in the chart 2360 illustrates mapping data generated using the various techniques discussed herein. The grey bars in the chart 2360 illustrate data associated with a NN configuration that is trained by training data generated by conventional non-neural network-based models, such as Real Time Analysis (RTA) models. Example of the RTA models are discussed in U.S. Patent No. US10304189B2, titled "Data processing system and methods," issued 28 May 2019. Thus, the grey bars in the chart 2360 illustrate mapping data generated using conventional techniques. In an example, the white bars of the chart 2360 can be generated at operations 1704b1 of the method 1700 of Fig. 17A. The chart 2360 illustrates percentage of base call predictions mapped to oligo 1, percentage of base call predictions mapped to oligo 2, and percentage of base call predictions that cannot be conclusively mapped to either of the oligos 1 or 2 (*i.e.*, inconclusive percentage). As seen, the inconclusive percentage for the training data generated using the techniques discussed herein is slightly higher than the inconclusive percentage for the training data generated using conventional techniques. Thus, initially (*e.g.*, at the beginning of the training iterations), the conventional techniques slightly outperform the training data generation techniques discussed herein.

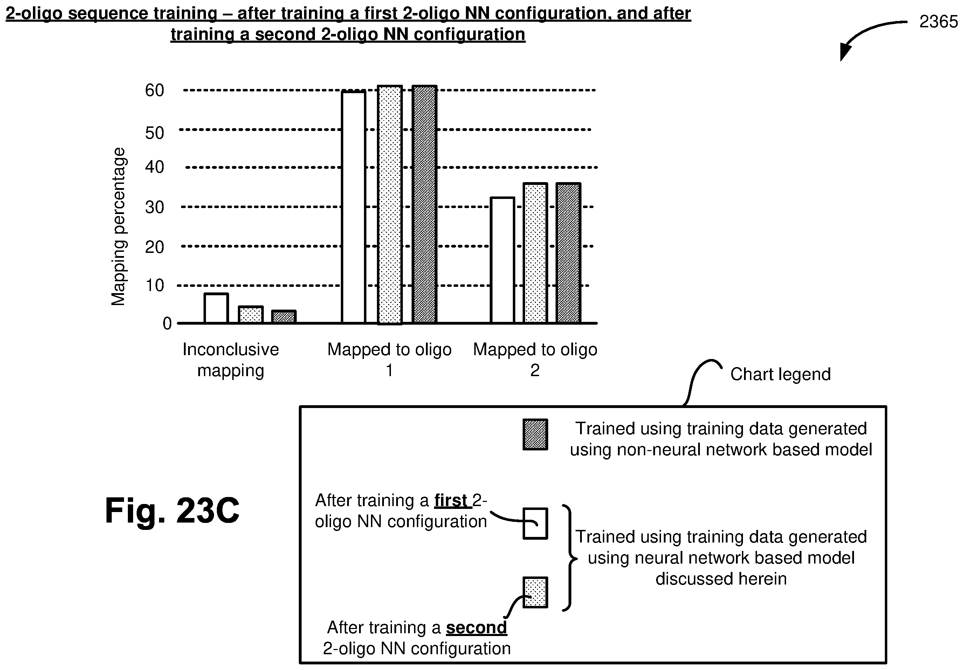

Referring now to Fig. 23C, illustrated is a chart 2365 that depicts mapping percentage in training data generated using (i) a first 2-oligo NN configuration (such as the NN configuration 1615) that is trained using the neural network based training data generation techniques discussed herein (white bars), (ii) a second 2-oligo NN configuration that is trained using the neural network based training data generation techniques discussed herein (dotted bars), and (iii) a NN configuration trained using conventional 2-oliogo training data generation techniques, such as RTA based conventional training data generation techniques (grey bars). In an example, the first 2-oligo NN configuration (white bars) and the second 2-oligo NN configuration (dotted bars) respectively correspond to operations 1704b and 1704c of method 1700 of Fig. 17A. The chart 2365 illustrates percentage of base call predictions mapped to oligo 1, percentage of base call predictions mapped to oligo 2, and percentage of base call predictions that cannot be conclusively mapped to either of the oligos 1 or 2 (*i.e.*, inconclusive percentage). As seen, the inconclusive percentage for the training data generated using first 2-oligo NN configuration is higher than each of (i) the training data generated using second 2-oligo NN configuration and (ii) the training data generated using the conventional techniques. Furthermore, the inconclusive percentage for the training data generated using the second 2-oligo NN configuration is almost comparable to the training data generated using the conventional techniques. Thus, with iterations and more complex NN configurations, the training data generated using the NN-based configurations are almost comparable to the training data generated using the conventional techniques.

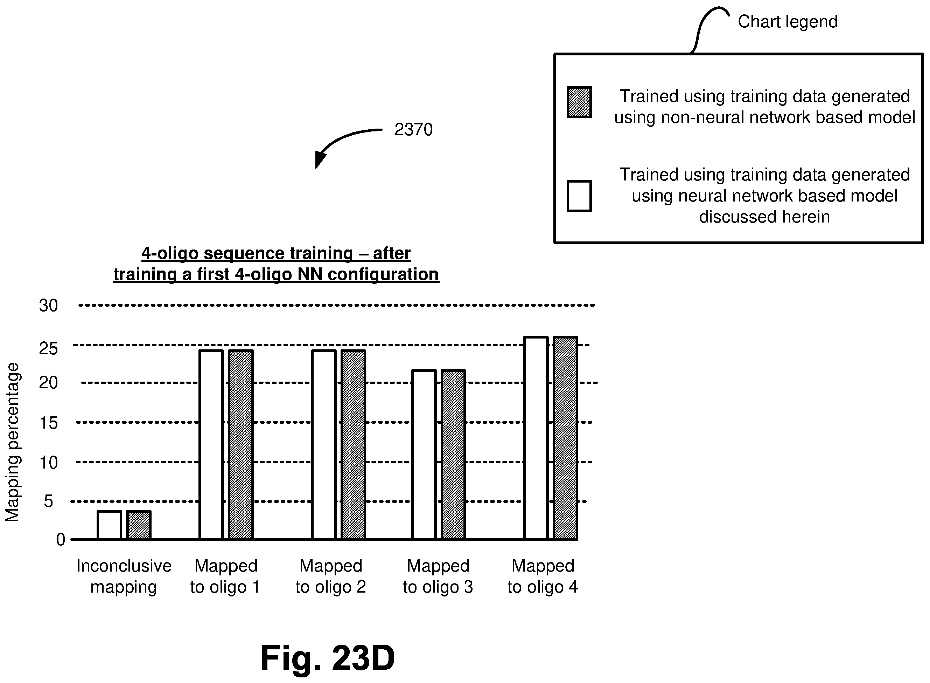

Referring now to Fig. 23D, illustrated is a chart 2370 that depicts mapping percentage of training data generated by (i) a first 4-oligo NN configuration trained using the neural network-based training data generation techniques discussed herein (white bars), and (ii) a NN configuration trained using conventional 4-oligo training data generation techniques, *e.g.*, RTA based techniques (grey bars). As seen, the inconclusive percentage for the training data generated using the techniques discussed herein is comparable to the inconclusive percentage for the training data generated using conventional techniques. Thus, the conventional techniques and the training data generation techniques discussed herein generate comparable results, when the training transitions to the 4-oligo sequence.

Referring now to Fig. 23E, illustrated is a chart 2375 that depicts error rates in data generated by (i) a NN configuration trained using complex organism sequence discussed herein, *e.g.*, with respect to operations 2328 of method 2300 of Fig. 23A (solid line), and (ii) a NN configuration trained using conventional complex organism training data generation techniques, *e.g.*, RTA based techniques (dashed lines). As seen, the error rates for the data generated using the techniques discussed herein is comparable to the data generated using conventional techniques. Thus, the conventional techniques and the training data generation techniques discussed herein generate comparable results. As discussed, the training data generation techniques discussed herein may be used instead of the conventional techniques, when, for example, the conventional techniques are not available or ready for training data generation.

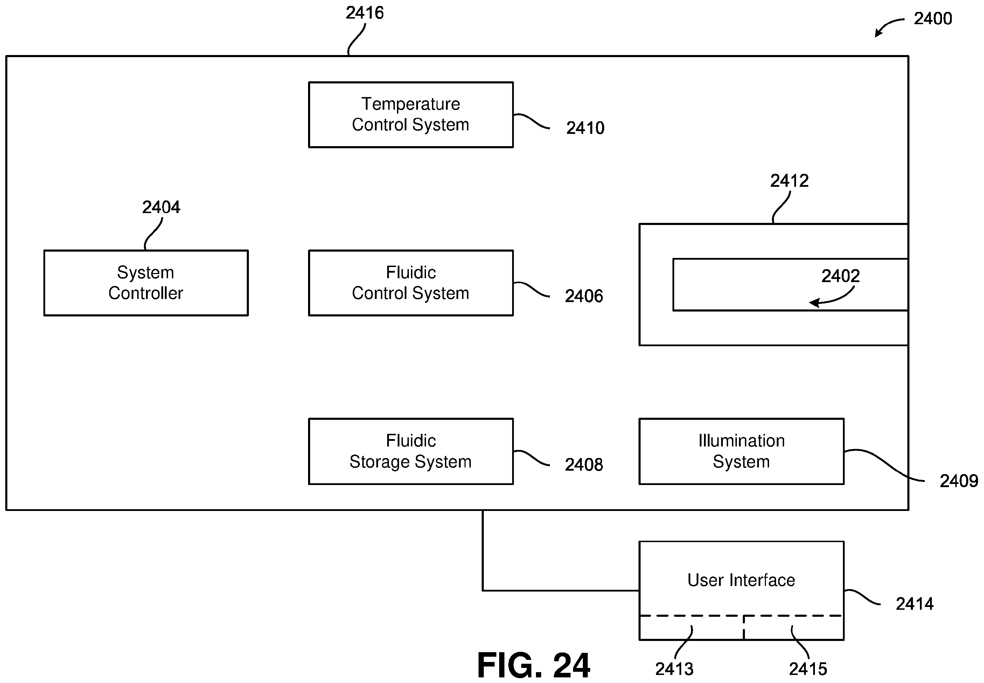

**Fig. 24** is a block diagram of a base calling system 2400 in accordance with one implementation. The base calling system 2400 may operate to obtain any information or data that relates to at least one of a biological or chemical substance. In some implementations, the base calling system 2400 is a workstation that may be similar to a bench-top device or desktop computer. For example, a majority (or all) of the systems and components for conducting the desired reactions can be within a common housing 2416.

In particular implementations, the base calling system 2400 is a nucleic acid sequencing system (or sequencer) configured for various applications, including but not limited to de novo sequencing, resequencing of whole genomes or target genomic regions, and metagenomics. The sequencer may also be used for DNA or RNA analysis. In some implementations, the base calling system 2400 may also be configured to generate reaction sites in a biosensor. For example, the base calling system 2400 may be configured to receive a sample and generate surface attached clusters of clonally amplified nucleic acids derived from the sample. Each cluster may constitute or be part of a reaction site in the biosensor.

The exemplary base calling system 2400 may include a system receptacle or interface 2412 that is configured to interact with a biosensor 2402 to perform desired reactions within the biosensor 2402. In the following description with respect to Fig. 24, the biosensor 2402 is loaded into the system receptacle 2412. However, it is understood that a cartridge that includes the biosensor 2402 may be inserted into the system receptacle 2412 and in some states the cartridge can be removed temporarily or permanently. As described above, the cartridge may include, among other things, fluidic control and fluidic storage components.

In particular implementations, the base calling system 2400 is configured to perform a large number of parallel reactions within the biosensor 2402. The biosensor 2402 includes one or more reaction sites where desired reactions can occur. The reaction sites may be, for example, immobilized to a solid surface of the biosensor or immobilized to beads (or other movable substrates) that are located within corresponding reaction chambers of the biosensor. The reaction sites can include, for example, clusters of clonally amplified nucleic acids. The biosensor 2402 may include a solid-state imaging device (*e.g.,* CCD or CMOS imager) and a flow cell mounted thereto. The flow cell may include one or more flow channels that receive a solution from the base calling system 2400 and direct the solution toward the reaction sites. Optionally, the biosensor 2402 can be configured to engage a thermal element for transferring thermal energy into or out of the flow channel.

The base calling system 2400 may include various components, assemblies, and systems (or sub-systems) that interact with each other to perform a predetermined method or assay protocol for biological or chemical analysis. For example, the base calling system 2400 includes a system controller 2404 that may communicate with the various components, assemblies, and sub-systems of the base calling system 2400 and also the biosensor 2402. For example, in addition to the system receptacle 2412, the base calling system 2400 may also include a fluidic control system 2406 to control the flow of fluid throughout a fluid network of the base calling system 2400 and the biosensor 2402; a fluidic storage system 2408 that is configured to hold all fluids (e.g., gas or liquids) that may be used by the bioassay system; a temperature control system 2410 that may regulate the temperature of the fluid in the fluid network, the fluidic storage system 2408, and/or the biosensor 2402; and an illumination system 2409 that is configured to illuminate the biosensor 2402. As described above, if a cartridge having the biosensor 2402 is loaded into the system receptacle 2412, the cartridge may also include fluidic control and fluidic storage components.

Also shown, the base calling system 2400 may include a user interface 2414 that interacts with the user. For example, the user interface 2414 may include a display 2413 to display or request information from a user and a user input device 2415 to receive user inputs. In some implementations, the display 2413 and the user input device 2415 are the same device. For example, the user interface 2414 may include a touch-sensitive display configured to detect the presence of an individual's touch and also identify a location of the touch on the display. However, other user input devices 2415 may be used, such as a mouse, touchpad, keyboard, keypad, handheld scanner, voice-recognition system, motion-recognition system, and the like. As will be discussed in greater detail below, the base calling system 2400 may communicate with various components, including the biosensor 2402 (e.g., in the form of a cartridge), to perform the desired reactions. The base calling system 2400 may also be configured to analyze data obtained from the biosensor to provide a user with desired information.

The system controller 2404 may include any processor-based or microprocessor-based system, including systems using microcontrollers, Reduced Instruction Set Computers (RISC), Application Specific Integrated Circuits (ASICs), Field Programmable Gate Array (FPGAs), logic circuits, and any other circuit or processor capable of executing functions described herein. The above examples are exemplary only, and are thus not intended to limit in any way the definition and/or meaning of the term system controller. In the exemplary implementation, the system controller 2404 executes a set of instructions that are stored in one or more storage elements, memories, or modules in order to at least one of obtain and analyze detection data. Detection data can include a plurality of sequences of pixel signals, such that a sequence of pixel signals from each of the millions of sensors (or pixels) can be detected over many base calling cycles. Storage elements may be in the form of information sources or physical memory elements within the base calling system 2400.

The set of instructions may include various commands that instruct the base calling system 2400 or biosensor 2402 to perform specific operations such as the methods and processes of the various implementations described herein. The set of instructions may be in the form of a software program, which may form part of a tangible, non-transitory computer readable medium or media. As used herein, the terms "software" and "firmware" are interchangeable and include any computer program stored in memory for execution by a computer, including RAM memory, ROM memory, EPROM memory, EEPROM memory, and non-volatile RAM (NVRAM) memory. The above memory types are exemplary only, and are thus not limiting as to the types of memory usable for storage of a computer program.

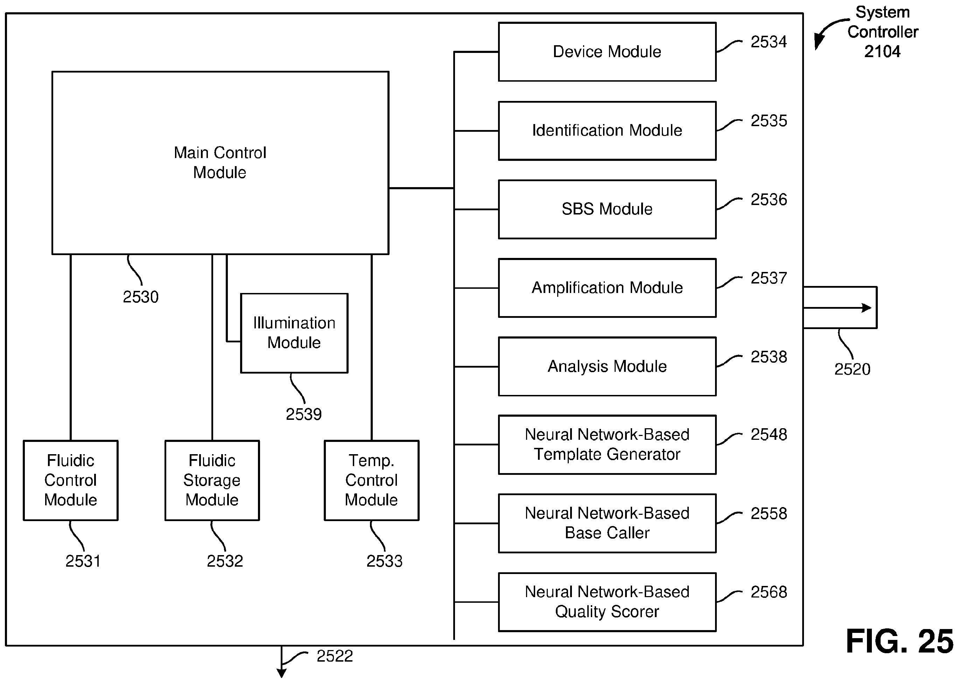

The software may be in various forms such as system software or application software. Further, the software may be in the form of a collection of separate programs, or a program module within a larger program or a portion of a program module. The software also may include modular programming in the form of object-oriented programming. After obtaining the detection data, the detection data may be automatically processed by the base calling system 2400, processed in response to user inputs, or processed in response to a request made by another processing machine (e.g., a remote request through a communication link). In the illustrated implementation, the system controller 2404 includes an analysis module 2538 (illustrated in Fig. 25). In other implementations, system controller 2404 does not include the analysis module 2538 and instead has access to the analysis module 2538 (e.g., the analysis module 2538 may be separately hosted on cloud).

The system controller 2404 may be connected to the biosensor 2402 and the other components of the base calling system 2400 via communication links. The system controller 2404 may also be communicatively connected to off-site systems or servers. The communication links may be hardwired, corded, or wireless. The system controller 2404 may receive user inputs or commands, from the user interface 2414 and the user input device 2415.

The fluidic control system 2406 includes a fluid network and is configured to direct and regulate the flow of one or more fluids through the fluid network. The fluid network may be in fluid communication with the biosensor 2402 and the fluidic storage system 2408. For example, select fluids may be drawn from the fluidic storage system 2408 and directed to the biosensor 2402 in a controlled manner, or the fluids may be drawn from the biosensor 2402 and directed toward, for example, a waste reservoir in the fluidic storage system 2408. Although not shown, the fluidic control system 2406 may include flow sensors that detect a flow rate or pressure of the fluids within the fluid network. The sensors may communicate with the system controller 2404.

The temperature control system 2410 is configured to regulate the temperature of fluids at different regions of the fluid network, the fluidic storage system 2408, and/or the biosensor 2402. For example, the temperature control system 2410 may include a thermocycler that interfaces with the biosensor 2402 and controls the temperature of the fluid that flows along the reaction sites in the biosensor 2402. The temperature control system 2410 may also regulate the temperature of solid elements or components of the base calling system 2400 or the biosensor 2402. Although not shown, the temperature control system 2410 may include sensors to detect the temperature of the fluid or other components. The sensors may communicate with the system controller 2404.

The fluidic storage system 2408 is in fluid communication with the biosensor 2402 and may store various reaction components or reactants that are used to conduct the desired reactions therein. The fluidic storage system 2408 may also store fluids for washing or cleaning the fluid network and biosensor 2402 and for diluting the reactants. For example, the fluid storage system 2408 may include various reservoirs to store samples, reagents, enzymes, other biomolecules, buffer solutions, aqueous, and nonpolar solutions, and the like. Furthermore, the fluidic storage system 2408 may also include waste reservoirs for receiving waste products from the biosensor 2402. In implementations that include a cartridge, the cartridge may include one or more of a fluid storage system, fluidic control system or temperature control system. Accordingly, one or more of the components set forth herein as relating to those systems can be contained within a cartridge housing. For example, a cartridge can have various reservoirs to store samples, reagents, enzymes, other biomolecules, buffer solutions, aqueous, and nonpolar solutions, waste, and the like. As such, one or more of a fluid storage system, fluidic control system or temperature control system can be removably engaged with a bioassay system via a cartridge or other biosensor.

The illumination system 2409 may include a light source (*e.g.*, one or more LEDs) and a plurality of optical components to illuminate the biosensor. Examples of light sources may include lasers, arc lamps, LEDs, or laser diodes. The optical components may be, for example, reflectors, dichroics, beam splitters, collimators, lenses, filters, wedges, prisms, mirrors, detectors, and the like. In implementations that use an illumination system, the illumination system 2409 may be configured to direct an excitation light to reaction sites. As one example, fluorophores may be excited by green wavelengths of light, as such the wavelength of the excitation light may be approximately 532 nm. In one implementation, the illumination system 2409 is configured to produce illumination that is parallel to a surface normal of a surface of the biosensor 2402. In another implementation, the illumination system 2409 is configured to produce illumination that is off-angle relative to the surface normal of the surface of the biosensor 2402. In yet another implementation, the illumination system 2409 is configured to produce illumination that has plural angles, including some parallel illumination and some off-angle illumination.

The system receptacle or interface 2412 is configured to engage the biosensor 2402 in at least one of a mechanical, electrical, and fluidic manner. The system receptacle 2412 may hold the biosensor 2402 in a desired orientation to facilitate the flow of fluid through the biosensor 2402. The system receptacle 2412 may also include electrical contacts that are configured to engage the biosensor 2402 so that the base calling system 2400 may communicate with the biosensor 2402 and/or provide power to the biosensor 2402. Furthermore, the system receptacle 2412 may include fluidic ports (*e.g.*, nozzles) that are configured to engage the biosensor 2402. In some implementations, the biosensor 2402 is removably coupled to the system receptacle 2412 in a mechanical manner, in an electrical manner, and also in a fluidic manner.

In addition, the base calling system 2400 may communicate remotely with other systems or networks or with other bioassay systems 2400. Detection data obtained by the bioassay system(s) 2400 may be stored in a remote database.

**Fig. 25** is a block diagram of the system controller 2404 that can be used in the system of Fig. 24. In one implementation, the system controller 2404 includes one or more processors or modules that can communicate with one another. Each of the processors or modules may include an algorithm (*e.g.*, instructions stored on a tangible and/or non-transitory computer readable storage medium) or sub-algorithms to perform particular processes. The system controller 2404 is illustrated conceptually as a collection of modules, but may be implemented utilizing any combination of dedicated hardware boards, DSPs, processors, etc. Alternatively, the system controller 2404 may be implemented utilizing an off-the-shelf PC with a single processor or multiple processors, with the functional operations distributed between the processors. As a further option, the modules described below may be implemented utilizing a hybrid configuration in which certain modular functions are performed utilizing dedicated hardware, while the remaining modular functions are performed utilizing an off-the-shelf PC and the like. The modules also may be implemented as software modules within a processing unit.

During operation, a communication port 2520 may transmit information (*e.g.*, commands) to or receive information (*e.g.*, data) from the biosensor 2402 (Fig. 24) and/or the sub-systems 2406, 2408, 2410 (Fig. 24). In implementations, the communication port 2520 may output a plurality of sequences of pixel signals. A communication port 2520 may receive user input from the user interface 2414 (Fig. 24) and transmit data or information to the user interface 2414. Data from the biosensor 2402 or sub-systems 2406, 2408, 2410 may be processed by the system controller 2404 in real-time during a bioassay session. Additionally, or alternatively, data may be stored temporarily in a system memory during a bioassay session and processed in slower than real-time or off-line operation.

As shown in Fig. 25, the system controller 2404 may include a plurality of modules 2531-2539 that communicate with a main control module 2530. The main control module 2530 may communicate with the user interface 2414 (Fig. 24). Although the modules 2531-2539 are shown as communicating directly with the main control module 2530, the modules 2531-2539 may also communicate directly with each other, the user interface 2414, and the biosensor 2402. Also, the modules 2531-2539 may communicate with the main control module 2530 through the other modules.

The plurality of modules 2531-2539 include system modules 2531-2533, 2539 that communicate with the sub-systems 2406, 2408, 2410, and 2409, respectively. The fluidic control module 2531 may communicate with the fluidic control system 2406 to control the valves and flow sensors of the fluid network for controlling the flow of one or more fluids through the fluid network. The fluidic storage module 2532 may notify the user when fluids are low or when the waste reservoir is at or near capacity. The fluidic storage module 2532 may also communicate with the temperature control module 2533 so that the fluids may be stored at a desired temperature. The illumination module 2539 may communicate with the illumination system 2409 to illuminate the reaction sites at designated times during a protocol, such as after the desired reactions (*e.g.*, binding events) have occurred. In some implementations, the illumination module 2539 may communicate with the illumination system 2409 to illuminate the reaction sites at designated angles.

The plurality of modules 2531-2539 may also include a device module 2534 that communicates with the biosensor 2402 and an identification module 2535 that determines identification information relating to the biosensor 2402. The device module 2534 may, for example, communicate with the system receptacle 2412 to confirm that the biosensor has established an electrical and fluidic connection with the base calling system 2400. The identification module 2535 may receive signals that identify the biosensor 2402. The identification module 2535 may use the identity of the biosensor 2402 to provide other information to the user. For example, the identification module 2535 may determine and then display a lot number, a date of manufacture, or a protocol that is recommended to be run with the biosensor 2402.

The plurality of modules 2531-2539 also includes an analysis module 2538 (also called signal processing module or signal processor) that receives and analyzes the signal data (*e.g.*, image data) from the biosensor 2402. Analysis module 2538 includes memory (*e.g.*, RAM or Flash) to store detection data. Detection data can include a plurality of sequences of pixel signals, such that a sequence of pixel signals from each of the millions of sensors (or pixels) can be detected over many base calling cycles. The signal data may be stored for subsequent analysis or may be transmitted to the user interface 2414 to display desired information to the user. In some implementations, the signal data may be processed by the solid-state imager (*e.g.*, CMOS image sensor) before the analysis module 2538 receives the signal data.

The analysis module 2538 is configured to obtain image data from the light detectors at each of a plurality of sequencing cycles. The image data is derived from the emission signals detected by the light detectors and process the image data for each of the plurality of sequencing cycles through a neural network (*e.g.*, a neural network-based template generator 2548, a neural network-based base caller 2558 (*e.g.*, see Figs. 7, 9, and 10), and/or a neural network-based quality scorer 2568) and produce a base call for at least some of the analytes at each of the plurality of sequencing cycle.

Protocol modules 2536 and 2537 communicate with the main control module 2530 to control the operation of the sub-systems 2406, 2408, and 2410 when conducting predetermined assay protocols. The protocol modules 2536 and 2537 may include sets of instructions for instructing the base calling system 2400 to perform specific operations pursuant to predetermined protocols. As shown, the protocol module may be a sequencing-by-synthesis (SBS) module 2536 that is configured to issue various commands for performing sequencing-by-synthesis processes. In SBS, extension of a nucleic acid primer along a nucleic acid template is monitored to determine the sequence of nucleotides in the template. The underlying chemical process can be polymerization (*e.g.*, as catalyzed by a polymerase enzyme) or ligation (*e.g.*, catalyzed by a ligase enzyme). In a particular polymerase-based SBS implementation, fluorescently labeled nucleotides are added to a primer (thereby extending the primer) in a template dependent fashion such that detection of the order and type of nucleotides added to the primer can be used to determine the sequence of the template. For example, to initiate a first SBS cycle, commands can be given to deliver one or more labeled nucleotides, DNA polymerase, etc., into/through a flow cell that houses an array of nucleic acid templates. The nucleic acid templates may be located at corresponding reaction sites. Those reaction sites where primer extension causes a labeled nucleotide to be incorporated can be detected through an imaging event. During an imaging event, the illumination system 2409 may provide an excitation light to the reaction sites. Optionally, the nucleotides can further include a reversible termination property that terminates further primer extension once a nucleotide has been added to a primer. For example, a nucleotide analog having a reversible terminator moiety can be added to a primer such that subsequent extension cannot occur until a deblocking agent is delivered to remove the moiety. Thus, for implementations that use reversible termination a command can be given to deliver a deblocking reagent to the flow cell (before or after detection occurs). One or more commands can be given to effect wash(es) between the various delivery steps. The cycle can then be repeated n times to extend the primer by n nucleotides, thereby detecting a sequence of length n. Exemplary sequencing techniques are described, for example, in Bentley et al., Nature 456:53-59 (2008); WO 04/018497; US 7,057,026; WO 91/06678; WO 07/123744; US 7,329,492; US 7,211,414; US 7,315,019; and US 7,405,281.

For the nucleotide delivery step of an SBS cycle, either a single type of nucleotide can be delivered at a time, or multiple different nucleotide types (*e.g.*, A, C, T and G together) can be delivered. For a nucleotide delivery configuration where only a single type of nucleotide is present at a time, the different nucleotides need not have distinct labels since they can be distinguished based on temporal separation inherent in the individualized delivery. Accordingly, a sequencing method or apparatus can use single color detection. For example, an excitation source need only provide excitation at a single wavelength or in a single range of wavelengths. For a nucleotide delivery configuration where delivery results in multiple different nucleotides being present in the flow cell at one time, sites that incorporate different nucleotide types can be distinguished based on different fluorescent labels that are attached to respective nucleotide types in the mixture. For example, four different nucleotides can be used, each having one of four different fluorophores. In one implementation, the four different fluorophores can be distinguished using excitation in four different regions of the spectrum. For example, four different excitation radiation sources can be used. Alternatively, fewer than four different excitation sources can be used, but optical filtration of the excitation radiation from a single source can be used to produce different ranges of excitation radiation at the flow cell.

In some implementations, fewer than four different colors can be detected in a mixture having four different nucleotides. For example, pairs of nucleotides can be detected at the same wavelength, but distinguished based on a difference in intensity for one member of the pair compared to the other, or based on a change to one member of the pair (*e.g.*, via chemical modification, photochemical modification or physical modification) that causes apparent signal to appear or disappear compared to the signal detected for the other member of the pair. Exemplary apparatus and methods for distinguishing four different nucleotides using detection of fewer than four colors are described for example in US Pat. App. Ser. Nos. 61/538,294 and 61/619,878. U.S. Application No. 13/624,200, which was filed on September 21, 2012.

The plurality of protocol modules may also include a sample-preparation (or generation) module 2537 that is configured to issue commands to the fluidic control system 2406 and the temperature control system 2410 for amplifying a product within the biosensor 2402. For example, the biosensor 2402 may be engaged to the base calling system 2400. The amplification module 2537 may issue instructions to the fluidic control system 2406 to deliver necessary amplification components to reaction chambers within the biosensor 2402. In other implementations, the reaction sites may already contain some components for amplification, such as the template DNA and/or primers. After delivering the amplification components to the reaction chambers, the amplification module 2537 may instruct the temperature control system 2410 to cycle through different temperature stages according to known amplification protocols. In some implementations, the amplification and/or nucleotide incorporation is performed isothermally.

The SBS module 2536 may issue commands to perform bridge PCR where clusters of clonal amplicons are formed on localized areas within a channel of a flow cell. After generating the amplicons through bridge PCR, the amplicons may be "linearized" to make single stranded template DNA, or sstDNA, and a sequencing primer may be hybridized to a universal sequence that flanks a region of interest. For example, a reversible terminator-based sequencing by synthesis method can be used as set forth above or as follows.

Each base calling or sequencing cycle can extend an sstDNA by a single base which can be accomplished for example by using a modified DNA polymerase and a mixture of four types of nucleotides. The different types of nucleotides can have unique fluorescent labels, and each nucleotide can further have a reversible terminator that allows only a single-base incorporation to occur in each cycle. After a single base is added to the sstDNA, excitation light may be incident upon the reaction sites and fluorescent emissions may be detected. After detection, the fluorescent label and the terminator may be chemically cleaved from the sstDNA. Another similar base calling or sequencing cycle may follow. In such a sequencing protocol, the SBS module 2536 may instruct the fluidic control system 2406 to direct a flow of reagent and enzyme solutions through the biosensor 2402. Exemplary reversible terminator-based SBS methods which can be utilized with the apparatus and methods set forth herein are described in US Patent Application Publication No. 2007/0166705 A1, US Patent Application Publication No. 2006/0188901 A1, US Patent No. 7,057,026, US Patent Application Publication No. 2006/0240439 A1, US Patent Application Publication No. 2006/02814714709 A1, PCT Publication No. WO 05/065814, PCT Publication No. WO 06/064199. Exemplary reagents for reversible terminator-based SBS are described in US 7,541,444; US 7,057,026; US 7,427,673; US 7,566,537; and US 7,592,435.

In some implementations, the amplification and SBS modules may operate in a single assay protocol where, for example, template nucleic acid is amplified and subsequently sequenced within the same cartridge.

The base calling system 2400 may also allow the user to reconfigure an assay protocol. For example, the base calling system 2400 may offer options to the user through the user interface 2414 for modifying the determined protocol. For example, if it is determined that the biosensor 2402 is to be used for amplification, the base calling system 2400 may request a temperature for the annealing cycle. Furthermore, the base calling system 2400 may issue warnings to a user if a user has provided user inputs that are generally not acceptable for the selected assay protocol.

In implementations, the biosensor 2402 includes millions of sensors (or pixels), each of which generates a plurality of sequences of pixel signals over successive base calling cycles. The analysis module 2538 detects the plurality of sequences of pixel signals and attributes them to corresponding sensors (or pixels) in accordance to the row-wise and/or column-wise location of the sensors on an array of sensors.

Each sensor in the array of sensors can produce sensor data for a tile of the flow cell, where a tile in an area on the flow cell at which clusters of genetic material are disposed during the based calling operation. The sensor data can comprise image data in an array of pixels. For a given cycle, the sensor data can include more than one image, producing multiple features per pixel as the tile data.

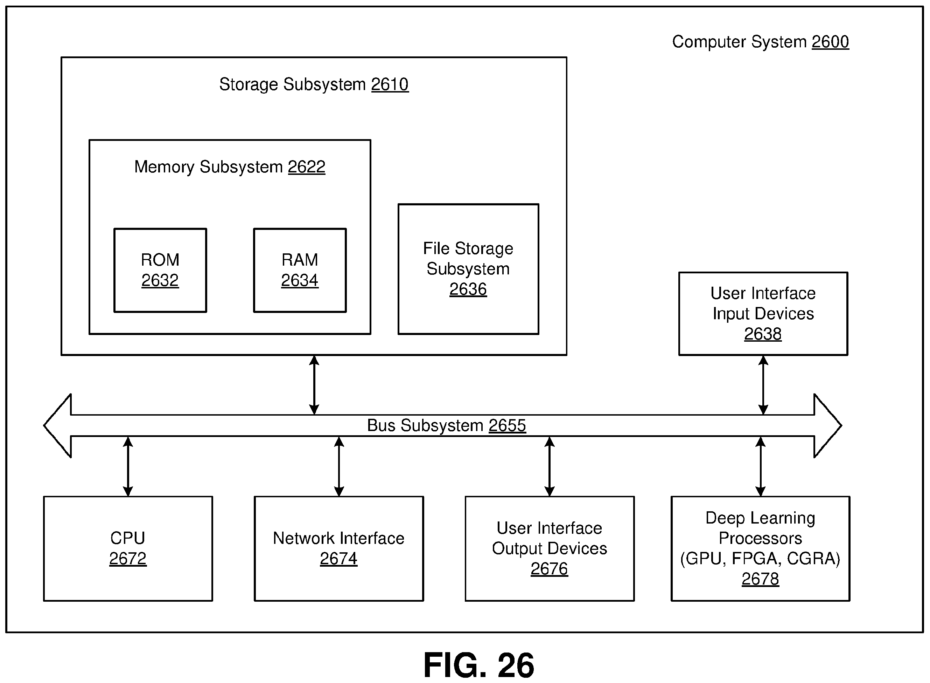

**Fig. 26** is a simplified block diagram of a computer 2600 system that can be used to implement the technology disclosed. Computer system 2600 includes at least one central processing unit (CPU) 2672 that communicates with a number of peripheral devices via bus subsystem 2655. These peripheral devices can include a storage subsystem 2610 including, for example, memory devices and a file storage subsystem 2636, user interface input devices 2638, user interface output devices 2676, and a network interface subsystem 2674. The input and output devices allow user interaction with computer system 2600. Network interface subsystem 2674 provides an interface to outside networks, including an interface to corresponding interface devices in other computer systems.

User interface input devices 2638 can include a keyboard; pointing devices such as a mouse, trackball, touchpad, or graphics tablet; a scanner; a touch screen incorporated into the display; audio input devices such as voice recognition systems and microphones; and other types of input devices. In general, use of the term "input device" is intended to include all possible types of devices and ways to input information into computer system 2600.

User interface output devices 2676 can include a display subsystem, a printer, a fax machine, or non-visual displays such as audio output devices. The display subsystem can include an LED display, a cathode ray tube (CRT), a flat-panel device such as a liquid crystal display (LCD), a projection device, or some other mechanism for creating a visible image. The display subsystem can also provide a non-visual display such as audio output devices. In general, use of the term "output device" is intended to include all possible types of devices and ways to output information from computer system 2600 to the user or to another machine or computer system.

Storage subsystem 2610 stores programming and data constructs that provide the functionality of some or all of the modules and methods described herein. These software modules are generally executed by deep learning processors 2678.

In one implementation, the neural networks are implemented using deep learning processors 2678 can be configurable and reconfigurable processors, field-programmable gate arrays (FPGAs), application-specific integrated circuits (ASICs), and/or coarse-grained reconfigurable architectures (CGRAs) and graphics processing units (GPUs) other configured devices. Deep learning processors 2678 can be hosted by a deep learning cloud platform such as Google Cloud Platform™, Xilinx™, and Cirrascale™. Examples of deep learning processors 14978 include Google's Tensor Processing Unit (TPU)™, rackmount solutions like GX4 Rackmount Series™, GX149 Rackmount Series™, NVIDIA DGX-1™, Microsoft' Stratix V FPGA™, Graphcore's Intelligent Processor Unit (IPU)™, Qualcomm's Zeroth Platform™ with Snapdragon processors™, NVIDIA's Volta™, NVIDIA's DRIVE PX™, NVIDIA's JETSON TX1/TX2 MODULE™, Intel's Nirvana™, Movidius VPU™, Fujitsu DPI™, ARM's DynamicIQ™, IBM TrueNorth™, and others.

Memory subsystem 2622 used in the storage subsystem 2610 can include a number of memories including a main random access memory (RAM) 2634 for storage of instructions and data during program execution and a read only memory (ROM) 2632 in which fixed instructions are stored. A file storage subsystem 2636 can provide persistent storage for program and data files, and can include a hard disk drive, a floppy disk drive along with associated removable media, a CD-ROM drive, an optical drive, or removable media cartridges. The modules implementing the functionality of certain implementations can be stored by file storage subsystem 2636 in the storage subsystem 2610, or in other machines accessible by the processor.

Bus subsystem 2655 provides a mechanism for letting the various components and subsystems of computer system 2600 communicate with each other as intended. Although bus subsystem 2655 is shown schematically as a single bus, alternative implementations of the bus subsystem can use multiple busses.

Computer system 2600 itself can be of varying types including a personal computer, a portable computer, a workstation, a computer terminal, a network computer, a television, a mainframe, a server farm, a widely-distributed set of loosely networked computers, or any other data processing system or user device. Due to the ever-changing nature of computers and networks, the description of computer system 2600 depicted in Fig. 26 is intended only as a specific example for purposes of illustrating the preferred implementations of the present invention. Many other configurations of computer system 2600 are possible having more or less components than the computer system depicted in Fig. 26.

Aspects of the disclosure are defined by the appended claims.

**Claims**

1. A computer-implemented method of progressively training a base caller, including:

iteratively initially training a base caller with analyte of a single-oligo base sequence with a single known oligo base sequence, and generating labelled training data using the initially trained base caller, the base caller having an initial neural network configuration comprising a number of layers and parameters;

(i) further training the base caller with analyte comprising multi-oligo base sequences comprising at least two known oligo base sequences that differ from one another by a threshold edit distance between respective nucleotide bases thereof, and generating labelled training data using the further trained base caller; and

iteratively further training the base caller by repeating step (i), while, during at least one iteration, increasing a complexity of the initial neural network configuration of the base caller by increasing the number of layers and parameters relative to the initial neural network configuration to adjust the initial neural network configuration for the multi-oligo base sequences, wherein labelled training data generated during an iteration is used to train the base caller during an immediate subsequent iteration.

2. The computer-implemented method of claim 1, further comprising:
during at least one iteration of further training the base caller with the analyte comprising multi-oligo base sequences:

increasing, within the analyte, a number of unique oligo base sequences of the multi-oligo base sequences; and further increasing the number of layers and parameters to further adjust the initial neural network configuration for the increased number of unique oligo base sequences.

3. The computer-implemented method of claim 1 or 2, wherein iteratively initially training the base caller with the analyte comprising the single-oligo base sequence comprises:
during a first iteration of iteratively initially training the base caller:

populating the single-oligo base sequence into a plurality of clusters of a flow cell;
generating a plurality of sequence signals corresponding to the plurality of clusters, each sequence signal of the plurality of sequence signals representative of base sequences loaded in a corresponding cluster of the plurality of clusters;
predicting, based on each sequence signal of the plurality of sequence signals, corresponding base calls for the single known oligo base sequence, to thereby generate a plurality of predicted base calls;
generating, for each sequence signal of the plurality of sequence signals, a corresponding error signal, based on comparing (i) corresponding predicted base calls and (ii) bases of the single known oligo base sequence, thereby generating a plurality of error signals corresponding to the plurality of sequence signals; and
initially training the base caller during the first iteration, based on the plurality of error signals.

4. The computer-implemented method of claim 3, wherein iteratively initially training the base caller with the analyte comprising the single-oligo base sequence further comprises:
during a second iteration of iteratively initially training the base caller that occurs after the first iteration of iteratively initially training the base caller:

using the base caller that has been partially trained during the first iteration, predicting, based on each sequence signal of the plurality of sequence signals, corresponding further base calls for the single known oligo base sequence, to thereby generate a plurality of further predicted base calls;
generating, for each sequence signal of the plurality of sequence signals, a corresponding further error signal, based on comparing (i) corresponding further predicted base calls and (ii) bases of the single known oligo base sequence, thereby generating a plurality of further error signals corresponding to the plurality of sequence signals; and
further initially training the base caller during the second iteration, based on the plurality of further error signals.

5. The computer-implemented method of claim 4, wherein:
the plurality of sequence signals corresponding to the plurality of clusters, which are generated during the first iteration of iteratively initially training the base caller, is reused for the second iteration of iteratively initially training the base caller.

6. The computer-implemented method of any of claims 4-5, wherein comparing (i) corresponding predicted base calls and (ii) bases of the single known oligo base sequence comprises:
for a first predicted base calls, (i) comparing a first base of the first predicted base calls with a first base of the single known oligo base sequence and (ii) comparing a second base of the first predicted base calls and a second base of the single known oligo base sequence, to generate a corresponding first error signal.

7. The computer-implemented method of any of claims 1-6, wherein iteratively further training the base caller comprises:

further training the base caller for N1 iterations with analyte comprising two known unique oligo base sequences; and
further training the base caller for N2 iterations with analyte comprising three known unique oligo base sequences,
wherein the N1 iterations are performed prior to the N2 iterations.

8. The computer-implemented method of any of claims 1-7, wherein while iteratively initially training the base caller with the analyte comprising the single-oligo base sequence, a first neural network configuration is loaded within the base caller, and wherein iteratively further training the base caller comprises:
further training the base caller for N1 iterations with analyte comprising two known unique oligo base sequences, such that

(i) for a first subset of the N1 iterations, a second neural network configuration is loaded within the base caller, and
(ii) for a second subset of the N1 iterations occurring after the first subset of the N1 iterations, a third neural network configuration is loaded within the base caller, wherein the first, second, and third neural network configurations are different from each other.

9. The computer-implemented method of claim 8, wherein the second neural network configuration is more complex than the first neural network configuration, and wherein the third neural network configuration is more complex than the second neural network configuration.

10. The computer-implemented method of claim 8 or 9, wherein the second neural network configuration has a greater number of layers, weights, or parameters than the first neural network configuration.

11. The computer-implemented method of any of claims 8-10, wherein the third neural network configuration has a greater number of layers, weights, or parameters than the second neural network configuration.

12. The computer-implemented method of any of claims 8-11, wherein further training the base caller for the N1 iterations with the analyte comprising two known unique oligo base sequences comprises, for one iteration of the N1 iterations:

populating (i) a first plurality of clusters of a flow cell with a first known oligo base sequence of the two known unique oligo base sequences and (ii) a second plurality of clusters of the flow cell with a second known oligo base sequence of the two known unique oligo base sequences;
predicting, for each cluster of the first and second pluralities of clusters, corresponding base calls, such that a plurality of predicted base calls are generated;
mapping (i) a first predicted base call of the plurality of predicted base calls to the first known oligo base sequence and (ii) a second predicted base call of the plurality of predicted base calls to the second known oligo base sequence, while refraining from mapping a third predicted base call of the plurality of predicted base calls to any of the first or second known oligo base sequences;
generating (i) a first error signal, based on comparing the first predicted base call to the first known oligo base sequence, and (ii) a second error signal, based on comparing the second predicted base call to the second known oligo base sequence; and
further training the base caller, based on the first and second error signals.

13. The computer-implemented method of claim 12, wherein mapping the first predicted base call to the first known oligo base sequence of the two known unique oligo base sequences comprises:

comparing each base of the first predicted base call to corresponding bases of the first and second known oligo base sequences;
determining that the first predicted base call has at least a threshold number of similarity of bases with the first known oligo base sequence, and has less than the threshold number of similarity of bases with the second known oligo base sequence; and
based on determining that the first predicted base call has at least the threshold number of similarity of bases with

## EP 4 364 155 B1

the first known oligo base sequence, mapping the first predicted base call to the first known oligo base sequence.

14. A system including one or more processors coupled to memory, the memory loaded with computer instructions that, when executed on the one or more processors, cause the system to perform a method according to any one of claims 1-13.

15. A non-transitory computer readable storage medium impressed with computer program instructions that, when executed on one or more processors, cause the one or more processors to perform a method according to any one of claims 1-13.

**Patentansprüche**

1. Computerimplementiertes Verfahren des schrittweisen Trainierens eines Base Callers, das einschließt:

iteratives anfängliches Trainieren eines Base Callers mit einem Analyten einer einzelnen Oligo-Basensequenz mit einer einzelnen bekannten Oligo-Basensequenz und Erzeugen markierter Trainingsdaten unter Verwendung des anfänglich trainierten Base Callers, wobei der Base Caller eine anfängliche neuronale Netzwerkkonfiguration aufweist, umfassend eine Anzahl von Schichten und Parametern;

(i) weiteres Trainieren des Base Callers mit einem Analyten, umfassend Multi-Oligo-Basensequenzen, umfassend mindestens zwei bekannte Oligo-Basensequenzen, die sich voneinander durch eine Schwellenwert-Edit-Distanz zwischen den jeweiligen Nukleotidbasen unterscheiden, und Erzeugen markierter Trainingsdaten unter Verwendung des weiter trainierten Base Callers; und

iteratives weiteres Trainieren des Base Callers durch Wiederholen von Schritt (i), derweil, während mindestens einer Iteration, eine Komplexität der anfänglichen neuronalen Netzwerkkonfiguration des Base Callers durch Erhöhen der Anzahl von Schichten und Parametern in Bezug auf die anfängliche neuronale Netzwerkkonfiguration erhöht wird, um die anfängliche neuronale Netzwerkkonfiguration an die Multi-Oligo-Basensequenzen anzupassen, wobei markierte Trainingsdaten, die während einer Iteration erzeugt wurden, verwendet werden, um den Base Caller während einer unmittelbar nachfolgenden Iteration zu trainieren.

2. Computerimplementiertes Verfahren nach Anspruch 1, ferner umfassend:
während mindestens einer Iteration des weiteren Trainierens des Base Callers mit dem Analyten, umfassend Multi-Oligo-Basensequenzen:

Erhöhen, innerhalb des Analyten, einer Anzahl einzigartiger Oligo-Basensequenzen der Multi-Oligo-Basensequenzen; und
weiteres Erhöhen der Anzahl von Schichten und Parametern, um die anfängliche neuronale Netzwerkkonfiguration weiter an die erhöhte Anzahl einzigartiger Oligo-Basensequenzen anzupassen.

3. Computerimplementiertes Verfahren nach Anspruch 1 oder 2, wobei das iterative anfängliche Trainieren des Base Callers mit dem Analyten, umfassend die einzelne Oligo-Basensequenz, umfasst:
während einer ersten Iteration des iterativen anfänglichen Trainierens des Base Callers:

Auffüllen der einzelnen Oligo-Basensequenz in eine Vielzahl von Clustern einer Durchflusszelle;
Erzeugen einer Vielzahl von Sequenzsignalen, die der Vielzahl von Clustern entsprechen, wobei jedes Sequenzsignal der Vielzahl von Sequenzsignalen für Basensequenzen repräsentativ ist, die in einen entsprechenden Cluster der Vielzahl von Clustern geladen sind;
Vorhersagen, basierend auf jedem Sequenzsignal der Vielzahl von Sequenzsignalen, entsprechender Base Calls für die einzelne bekannte Oligo-Basensequenz, um dadurch eine Vielzahl vorhergesagter Base Calls zu erzeugen;
Erzeugen, für jedes Sequenzsignal der Vielzahl von Sequenzsignalen, eines entsprechenden Fehlersignals, basierend auf dem Vergleichen (i) entsprechender vorhergesagter Base Calls und (ii) Basen der einzelnen bekannten Oligo-Basensequenz, wodurch eine Vielzahl von Fehlersignalen erzeugt wird, die der Vielzahl von Sequenzsignalen entsprechen; und
anfängliches Trainieren des Base Callers während der ersten Iteration, basierend auf der Vielzahl von Fehlersignalen.

43

**4.** Computerimplementiertes Verfahren nach Anspruch 3, wobei das iterative anfängliche Trainieren des Base Callers mit dem Analyten, umfassend die einzelne Oligo-Basensequenz, ferner umfasst:
während einer zweiten Iteration des iterativen anfänglichen Trainierens des Base Callers, die nach der ersten Iteration des iterativen anfänglichen Trainierens des Base Callers erfolgt:

unter Verwendung des Base Callers, der während der ersten Iteration teilweise trainiert wurde, Vorhersagen, basierend auf jedem Sequenzsignal der Vielzahl von Sequenzsignalen, entsprechender weiterer Base Calls für die einzelne bekannte Oligo-Basensequenz, um dadurch eine Vielzahl weiterer vorhergesagter Base Calls zu erzeugen;
Erzeugen, für jedes Sequenzsignal der Vielzahl von Sequenzsignalen, eines entsprechenden weiteren Fehlersignals, basierend auf dem Vergleichen (i) entsprechender weiterer vorhergesagter Base Calls und (ii) Basen der einzelnen bekannten Oligo-Basensequenz, wodurch eine Vielzahl weiterer Fehlersignale erzeugt wird, die der Vielzahl von Sequenzsignalen entsprechen; und
weiteres anfängliches Trainieren des Base Callers während der zweiten Iteration, basierend auf der Vielzahl von Fehlersignalen.

**5.** Computerimplementiertes Verfahren nach Anspruch 4, wobei:
die Vielzahl von Sequenzsignalen, die der Vielzahl von Clustern entsprechen, die während der ersten Iteration des iterativen anfänglichen Trainierens des Base Callers erzeugt werden, für die zweite Iteration des iterativen anfänglichen Trainierens des Base Callers wiederverwendet werden.

**6.** Computerimplementiertes Verfahren nach einem der Ansprüche 4 bis 5, wobei das Vergleichen (i) entsprechender vorhergesagter Base Calls und (ii) Basen der einzelnen bekannten Oligo-Basensequenz umfasst:
für erste vorhergesagte Base Calls (i) Vergleichen einer ersten Base der ersten vorhergesagten Base Calls mit einer ersten Base der einzelnen bekannten Oligo-Basensequenz und (ii) Vergleichen einer zweiten Base der ersten vorhergesagten Base Calls und einer zweiten Base der einzelnen bekannten Oligo-Basensequenz, um ein entsprechendes erstes Fehlersignal zu erzeugen.

**7.** Computerimplementiertes Verfahren nach einem der Ansprüche 1 bis 6, wobei das iterative weitere Trainieren des Base Callers umfasst:

weiteres Trainieren des Base Callers für N1-Iterationen mit dem Analyten, umfassend zwei bekannte einzigartige Oligo-Basensequenzen; und
weiteres Trainieren des Base Callers für N2-Iterationen mit dem Analyten, umfassend drei bekannte einzigartige Oligo-Basensequenzen,
wobei die N1-Iterationen vor den N2-Iterationen durchgeführt werden.

**8.** Computerimplementiertes Verfahren nach einem der Ansprüche 1 bis 7, wobei während des iterativen anfänglichen Trainierens des Base Callers mit dem Analyten, umfassend die einzelne Oligo-Basensequenz, eine erste neuronale Netzwerkkonfiguration in den Base Caller geladen wird, und wobei das iterative weitere Trainieren des Base Callers umfasst:
weiteres Trainieren des Base Callers für N1-Iterationen mit dem Analyten, umfassend zwei bekannte einzigartige Oligo-Basensequenzen auf derartige Weise, dass

(i) für eine erste Teilmenge der N1-Iterationen eine zweite neuronale Netzwerkkonfiguration in den Base Caller geladen wird, und
(ii) für eine zweite Teilmenge der N1-Iterationen, die nach der ersten Teilmenge der N1-Iterationen auftritt, eine dritte neuronale Netzwerkkonfiguration in den Base Caller geladen wird, wobei sich die erste, zweite und dritte neuronale Netzwerkkonfiguration voneinander unterscheiden.

**9.** Computerimplementiertes Verfahren nach Anspruch 8, wobei die zweite neuronale Netzwerkkonfiguration komplexer ist als die erste neuronale Netzwerkkonfiguration, und wobei die dritte neuronale Netzwerkkonfiguration komplexer ist als die zweite neuronale Netzwerkkonfiguration.

**10.** Computerimplementiertes Verfahren nach Anspruch 8 oder 9, wobei die zweite neuronale Netzwerkkonfiguration eine größere Anzahl an Schichten, Gewichten oder Parametern aufweist als die erste neuronale Netzwerkkonfiguration.

**11.** Computerimplementiertes Verfahren nach einem der Ansprüche 8 bis 10, wobei die dritte neuronale Netzwerkkonfiguration eine größere Anzahl an Schichten, Gewichten oder Parametern aufweist als die zweite neuronale Netzwerkkonfiguration.

**12.** Computerimplementiertes Verfahren nach einem der Ansprüche 8 bis 11, wobei das weitere Trainieren des Base Callers für die N1-Iterationen mit dem Analyten, umfassend zwei bekannte einzigartige Oligo-Basensequenzen, für eine Iteration der N1-Iterationen umfasst:

Auffüllen (i) einer ersten Vielzahl von Clustern einer Durchflusszelle mit einer ersten bekannten Oligo-Basensequenz der zwei bekannten einzigartigen Oligo-Basensequenzen und (ii) einer zweiten Vielzahl von Clustern der Durchflusszelle mit einer zweiten bekannten Oligo-Basensequenz der zwei bekannten einzigartigen Oligo-Basensequenzen;

Vorhersagen, für jeden Cluster der ersten und zweiten Vielzahl von Clustern, entsprechender Base Calls auf derartige Weise, dass eine Vielzahl vorhergesagter Base Calls erzeugt wird;

Zuordnen (i) eines ersten vorhergesagten Base Calls aus der Vielzahl vorhergesagter Base Calls zur ersten bekannten Oligo-Basensequenz und (ii) eines zweiten vorhergesagten Base Calls aus der Vielzahl vorhergesagter Base Calls zur zweiten bekannten Oligo-Basensequenz, wohingegen auf das Zuordnen eines dritten vorhergesagten Base Calls aus der Vielzahl vorhergesagter Base Calls einer beliebigen der ersten oder zweiten bekannten Oligo-Basensequenz verzichtet wird;

Erzeugen (i) eines ersten Fehlersignals, basierend auf dem Vergleichen des ersten vorhergesagten Base Calls mit der ersten bekannten Oligo-Basensequenz, und (ii) eines zweiten Fehlersignals, basierend auf dem Vergleichen des zweiten vorhergesagten Base Calls mit der zweiten bekannten Oligo-Basensequenz; und weiteres Trainieren des Base Callers basierend auf dem ersten und zweiten Fehlersignal.

**13.** Computerimplementiertes Verfahren nach Anspruch 12, wobei das Zuordnen des ersten vorhergesagten Base Calls zur ersten bekannten Oligo-Basensequenz der zwei bekannten einzigartigen Oligo-Basensequenzen umfasst:

Vergleichen jeder Base des ersten vorhergesagten Base Calls mit entsprechenden Basen der ersten und zweiten bekannten Oligo-Basensequenz;

Bestimmen, dass der erste vorhergesagte Base Call mindestens eine Schwellenwertzahl der Ähnlichkeit an Basen mit der ersten bekannten Oligo-Basensequenz aufweist, und weniger als die Schwellenwertzahl der Ähnlichkeit an Basen mit der zweiten bekannten Oligo-Basensequenz aufweist; und

basierend auf dem Bestimmen, dass der erste vorhergesagte Base Call mindestens eine Schwellenwertzahl der Ähnlichkeit an Basen mit der ersten bekannten Oligo-Basensequenz aufweist, Zuordnen des ersten vorhergesagten Base Calls zur ersten bekannten Oligo-Basensequenz.

**14.** System einschließlich eines oder mehrerer Prozessoren, die mit einem Speicher gekoppelt sind, wobei der Speicher mit Computeranweisungen geladen ist, die, wenn sie auf dem einen oder den mehreren Prozessoren ausgeführt werden, das System veranlassen, ein Verfahren nach einem der Ansprüche 1 bis 13 durchzuführen.

**15.** Nicht-transitorisches computerlesbares Speichermedium, auf dem Computerprogrammanweisungen eingeprägt sind, die, wenn sie auf einem oder mehreren Prozessor ausgeführt werden, den einen oder die mehreren Prozessoren veranlassen, ein Verfahren nach einem der Ansprüche 1 bis 13 durchführen.

## Revendications

**1.** Procédé mis en œuvre par ordinateur d'entraînement progressif d'un appelant de base, comportant :

l'entraînement initial itératif d'un appelant de base avec un analyte d'une séquence de base à oligo unique avec une séquence de base à oligo unique connue, et la génération de données d'entraînement étiquetées à l'aide de l'appelant de base initialement entraîné, l'appelant de base ayant une configuration de réseau neuronal initiale comprenant un certain nombre de couches et de paramètres ;

(i) l'entraînement supplémentaire de l'appelant de base avec un analyte comprenant des séquences de base à oligos multiples comprenant au moins deux séquences de base à oligos connues qui diffèrent l'une de l'autre par une distance d'édition seuil entre leurs bases nucléotidiques respectives, et la génération de données d'entraînement étiquetées à l'aide de l'appelant de base entraîné en plus ; et

l'entraînement supplémentaire itératif de l'appelant de base en répétant l'étape (i), tout en augmentant, au cours d'au moins une itération, une complexité de la configuration de réseau neuronal initiale de l'appelant de base en augmentant le nombre de couches et de paramètres par rapport à la configuration de réseau neuronal initiale pour ajuster la configuration de réseau neuronal initiale pour les séquences de base à oligos multiples, dans lequel les données d'entraînement étiquetées générées au cours d'une itération étant utilisées pour entraîner l'appelant de base au cours d'une itération immédiatement ultérieure.

2. Procédé mis en œuvre par ordinateur selon la revendication 1, comprenant en outre :
au cours d'au moins une itération d'entraînement supplémentaire de l'appelant de base avec l'analyte comprenant des séquences de bases à oligos multiples :

l'augmentation, dans l'analyte, d'un nombre de séquences de base à oligo unique des séquences de base à oligos multiples ; et
l'augmentation supplémentaire du nombre de couches et de paramètres pour ajuster davantage la configuration de réseau neuronal initiale au nombre accru de séquences de base à oligo unique.

3. Procédé mis en œuvre par ordinateur selon la revendication 1 ou 2, dans lequel l'entraînement initial itératif de l'appelant de base avec l'analyte comprenant la séquence de base à oligo unique comprend :
au cours d'une première itération d'entraînement initial itératif de l'appelant de base :

le peuplement de la séquence de base à oligo unique en une pluralité de grappes d'une cuve à circulation ;
la génération d'une pluralité de signaux de séquence correspondant à la pluralité de grappes, chaque signal de séquence de la pluralité de signaux de séquence étant représentatif des séquences de base chargées dans une grappe correspondante de la pluralité de grappes ;
la prédiction, en fonction de chaque signal de séquence de la pluralité de signaux de séquence, d'appels de base correspondants pour la séquence de base à oligo unique connue, pour générer de ce fait une pluralité d'appels de base prédits ;
la génération, pour chaque signal de séquence de la pluralité de signaux de séquence, d'un signal d'erreur correspondant, en fonction de la comparaison (i) d'appels de base prédits correspondants et (ii) de bases de la séquence de base à oligo unique connue, générant de ce fait une pluralité de signaux d'erreur correspondant à la pluralité de signaux de séquence ; et
l'entraînement initial de l'appelant de base au cours de la première itération, en fonction de la pluralité de signaux d'erreur.

4. Procédé mis en œuvre par ordinateur selon la revendication 3, dans lequel l'entraînement initial itératif de l'appelant de base avec l'analyte comprenant la séquence de base à oligo unique comprend en outre :
au cours d'une seconde itération de l'entraînement initial itératif de l'appelant de base qui a lieu après la première itération d'entraînement initial itératif de l'appelant de base :

à l'aide de l'appelant de base qui a été partiellement entraîné au cours de la première itération, la prédiction, sur la base de chaque signal de séquence de la pluralité de signaux de séquence, d'autres appels de base correspondants pour la séquence de base à oligo unique connue, pour générer de ce fait une pluralité d'autres appels de base prédits ;
la génération, pour chaque signal de séquence de la pluralité de signaux de séquence, d'un autre signal d'erreur correspondant, en fonction de la comparaison (i) d'autres appels de base prédits correspondants et (ii) de bases de la séquence de base à oligo unique connue, générant de ce fait une pluralité d'autres signaux d'erreur correspondant à la pluralité de signaux de séquence ; et
l'entraînement initial supplémentaire de l'appelant de base au cours de la seconde itération, en fonction de la pluralité d'autres signaux d'erreur.

5. Procédé mis en œuvre par ordinateur selon la revendication 4, dans lequel :
la pluralité de signaux de séquence correspondant à la pluralité de grappes, qui sont générés au cours de la première itération de l'entraînement initial itératif de l'appelant de base, est réutilisée pour la seconde itération de l'entraînement initial itératif de l'appelant de base.

6. Procédé mis en œuvre par ordinateur selon l'une quelconque des revendications 4 à 5, dans lequel la comparaison (i) des appels de base prédits correspondants et (ii) des bases de la séquence de base à oligo unique connue comprend :
pour un premier appel de base prédit, (i) la comparaison d'une première base des premiers appels de base prédits avec une première base de la séquence de base à oligo unique connue et (ii) la comparaison d'une seconde base des premiers appels de base prédits avec une seconde base de la séquence de base à oligo unique connue, pour générer un premier signal d'erreur correspondant.

7. Procédé mis en œuvre par ordinateur selon l'une quelconque des revendications 1 à 6, dans lequel l'entraînement supplémentaire itératif de l'appelant de base comprend :

l'entraînement supplémentaire de l'appelant de base pendant N1 itérations avec un analyte comprenant deux séquences de base à oligo unique connues ; et
l'entraînement supplémentaire de l'appelant de base pendant N2 itérations avec un analyte comprenant trois séquences de base à oligo unique connues,
dans lequel les N1 itérations sont réalisées avant les N2 itérations.

8. Procédé mis en œuvre par ordinateur selon l'une quelconque des revendications 1 à 7, dans lequel, lors de l'entraînement initial itératif de l'appelant de base avec l'analyte comprenant la séquence de base à oligo unique, une première configuration de réseau neuronal est chargée au sein de l'appelant de base, et dans lequel l'entraînement itératif supplémentaire de l'appelant de base comprend :
l'entraînement supplémentaire de l'appelant de base pendant N1 itérations avec l'analyte comprenant deux séquences de base à oligo unique connues, de telle sorte que

(i) pour un premier sous-ensemble des itérations N1, une deuxième configuration de réseau neuronal est chargée au sein de l'appelant de base, et
(ii) pour un second sous-ensemble des N1 itérations ayant lieu après le premier sous-ensemble des N1 itérations, une troisième configuration de réseau neuronal est chargée au sein de l'appelant de base, les première, deuxième et troisième configurations de réseau neuronal étant différentes l'une de l'autre.

9. Procédé mis en œuvre par ordinateur selon la revendication 8, dans lequel la deuxième configuration de réseau neuronal est plus complexe que la première configuration de réseau neuronal, et dans lequel la troisième configuration de réseau neuronal est plus complexe que la deuxième configuration de réseau neuronal.

10. Procédé mis en œuvre par ordinateur selon la revendication 8 ou 9, dans lequel la deuxième configuration de réseau neuronal a un plus grand nombre de couches, de poids, ou de paramètres que la première configuration de réseau neuronal.

11. Procédé mis en œuvre par ordinateur selon l'une quelconque des revendications 8 à 10, dans lequel la troisième configuration de réseau neuronal comporte un plus grand nombre de couches, de poids, ou de paramètres que la deuxième configuration de réseau neuronal.

12. Procédé mis en œuvre par ordinateur selon l'une quelconque des revendications 8 à 11, dans lequel l'entraînement supplémentaire de l'appelant de base pendant les N1 itérations avec l'analyte comprenant deux séquences de base à oligo unique connues comprend, pour une itération des N1 itérations :

le peuplement (i) d'une première pluralité de grappes d'une cuve à circulation avec une première séquence de base à oligo connue parmi les deux séquences de base à oligo unique connues et (ii) une seconde pluralité de grappes de la cuve de circulation avec une seconde séquence de base à oligo connue parmi les deux séquences de base à oligo unique connues ;
la prédiction, pour chaque grappe des première et seconde pluralités de grappes, d'appels de base correspondants, de telle sorte qu'une pluralité d'appels de base prédits sont générés ;
la mise en correspondance (i) d'un premier appel de base prédit de la pluralité d'appels de base prédits avec la première séquence de base à oligo connue et (ii) d'un deuxième appel de base prédit de la pluralité d'appels de base prédits avec la seconde séquence de base à oligo connue, tout en s'abstenant de mettre en correspondance un troisième appel de base prédit de la pluralité d'appels de base prédits avec l'une quelconque des première ou seconde séquences de base à oligo connues ;
la génération (i) d'un premier signal d'erreur, en fonction de la comparaison du premier appel de base prédit avec la première séquence de base à oligo connue, et (ii) d'un second signal d'erreur, en fonction de la comparaison du deuxième appel de base prédit avec la seconde séquence de base à oligo connue ; et
l'entraînement supplémentaire de l'appelant de base, en fonction des premier et second signaux d'erreur.

**13.** Procédé mis en œuvre par ordinateur selon la revendication 12, dans lequel la mise en correspondance du premier appel de base prédit avec la première séquence de base à oligo connue des deux séquences de base à oligo unique connues comprend :

la comparaison de chaque base du premier appel de base prédit aux bases correspondantes des première et seconde séquences de base à oligo connues ;
la détermination que le premier appel de base prédit a au moins un nombre seuil de similitudes de bases avec la première séquence de base à oligo connue, et a moins que le nombre seuil de similitudes de bases avec la seconde séquence de base à oligo connue ; et
en fonction de la détermination que le premier appel de base prédit a au moins le nombre seuil de similitudes de bases avec la première séquence de base à oligo connue, la mise en correspondance du premier appel de base prédit avec la première séquence de base à oligo connue.

**14.** Système comportant un ou plusieurs processeurs couplés à une mémoire, la mémoire étant chargée d'instructions informatiques qui, lorsqu'elles sont exécutées sur le ou les processeurs, amènent le système à réaliser un procédé selon l'une quelconque des revendications 1 à 13.

**15.** Support de stockage non transitoire lisible par ordinateur contenant des instructions de programme informatiques qui, lorsqu'elles sont exécutées par un ou plusieurs processeurs, amènent le ou les processeurs à réaliser un procédé selon l'une quelconque des revendications 1 à 13.

**Fig. 1**

200

Lane 202a

Lane 202b

Lane 202P

A section 208 of
a lane

Column 1 of tiles for Lane (P-1)

Column 2 of tiles for Lane (P-1)

Tiles 212

Tiles 212

Clusters 216 within an
example tile

**Fig. 2**

EP 4 364 155 B1

Cluster

304

308

Tile 212

Lane 202

**Fig. 3**

**Fig. 4**

**Fig. 5**

FPGA PCIe Interface & Wrapper

CPU
Comms

609

Patch
Data

613

Weights
and Biases

Cnfg &
Cntrl

611

612

Cluster

601

615

Proc.
Data

Status

616

600

DRAM
Comms

610

On-
Board
DRAM

602

**Fig. 6**

**Fig. 7**

801
TILE M
cycle K-2
CH. 1

802
TILE M
cycle K-1
CH. 1

803
TILE M
cycle K
CH. 1

804
TILE M
cycle K+1
CH. 1

805
TILE M
cycle K+2
CH. 1

TILE M
cycle K-2
CH. 2

811

TILE M
cycle K-1
CH. 2

812

TILE M
cycle K
CH. 2

813

TILE M
cycle K+1
CH. 2

814

TILE M
cycle K+2
CH. 2

815

820  DFC

**Fig. 8A**

851  852  850

861  862

801  802  803  804  805

811  812  813  814  815

870  820

**Fig. 8B**

| 910 | BN_0_on_CPU | 900 |

| 921 | SP_CONV_1_on_FPGA | |
| 922 | SP_CONV_2_on_FPGA | 901 |
| 923 | SP_CONV_3_on_FPGA | |

923A

| 924 | TEMP_CONV_0_on_FPGA | |

924A

| 925 | TEMP_CONV_1_on_FPGA | |

| 930 | Softmax_on_CPU | 902 |

# FIG. 9

FIG. 10

# Specialized Architecture

Spatial Convolution Network    Temporal Convolutional Network    Output Layer

In one implementation:
Each spatial convolution layer has 64 depthwise segregated convolution filters of size 3x3

In one implementation:
Each temporal convolution layer has 64 depthwise combinatory convolution filters of size 3x3

In one implementation:
Input Data:

Raw pixels (15x15 image patch centered around a target cluster)
Subpixel distances
Thousands of trainable parameters
Separate model per 10 cycles
5 + 2 convolution layers
No full-connected layer

# FIG. 11

EP 4 364 155 B1

FIG. 12

Segregated Convolutions

Spatial Convolution Layers

Spatial Convolution
Layer 1

Spatial Convolution
Layer k

set of inputs

input at time step t+1

input at time step t

input at time step t-1

Kernel

Kernel

Kernel

Filter 1

Filter k

Filter 1

Filter k

Filter 1

Filter k

# Combinatory Convolutions

### filter depth (B) = 2

Input 1        Input 2        Input 3

Input Channel 1

Input Channel 1

B = 2    Filter 1

B = 2    Filter 1

Input Channel k

Input Channel k

B = 2    Filter 1

B = 2    Filter 1

# FIG. 13A

Temporal Convolution Layer

EP 4 364 155 B1

# Combinatory Convolutions
### filter depth (B) = 3

Input 1       Input 2       Input 3       Input 4

Input Channel 1    Input Channel 1    Input Channel 1    Input Channel 1

B = 3     B = 3

Filter 1      Filter 1

Input Channel k    Input Channel k    Input Channel k    Input Channel k

B = 3     B = 3

Filter k      Filter k

## FIG. 13B

Temporal Convolution Layer

EP 4 364 155 B1

**Single Oligo Training Stage**

Known synthetic sequence (e.g., known oligo sequence of Oligo # 1) 1406 (also used as ground truth)

| Ga1 | Ga2 | Ga3 | Ga4 | Ga5 | Ga6 | Ga7 | Ga8 |

Sequencing machine 1404

Flow cell 1405

Clusters 1407 (e.g., clusters 1407a, 1407b, ..., 1407G) (10,000 numbers of clusters are assumed in the flow cell 1405, merely as an example)

Sequence signals 1412 (e.g., 1412a, 1412b, ..., 1412G)

Base Caller 1414 (Neural network configuration 1415)

Mapping logic 1416

Predicted Base Call Sequences 1418

1418a
1418b
1418G

**Base Calling System 1400**

1418a

| Sa1 | Sa2 | Sa3 | Sa4 | Sa5 | Sa6 | Sa7 | Sa8 |

| C | A | T | C | G | C | A | G |

Ground truth 1406 (Oligo # 1)

| Ga1 | Ga2 | Ga3 | Ga4 | Ga5 | Ga6 | Ga7 | Ga8 |

| A | C | T | T | G | C | A | C |

Compare 1413a

Gradient update 1417

**Fig. 14A**

1418a

| Sa1 | Sa2 | Sa3 | Sa4 | Sa5 | Sa6 | Sa7 | Sa8 |

⇓

| C | A | T | C | G | C | A | G |

| A | C | T | T | G | C | A | C |

⇑

| Ga1 | Ga2 | Ga3 | Ga4 | Ga5 | Ga6 | Ga7 | Ga8 |
Ground truth 1406 (Oligo # 1)

Compare
1413a

| C should be A | A should be C | Match (no error) | C should be T | Match (no error) | Match (no error) | Match (no error) | G should be C |

Gradient update 1417

**Fig. 14A1**

EP 4 364 155 B1

**Single Oligo Training Stage**

Fig. 14B

Oligo # 1501A (Ground truth 1506a)

| G1 | G1 | G1 | G1 | G1 | G1 | G1 | G1 |

Assume that 5.2k of clusters 1407 are loaded with Oligo # 1510A, and 4.8k of clusters 1407 are loaded with Oligo # 1510b

**Two Oligo Training Stage**
**(Training data generation phase)**

Oligo # 1501B (Ground truth 1506b)

| G2 | G2 | G2 | G2 | G2 | G2 | G2 | G2 |

**Sequencing machine**
**1404**

Flow cell 1405

Sequence signals 1512 (e.g., 1512a, 1512b, ..., 1512G)

Base Caller 1414 (Neural network configuration 1415)

Mapping logic 1416

Predicted Base Call Sequences 1518

1518a

| S | S | S | S | S | S | S | S |

1518b

| S | S | S | S | S | S | S | S |

. . .

1518G

| S | S | S | S | S | S | S | S |

**Base Calling System 1400**

Clusters 1407 (e.g., clusters 1407a, 1407b, ..., 1407G)

**Fig. 15A**

Some of these base call sequences 1518 will be for Oligo 1501A, and remaining of these base call sequences 1518 will be for Oligo 1501B

Oligo # 1501A (Ground truth 1506a)

| A | C | T | T | G | C | A | C |

Oligo # 1501B (Ground truth 1506b)

| C | C | T | A | G | C | A | C |

Edit distance of 2 (Less suitable for training)

**Fig. 15B**

Oligo # 1501A (Ground truth 1506a)

| A | C | T | T | G | C | A | C |

Oligo # 1501B (Ground truth 1506b)

| C | A | T | G | A | T | A | G |

Edit distance of 6 (More suitable for training)

**Fig. 15C**

**Two Oligo Training Stage**
**(Training data generation phase)**

Base call sequences 1518a, 1518b, …, 1518G predicted by Neural network configuration **1415**

Predicted Base Call Sequences 1518a

| C | A | G | G | C | T | A | C |

*Similarity of 2 bases*

Oligo # 1501A (Ground truth 1506a)

| A | C | T | T | G | C | A | C |

*Similarity of 5 bases*

Oligo # 1501B (Ground truth 1506b)

| C | A | T | G | A | T | A | G |

Predicted Base Call Sequences 1518a Mapped to Oligo **1501B**

Predicted Base Call Sequences 1518b

| C | A | T | G | C | A | C | C |

*Similarity of 2 bases*

Oligo # 1501A (Ground truth 1506a)

| A | C | T | T | G | C | A | C |

*Similarity of 3 bases*

Oligo # 1501B (Ground truth 1506b)

| C | A | T | G | A | T | A | G |

Predicted Base Call Sequences 1518b **Inconclusive** mapping

• • •                                                  • • •

Predicted Base Call Sequences 1518G

| A | C | T | T | C | A | A | C |

*Similarity of 6 bases*

Oligo # 1501A (Ground truth 1506a)

| A | C | T | T | G | C | A | C |

*Similarity of 2 bases*

Oligo # 1501B (Ground truth 1506b)

| C | A | T | G | A | T | A | G |

Predicted Base Call Sequences 1518G Mapped to Oligo **1501A**

**Fig. 15D**

**Two Oligo Training Stage**
**(Training data generation phase)**

| Labelled training data 1550 generated from the mapping of Fig. 15D (i.e., the training data is generated by the Neural network configuration **1415**) | | |
|---|---|---|
| **Sequence signals 1512** | **Base Call Sequences Predictions** | **Ground Truth** |
| Sequence signal 1512a | Predicted Base Call Sequences 1518a | Base Sequence of Oligo # 1501B (Ground truth 1506b) |
| Sequence signal 1512b | Predicted Base Call Sequences 1518b | Inconclusive, discard from training data |
| Sequence signal 1512c | Predicted Base Call Sequences 1518c | Base Sequence of Oligo # 1501A (Ground truth 1506a) |
| Sequence signal 1512d | Predicted Base Call Sequences 1518d | Base Sequence of Oligo # 1501A (Ground truth 1506a) |
| Sequence signal 1512e | Predicted Base Call Sequences 1518e | Inconclusive, discard from training data |
| Sequence signal 1512f | Predicted Base Call Sequences 1518f | Base Sequence of Oligo # 1501B (Ground truth 1506b) |
| Sequence signal 1512g | Predicted Base Call Sequences 1518g | Inconclusive, discard from training data |
| ▪ ▪ ▪ | ▪ ▪ ▪ | ▪ ▪ ▪ |
| Sequence signal 1512G | Predicted Base Call Sequences 1518G | Oligo # 1501A (Ground truth 1506a) |

Assume, merely as an example:

- **2,600** base call sequences are mapped to Oligo 1501A,
- **3,000** base call sequences are mapped to Oligo 1501B
- **4,400** base call sequences are inconclusive and not mapped to any oligo

# Fig. 15E

**Two Oligo Training Stage (Training data Consumption and Training phase)**

Fig. 16A

Mapping logic 1416 to map individual ones of the predicted base call sequences 1628 to either oligo 1501A or to oligo 1501B, or declare the mapping of the predicted base call sequence 1628 to be inconclusive (e.g., similar to Fig. 15D)

**Sequencing machine 1404**

Flow cell 1405

Clusters 1407 (e.g., clusters 1407a, 1407b, ..., 1407G)

Sequence signals 1512 (e.g., 1512a, 1512b, ..., 1512G)

Mapping logic 1416

Base Caller 1414 (Neural network configuration 1615)

Somewhat trained, as discussed with respect to Fig. 16A

**Base Calling System 1400**

Predicted Base Call Sequences 1628

Predicted Base Call Sequences 1628a

Predicted Base Call Sequences 1628b

Predicted Base Call Sequences 1628G

The predicted base call sequences 1628 are likely to be relatively more accurate than the predicted base call sequences 1618 of Fig. 16A, as the neural network configuration 1615 is now at least partially trained

# Fig. 16B

EP 4 364 155 B1

70

1650

**Two Oligo Training Stage (Training data generation phase – second iteration)**

| Labelled training data 1650 generated from the mapping of Fig. 16B (i.e., the training data is generated by the Neural network configuration **1615**) | | |
|---|---|---|
| **Sequence signals 1512** | **Base Call Sequences Predictions** | **Ground Truth** |
| Sequence signal 1512a | Predicted Base Call Sequences 1628a | Base Sequence of Oligo # 1501B (Ground truth 1506b) |
| Sequence signal 1512b | Predicted Base Call Sequences 1628b | Base Sequence of Oligo # 1501B (Ground truth 1506b) |
| Sequence signal 1512c | Predicted Base Call Sequences 1628c | Base Sequence of Oligo # 1501A (Ground truth 1506a) |
| Sequence signal 1512d | Predicted Base Call Sequences 1628d | Base Sequence of Oligo # 1501A (Ground truth 1506a) |
| Sequence signal 1512e | Predicted Base Call Sequences 1628e | Inconclusive, discard from training data |
| Sequence signal 1512f | Predicted Base Call Sequences 1628f | Base Sequence of Oligo # 1501B (Ground truth 1506b) |
| Sequence signal 1512g | Predicted Base Call Sequences 1628g | Inconclusive, discard from training data |
| . . . | . . . | . . . |
| Sequence signal 1512G | Predicted Base Call Sequences 1628G | Oligo # 1501A (Ground truth 1506a) |

Assume, merely as an example:
- **3,300** base call sequences are mapped to Oligo 1501A,
- **3,200** base call sequences are mapped to Oligo 1501B
- **3,500** base call sequences are inconclusive and not mapped to any oligo

EP 4 364 155 B1

# Fig. 16C

Clusters 1407
(e.g., clusters
1407a, 1407b, ...,
1407G)

**Two Oligo Training Stage (Training data Consumption and Training phase – second iteration)**

Base Calling System **1400**

Sequencing machine **1404**

Flow cell **1405**

Sequence signals **1512** (e.g., 1512a, 1512b, ..., 1512G)

Mapping logic **1416**

Base Caller **1414** (Neural network configuration **1615**)

Predicted Base Call Sequences **1638**

Predicted Base Call Sequences 1638a

Predicted Base Call Sequences 1638b

• • •

Predicted Base Call Sequences 1638G

Predicted Base Call Sequences 1638a

Base Sequence of Oligo # 1501B (Ground truth 1506b)

Predicted Base Call Sequences 1638b

Base Sequence of Oligo # 1501B (Ground truth 1506b)

Predicted Base Call Sequences 1638c

Base Sequence of Oligo # 1501A (Ground truth 1506a)

Predicted Base Call Sequences 1638d

Base Sequence of Oligo # 1501A (Ground truth 1506a)

Compare 1613

Gradient update **1617** to train NN configuration 1615

Predicted Base Call Sequences 1638f

Base Sequence of Oligo # 1501B (Ground truth 1506b)

• • •

Predicted Base Call Sequences 1638G

Base Sequence of Oligo # 1501A (Ground truth 1506a)

Not used for training the NN configuration 1615 – predicted base call sequences 1618e, 1618g discarded during the training iteration, not used for gradient update

Predicted Base Call Sequences 1638e

Predicted Base Call Sequences 1638g

**Fig. 16D**

EP 4 364 155 B1

(i) Iteratively train **first** NN configuration with single oligo sequence;  <u>1704a</u>
(ii) Generate **first 2-oligo labelled training data** using the trained first NN configuration (~**44%** inconclusive mapping)

↓

(i) Train **second** NN configuration using the **first 2-oligo labelled training data**; and  <u>1704b1</u>
(ii) Generate **second 2-oligo labelled training data** using the at least partially trained second NN configuration (~**35%** inconclusive mapping)

↓

(i) Further train the **second** NN configuration using the **second 2-oligo labelled training data**; and  <u>1704b2</u>
(ii) Generate **third 2-oligo labelled training data** using the further trained second NN configuration (~**32%** inconclusive mapping)

⋮

(i) Further train the **second** NN configuration using **(K-1)$^{th}$ 2-oligo labelled training data**; and  <u>1704bk</u>
(ii) Generate **K$^{th}$ 2-oligo labelled training data** using the further trained second NN configuration (~**20%** inconclusive mapping)

Operations 1704b – for training of second NN configuration and generating training data for next NN configuration

↓

Repeat operations 1704b1, ..., 1704bk to train a **third** NN configuration using **2-oligo ground truth data,** and generate 2-oligo labelled training data for training next NN configuration (~**17%** inconclusive mapping)  — 1704c

. . .   ⋮   . . .

Repeat operations 1704b1, ..., 1704bk to train a **P$^{th}$** NN configuration using **2-oligo ground truth data,** and generate 2-oligo labelled training data for training next NN configuration (~**12%** inconclusive mapping)  —1704P

— 1710

Complexity (e.g., number of layers, filters, topology complexity) of P$^{th}$ NN configuration > Complexity of (P-1)$^{th}$ NN configuration > ... > Complexity of second NN configuration > Complexity of first NN configuration

1700

**Fig. 17A**

1750

| Final Labelled training data 1750 generated by the P<sup>th</sup> NN configuration at the end of method 1700 | | |
|---|---|---|
| **Sequence signals 1512** | **Base Call Sequences Predictions** | **Ground Truth** |
| Sequence signal 1512a | Predicted Base Call Sequences 1728a | Base Sequence of Oligo # 1501B (Ground truth 1506b) |
| Sequence signal 1512b | Predicted Base Call Sequences 1728b | Base Sequence of Oligo # 1501B (Ground truth 1506b) |
| Sequence signal 1512c | Predicted Base Call Sequences 1728c | Base Sequence of Oligo # 1501A (Ground truth 1506a) |
| Sequence signal 1512d | Predicted Base Call Sequences 1728d | Base Sequence of Oligo # 1501A (Ground truth 1506a) |
| Sequence signal 1512e | Predicted Base Call Sequences 1728e | Base Sequence of Oligo # 1501B (Ground truth 1506b) |
| Sequence signal 1512f | Predicted Base Call Sequences 1728f | Base Sequence of Oligo # 1501B (Ground truth 1506b) |
| Sequence signal 1512g | Predicted Base Call Sequences 1728g | Inconclusive, discard from training data |
| . . . | . . . | . . . |
| Sequence signal 1512G | Predicted Base Call Sequences 1728G | Oligo # 1501A (Ground truth 1506a) |

Assume, merely as an example:

- **4.550** base call sequences are mapped to Oligo 1501A,
- **4.250** base call sequences are mapped to Oligo 1501B
- **1.200** base call sequences are inconclusive and not mapped to any oligo

EP 4 364 155 B1

# Fig. 17B

**Two Oligo Training Stage (Training data Consumption and Training phase – second iteration)**

Clusters 1407
(e.g., clusters
1407a, 1407b, ..,
1407G)

Base Calling System **1400**

Sequencing machine
**1404**

Flow cell **1405**

Sequence
signals
**1512**
(e.g., 1512a,
1512b, ...,
1512G)

Mapping
logic **1416**

Base Caller
**1414**
(**3-oligo** Neural
network
configuration
**1815**)

Predicted Base Call Sequences **1838**

Predicted Base Call
Sequences 1838a

Predicted Base Call
Sequences 1838b

▪ ▪ ▪

Predicted Base Call
Sequences 1838G

Predicted Base Call
Sequences 1838a

Base Sequence of Oligo #
1501B (Ground truth 1506b)

Predicted Base Call
Sequences 1838b

Base Sequence of Oligo #
1501B (Ground truth 1506b)

Predicted Base Call
Sequences 1838c

Base Sequence of Oligo #
1501A (Ground truth 1506a)

Predicted Base Call
Sequences 1838d

Base Sequence of Oligo #
1501A (Ground truth 1506a)

Compare
1613

Gradient update
**1817** to train NN
configuration
1615

Predicted Base Call
Sequences 1838e

Base Sequence of Oligo #
1501B (Ground truth 1506b)

Predicted Base Call
Sequences 1838f

Base Sequence of Oligo #
1501B (Ground truth 1506b)

▪ ▪ ▪

Predicted Base Call
Sequences 1838G

Base Sequence of Oligo #
1501A (Ground truth 1506a)

Not used for training the NN
configuration 1815

Predicted Base Call
Sequences 1838g

**Fig. 18A**

**Three Oligo Training Stage**
**(Training data generation phase)**

Oligo # 1801A (Ground truth 1806a)

| G1 | G1 | G1 | G1 | G1 | G1 | G1 | G1 |

Oligo # 1801B (Ground truth 1806b)

| G2 | G2 | G2 | G2 | G2 | G2 | G2 | G2 |

Oligo # 1801C (Ground truth 1806c)

| G2 | G2 | G2 | G2 | G2 | G2 | G2 | G2 |

Assume that 3.2k of clusters 1407 are loaded with Oligo 1810A, 3.3k of clusters 1407 are loaded with Oligo 1810b, and 3.5k of clusters 1407 are loaded with Oligo 1810c

Sequencing machine 1404

Flow cell 1405

Sequence signals 1812 (e.g., 1812a, 1812b, ..., 1812G)

Base Caller 1414 (**3-oligo** Neural network configuration **1815**)

Predicted Base Call Sequences 1818

1818a

| S | S | S | S | S | S | S | S |

1818b

| S | S | S | S | S | S | S | S |

· · ·

1818G

| S | S | S | S | S | S | S | S |

Mapping logic 1416

**Base Calling System 1400**

Clusters 1407 (e.g., clusters 1407a, 1407b, ..., 1407G)

# Fig. 18B

Some of these base call sequences 1818 will be for Oligo 1801A, some for Oligo 1801B, remaining for Oligo 1801C

**Three Oligo Training Stage (Training data generation phase)**
Base call sequences 1818a, 1818b, ..., 1818G predicted by Neural network configuration **1815**

Predicted Base Call
Sequences 1818a

| C | A | G | G | C | T | A | C |

Similarity of 2 bases

Similarity of 5 bases

Similarity of 1 bases

Oligo # 1801A (Ground truth 1806a)

| A | C | T | T | G | C | A | C |

Oligo # 1801B (Ground truth 1806b)

| C | A | T | G | A | T | A | G |

Oligo # 1801C (Ground truth 1806c)

| G | A | C | A | T | A | G | T |

Predicted Base Call
Sequences 1818a
Mapped to Oligo **1801B**

Predicted Base Call
Sequences 1818b

| G | A | T | A | C | G | G | T |

Similarity of 1 base

Similarity of 2 bases

Similarity of 5 bases

Oligo # 1801A (Ground truth 1806a)

| A | C | T | T | G | C | A | C |

Oligo # 1801B (Ground truth 1806b)

| C | A | T | G | A | T | A | G |

Oligo # 1801C (Ground truth 1806c)

| G | A | C | A | T | A | G | T |

Predicted Base Call
Sequences 1818b
Mapped to Oligo **1801C**

• • •

• • •

Predicted Base Call
Sequences 1818G

| T | G | A | T | G | A | T | A |

Similarity of 2 bases

Similarity of 0 base

Similarity of 1 base

Oligo # 1801A (Ground truth 1806a)

| A | C | T | T | G | C | A | C |

Oligo # 1801B (Ground truth 1806b)

| C | A | T | G | A | T | A | G |

Oligo # 1801C (Ground truth 1806c)

| G | A | C | A | T | A | G | T |

Predicted Base Call
Sequences 1818G
**Inconclusive** mapping

# Fig. 18C

1850

| Labelled training data 1850 generated by the 3-oligo NN configuration 1815 | | |
|---|---|---|
| Sequence signals 1812 | Base Call Sequences Predictions | Ground Truth |
| Sequence signal 1812a | Predicted Base Call Sequences 1818a | Base Sequence of Oligo # 1801B (Ground truth 1806b) |
| Sequence signal 1812b | Predicted Base Call Sequences 1818b | Base Sequence of Oligo # 1801C (Ground truth 1806c) |
| Sequence signal 1812c | Predicted Base Call Sequences 1818c | Base Sequence of Oligo # 1801A (Ground truth 1806a) |
| Sequence signal 1812d | Predicted Base Call Sequences 1818d | Base Sequence of Oligo # 1801A (Ground truth 1806a) |
| Sequence signal 1812e | Predicted Base Call Sequences 1818e | Base Sequence of Oligo # 1801B (Ground truth 1806b) |
| Sequence signal 1812f | Predicted Base Call Sequences 1818f | Inconclusive, discard from training data |
| Sequence signal 1812g | Predicted Base Call Sequences 1818g | Base Sequence of Oligo # 1801C (Ground truth 1806c) |
| . . . | . . . | . . . |
| Sequence signal 1812G | Predicted Base Call Sequences 1818G | Inconclusive, discard from training data |

**Fig. 18D**

EP 4 364 155 B1

*1704P*

Repeat operations 1704b1, ..., 1704bk to train a $P^{th}$ NN configuration using **2-oligo ground truth data,** and generate 2-oligo labelled training data for training next NN configuration

*1880*

Last operation of method 1700 of Fig. 17A

*1888a1*

(i) Train **first 3-oligo** NN configuration using the labelled training data from previous stage; and
(ii) Generate **3-oligo labelled training data** using the at least partially trained first 3-oligo NN configuration

*1888a2*

(i) Further train the **first 3-oligo** NN configuration using **3-oligo labelled training data from previous stage**; and
(ii) Generate **new 3-oligo labelled training data** using the further trained **first 3-oligo** NN configuration

. . .          . . .

*1888am*

(i) Further train the **first 3-oligo** NN configuration using **3-oligo labelled training data from previous stage**; and
(ii) Generate **new 3-oligo labelled training data** using the further trained **first 3-oligo** NN configuration

*1888b*

Repeat operations 1888a1, ..., 1888ak to iteratively train a **second 3-oligo NN configuration,** and generate 3-oligo labelled training data for training next stage

Operations 1888a for training of first 3-oligo NN configuration and generating training data for next NN configuration

. . .          . . .

*1888Q*

Repeat operations 1888a1, ..., 1888ak to iteratively train a $Q^{th}$ **3-oligo NN configuration,** and generate 3-oligo labelled training data for training next stage

*1890*

| Complexity of $Q^{th}$ 3-oligo NN configuration | > | Complexity of $(Q-1)^{th}$ 3-ologo NN configuration | > . . . > | Complexity of second 3-oligo NN configuration | > | Complexity of first 3-oligo NN configuration |

**Fig. 18E**

EP 4 364 155 B1

EP 4 364 155 B1

1900

1904a

Iteratively train a 1-oligo NN configuration using one-oligo sequence, and generate labelled training data

1904b

Iteratively train one or more 2-oligo NN configurations using two-oligo sequences, and generate corresponding 2-oligo labelled training data

1904c

Iteratively train one or more 3-oligo NN configurations using three-oligo sequences, and generate corresponding 3-oligo labelled training data

• • •            • • •

1904N

Iteratively train one or more N-oligo NN configurations using N-oligo sequences, and generate corresponding N-oligo labelled training data

**Fig. 19**

**Fig. 20A**

Subsequence 2004a

Subsequence 2004b

⋮ ⋮

Subsequence 2004(S-1)

Subsequence 2004S

Each subsequence 2004 (and multiple synthesized copies thereof) is loaded in a corresponding cluster 1407 (e.g., subsequence 2004a and copies thereof are loaded in cluster 1407a, subsequence 2004b and copies thereof are loaded in cluster 1407b, and so on)

<u>**Organism level Training Stage (Training data generation phase)**</u>

Although not illustrated, the <u>**first organism level**</u> NN configuration <u>**2015**</u> is initially trained using the N-oligo labelled training data from method 1904 of Fig. 19

Sequencing machine <u>1404</u>

Flow cell <u>1405</u>

Sequence signals <u>2012</u> (e.g., 2012a, 2012b, ..., 2012G)

Base Caller <u>1414</u> (<u>**first organism level**</u> NN configuration <u>**2015**</u>)

Mapping logic <u>1416</u>

Predicted Base Call Sequences <u>2018</u>

<u>2018a</u>
| S | S | S | S | S | S | S | S |

<u>2018b</u>
| S | S | S | S | S | S | S | S |

• • •

<u>2018G</u>
| S | S | S | S | S | S | S | S |

**Base Calling System <u>1400</u>**

Clusters 1407 (e.g., clusters 1407a, 1407b, ..., 1407G)

Individual ones of these base call sequences 2018 will be for corresponding ones of the subsequences 2004

# Fig. 20B

FIG. 20C

FIG. 20D

Subsequence 2004b having L1 bases

| A | C | C | T | G | A | G | C | G | A | B1 | B1 | B1 | B1 | . . . | B1 |

L2 (~10 as an example) number of bases

First L2 (e.g., ten) base sequence: A, C, C, T, G, A, G, C, G, A predicted by NN configuration 2015 – highly likely that this base sequence (or small variation thereof) will occur **only once** in Organism sequence 2000

Remaining bases are not used for mapping the subsequence 2004b to the sequence 2000

**Fig. 20E**

Mapping logic 1416

Note the mismatch between the second base, doe not need exact match, only "**substantial**" match

Mapping logic 1416 **uniquely and substantially** maps the first L2 base predictions of subsequence 2004b to a corresponding L2 bases in the sequence 2000

Known organism sequence 2000

| B1 | B1 | . . . | B1 | B1 | B1 | A | G | C | T | G | A | G | C | G | A | C | T | G | A | . . . | G | B1 | B1 | B1 | . . . |

Start of subsequence 2004b

Identified section 2000B (having L1 bases) of the organism sequence 2000, such that first L2 predictions of the subsequence 2004b "substantially" and "uniquely" match with the first L2 bases of the section 2000B

2050

Labelled training data **2050** generated from the mapping of Fig. 20E (i.e., the training data is generated by the Neural network configuration **2015**)

| | | |
|---|---|---|
| Subsequence 2004a | —Inconclusive— mapping | Inconclusive mapping to any section of organism sequence 2000 (NOT used for training) |

Subsequence 2004b

| A | C | C | T | G | A | G | C | G | A | B1 | B1 | B1 | B1 | . . . | B1 |

Section 2000B (having L1 bases) of the organism sequence 2000

*Mapped to*

| A | G | C | T | G | A | G | C | G | A | C | T | G | A | . . . | G |

| | | |
|---|---|---|
| Subsequence 2004c | Mapped to | Section 2000C (having same number of bases as subsequence 2004c) of the organism sequence 2000, such that first L2 predictions of the subsequence 2004c "substantially" and "uniquely" match with the first L2 bases of the section 2000C |
| Subsequence 2004d | —Inconclusive— mapping | Inconclusive mapping to any section of organism sequence 2000 (NOT used for training) |
| | . . . | |
| Subsequence 2004S | Mapped to | Section 2000S (having same number of bases as subsequence 2004S) of the organism sequence 2000, such that first L2 predictions of the subsequence 2004S "substantially" and "uniquely" match with the first L2 bases of the section 2000S |

# Fig. 20F

EP 4 364 155 B1

**Fig. 20G**

Iteratively training of one or more simple organism level NN configurations using the
relatively **simple organism sequence 2000**

2104a1

(i) Train the **first simple organism level** NN configuration **2015** using the N-oligo labelled training data from 1904N
of method 1900 of Fig. 19; and
(ii) Generate labelled training data (Fig. 20F) using the at least partially trained **first simple organism level** NN
configuration **2015** (Figs. 20E, 20F)

2104a2

(i) Further train the **first simple organism level** NN configuration **2015** using labelled training data from previous
stage (Fig. 20G); and
(ii) Generate further labelled training data using the at least partially trained **first simple organism level** NN
configuration **2015**

■ ■ ■                                          ■ ■ ■
2104am

(i) Further train the **first simple organism level** NN configuration **2015** using labelled training data from previous
stage; and
(ii) Generate further labelled training data using the at least partially trained **first simple organism level** NN
configuration **2015**

Operations
2104a for
training of first
simple
organism level
NN
configuration
and
generating
training data
for next NN
configuration

2104b

Repeat operations 2104a1, ..., 2104am to iteratively train a **second simple organism level NN configuration,** and
generate labelled training data for training next stage

■ ■ ■                                          ■ ■ ■
2104R

Repeat operations 2104a1, ..., 2104am to iteratively train a **R$^{th}$ simple organism level NN configuration,** and
generate labelled training data for training next stage

**Fig. 21**

2100

| Simple organism sequence <u>2000</u><br>(e.g., Phix) | ⟹ | Iteratively training R number of simple organism level NN configurations (e.g., see method 2100 of Fig. 21), and generating labelled training data |
| Complex organism sequence <u>2200a</u><br>(e.g., bacteria, such as E-coli) | ⟹ | Iteratively training Ra number of complex organism level NN configurations, and generating labelled training data (e.g., similar to method 2100 of Fig. 21) |
| Further complex organism sequence <u>2200T</u><br>(e.g., human or another mammal) | ⟹ | Iteratively training Rt number of further complex organism level NN configurations (e.g., similar to method 2100 of Fig. 21), and generating labelled training data |

## Fig. 22

Iteratively train, using one oligo ground truth data, a NN configuration (e.g., NN configuration 1415), and generate labelled training data

↓

2308

Iteratively train one or more NN configurations using 2-oligo sequence (e.g., see method 1700 of Fig. 17A), and generate labelled training data

↓

2312

Iteratively train one or more NN configurations using 3-oligo sequence (e.g., see method 1700 of Fig. 17A), and generate labelled training data

· · ·              · · ·

2316

Iteratively train one or more NN configurations using N-oligo sequence (e.g., see method 1900 of Fig. 19), and generate labelled training data

↓

2320

Iteratively train one or more NN configurations using simple organism sequence (e.g., see method 2100 of Fig. 21), and generate labelled training data

· · ·              · · ·

2328

Iteratively train one or more NN configurations using complex organism sequence (e.g., see Fig. 22), and generate labelled training data

# Fig. 23A

EP 4 364 155 B1

2360

**2-oligo sequence training – after
training a first 2-oligo NN configuration**

Chart legend

Trained using training data generated
using non-neural network based model

Trained using training data generated
using neural network based model
discussed herein

**Fig. 23B**

**2-oligo sequence training – after training a first 2-oligo NN configuration, and after training a second 2-oligo NN configuration**

2365

**Fig. 23C**

Chart legend

Trained using training data generated using non-neural network based model

After training a **first** 2-oligo NN configuration

Trained using training data generated using neural network based model discussed herein

After training a **second** 2-oligo NN configuration

EP 4 364 155 B1

Chart legend

Trained using training data generated using non-neural network based model

Trained using training data generated using neural network based model discussed herein

2370

**4-oligo sequence training – after training a first 4-oligo NN configuration**

Mapping percentage

30

25

20

15

10

5

0

Inconclusive mapping | Mapped to oligo 1 | Mapped to oligo 2 | Mapped to oligo 3 | Mapped to oligo 4

# Fig. 23D

EP 4 364 155 B1

Chart legend

| | |
|---|---|
| ———— | Trained using training data generated using non-neural network based model |
| — — — · | Trained using training data generated using neural network based model discussed herein |

2370

**After complex organism sequence training**

Error rate

0.35
0.3
0.25
0.2
0.15
0.1
0.05
0

r1c5  r1c10  r1c15  r1c20  r1c25  r1c30  r2c5  r2c10  r2c15  r2c20  r2c25  r2c30

Reads and read-cycles

(rxcy → $x^{th}$ read iteration and $y^{th}$ read cycle)

**Fig. 23E**

FIG. 24

FIG. 25

Device Module — 2534

Identification Module — 2535

SBS Module — 2536

Amplification Module — 2537

Analysis Module — 2538

Neural Network-Based Template Generator — 2548

Neural Network-Based Base Caller — 2558

Neural Network-Based Quality Scorer — 2568

Main Control Module

2530

Illumination Module

2539

Fluidic Control Module

2531

Fluidic Storage Module

2532

Temp. Control Module

2533

2520

2522

**FIG. 26**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62979384 A
- US 62979414 A
- US 82598720 A
- US 82599120 A
- US 82612620 A
- US 82613420 A
- US 82616820 A
- WO 2015095066 A1
- WO 04018497 A
- WO 04018493 A
- US 7057026 B
- WO 05024010 A
- WO 06120433 A
- WO 05065814 A
- WO 9844151 A
- WO 06064199 A
- WO 07010251 A
- US 2011057111 W
- US 20110059865 A1
- US 5641658 A
- WO 2007010251 A
- US 6090592 A
- US 20020055100 A1
- US 7115400 B
- US 20040096853 A1
- US 20040002090 A1
- US 20070128624 A1

- US 20080009420 A1
- US 20080242560 A1
- US 20080234136 A1
- US 20130079232
- US 7595882 B
- US 87459920 A
- US 17614721 A
- US 10304189 B2
- US 63217644 A
- WO 9106678 A
- WO 07123744 A
- US 7329492 B
- US 7211414 B
- US 7315019 B
- US 7405281 B
- US 61538294 A
- US 61619878 B
- US 62420012 A
- US 20070166705 A1
- US 20060188901 A1
- US 20060240439 A1
- US 200602814714709 A1
- US 7541444 B
- US 7427673 B
- US 7566537 B
- US 7592435 B

**Non-patent literature cited in the description**

- **BENTLEY et al.** *Nature*, 2008, vol. 456, 53-59